# EUROPEAN PATENT APPLICATION

(11) **EP 2 196 453 A1**
(43) Date of publication of application: **16.06.2010**
(21) Application number: 08305909.7
(22) Date of filing: 10.12.2008
(51) Int. Cl.: C07C 235/20, C07C 311/08, C07C 321/28, C07D 213/40, C07D 233/06, C07D 307/77, A61K 31/165, A61K 31/4164, A61K 31/435, A61K 31/343, A61P 31/14, A61P 31/18

(54) **Novel substituted aryl derivatives, their process of preparation and their therapeutical uses as anti-HIV agents**

(71) Applicant: Cellvir, 91000 Evry (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75016 Paris (FR)
(72) Inventor: Emiliani, Stéphane, 75019 Paris (FR); Berlioz-Torrent, Clarisse, 75016 Paris (FR); Benarous, Richard, 75013 Paris (FR); Paris, Jean-Marc, 77360 Vaires sur Marne (FR); Berrut, Sébastien, 69001 Lyon (FR); Barbey, Sabine, 91400 Saclay (FR)
(74) Representative: Jacobson, Claude

(57) **Abstract**

The present invention concerns novel substituted aryl derivatives, their process of preparation and their use for inhibiting integration and for treating viral diseases or disorders such as HIV.

## Description

The present invention is related to novel substituted aryl derivatives, pharmaceutical compositions comprising the same, processes for the preparation of said derivatives and uses of said compositions. Particularly, the present invention relates to pharmaceutical compositions that include substituted aryl derivatives of the invention, and their use in the treatment or the prevention of viral disorders, including HIV.

The Acquired Immuno Deficiency Syndrome (AIDS) is a disease due to infection by the Human Immunodeficiency Virus. AIDS is a global epidemic with virtually every country in the world reporting cases.

Sexual contact is the major mode of transmission of HIV worldwide. The virus can also be transmitted via blood or blood products and infected mothers can transmit HIV to their infants perinatally and as early as the first and second trimester of pregnancy. The virus can also be transmitted from the mother to infant via breast feeding. The prevalence of HIV infection among intravenous drug users is exceptionally high.

The clinical manifestations of HIV infection range from an asymptomatic state to severe disease. The majority of individuals experience no recognizable symptoms upon initial infection but some patients suffer from acute illness about three to six weeks after primary infection. This acute illness is characterized by fever, rigors, arthralgias, myalgias, maculopapulor rash, urticaria, abdominal cramps, diarrhea and aseptic meningitis.

Seroconversion generally occurs between 8 to 12 weeks after infection. Neurologic disease is common in HIV-infected individuals, the most common being encephalopathy or AIDS demantia complex.

UNAIDS estimates that there are around 33 millions HIV-infected patients worldwide. Although there are presently more than 25 anti-HIV drugs approved to treat HIV infected patients, a constant flow of new drugs is essential as the virus mutates rapidly and becomes drug resistant. It is estimated that 6-10% of patients under treatment become multi-resistant and are at strong risk of complete therapeutic failure.

Currently HIV infected patients are treated with Highly Active Anti Retroviral Therapies that rely on a combination of several drugs belonging to different classes. Up to 2003, all approved anti-HIV drugs were inhibitors of the catalytic activity of two viral enzymes, Reverse transcriptase (RT) inhibitors and Protease (PR) inhibitors. Reverse Transcriptase inhibitors include two different classes, Nucleoside/Nucleotide RT Inhibitors (NRTI) and Non Nucleoside RT Inhibitors (NNRTI). In 2003, a new class of Anti-retroviral drug (ARV), Fusion inhibitor (Enfuvirtide) was introduced (Cervia et al, Clin Infect Dis. 2003 Oct 15; 37(8):1102-6, 2003). And lately, in 2007, two other classes of ARV were approved, Entry inhibitors (Maraviroc) targeting the CCR5 co-receptor, and Integrase inhibitors (Raltegravir) (Hughes et al, J. Infect. 2008 Jul; 57(1):1-10.). Although these three novel drugs were very useful to treat patients in therapeutic failure because of multiresistance to RT and PR inhibitors, resistance mutations against these drugs have been already reported.

Although the development of these potent anti-HIV drugs, has allowed HIV-infected people to live longer and to benefit of a higher quality of life, it is clear that these drugs do not cure the HIV infection. Moreover, their prolonged use often results in significant toxicity and in the emergence of drug-resistant viruses. Importantly, the ability of HIV to establish latent reservoirs early in the course of infection ensures the persistence of the virus even in the face of intensive drug therapy and vigorous antiviral immune response. Thus, there is a continuous need for the development of novel anti-HIV therapies to overcome the problems of resistance to the present drugs and to improve treatment efficiency (Daar et al, Top HIV Med. 2008 Oct-Nov; 16(4):110-6).

One of the main characteristic of HIV, like other retroviruses is its ability to integrate in the genomic DNA of the infected host cells. Integration of HIV is one of the important steps of the HIV replication cycle that is required for effective expression, replication and spreading of HIV. One way to inhibit integration of HIV is, to inhibit the catalytic activity of Integrase as Integrase inhibitors like raltegravir and other anti-Integrase reported compounds that target the catalytic activity of Integrase (De Clercq et al, Expert Opin Emerg Drugs 2008 Sep;13(3):393-416.). Mutations conferring resistance to these Integrase inhibitors have been already reported.

However, integrase is not acting alone and needs the assistance and interaction of cellular co-factors in order to complete integration of HIV. Several integrase co-factors have been reported so far, including LEDGF/p75, Transportin SR and others (WO 03/046176 A2, Van Maele et al., Trends Biochem Sci. 2006 Feb; 31(2):98-105, 2006). So, another possible way to inhibit integration of HIV is to develop a novel class of compounds capable of interfering with Integrase-cofactors interaction, without necessarily inhibiting the catalytic activity of integrase. The compounds of the invention are inhibitors of HIV integration as assessed by quantitation of HIV integrated DNA in infected cells using Q-PCR assays. These compounds are thus useful for treating or preventing viral diseases or disorders, such as HIV, by inhibiting integration of the virus into the host genome.

Close structurally-related compounds are disclosed in US 4,540,703, EP 35046, JP 07041459, WO 93/14072, JP 63313773, JP 62178963, JP 59027803. However, none of these documents is concerned with anti-HIV activity.

By contrast, the compounds of the invention are integration inhibitors, and are thus useful for treating or preventing viral diseases or disorders, such as HIV, by inhibiting integration of the virus.

According to a first aspect, the present invention provides compounds of formula (I) 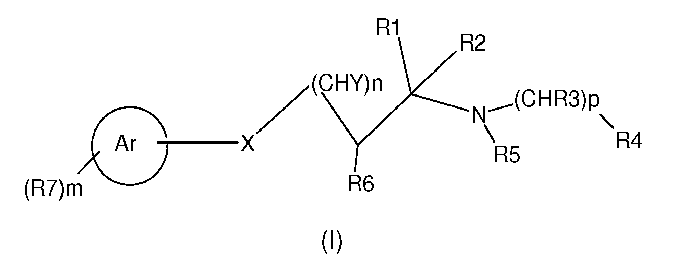 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
Or a R3 form with a substituent of R4 a cycloalkyl, aryl, heteroaryl or heterocycle fused with said R4;
Or a R3 form with R5 a N-containing heterocyle or heteroaryl;
R4 represents an aryl, heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
   - halogen atom; -Alkyl; -Aryl; -OAryl; -Alkyl-OR;
   - Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
   - C(=O)R; -C(=O)NRR'; -O-Alkyl-C(=O)NRR', -O-Alkyl-C(=O)OR, -OAlkyl-NRR', -NR-C(=O)R'; -C(=O)OR; -S(O)R, -S(O)₂R; -OC(=O)R; -OAlkenyl;
   - Alkenyl;
   - Heterocycle optionally substituted by an -Alkyl, -AlkylOR, -AlkylNRR',
   - Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally comprising one or more further heteroatom and optionally substituted by a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 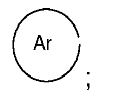
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 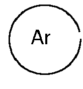 represents an aryl, heteroaryl or unsaturated heterocycle;
m is an integer comprised between 1 and 5, provided that when 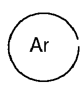 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts,
with the exception of the following compounds :
- the compounds where Ar is a tetrazol, X is S, n is 2, R6 is H, R1 and R2 form together a C=O with the carbon atom to which they are attached; and
- the compounds of formulae :

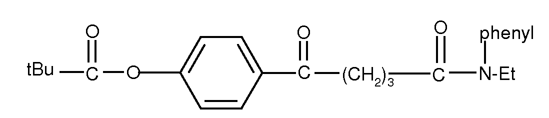

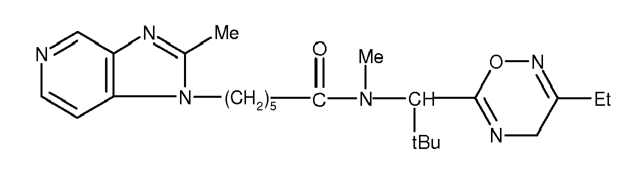

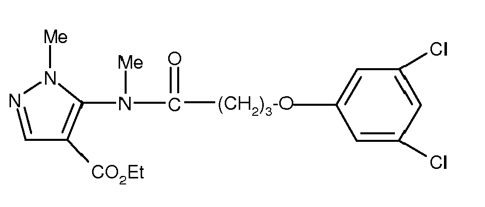

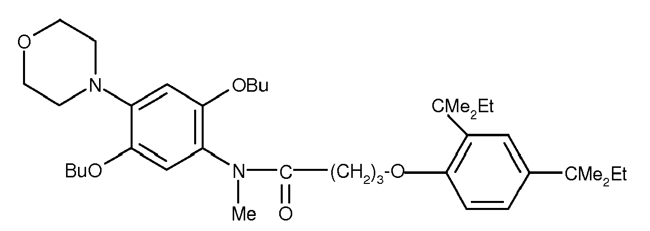

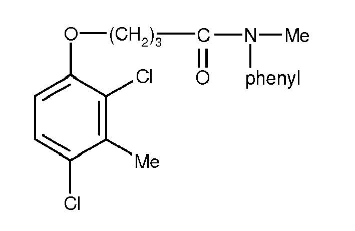

The present invention also encompasses the following preferred embodiements or any of their combination:
- X represents a group chosen from -CRR'-, -NT-, -O-, -S(O)q-, -C(=O)-, where the R of -NR- may be optionally linked with a R7 to form with the N atom to which it is attached a N-containing heterocycle fused with said Ar and/or
- R4 represents an aryl or heteroaryl said aryl or heteroaryl group being optionally fused with an aryl, heteroaryl, saturated or unsaturated cycloalkyl or heterocyclic ring and said optionally fused aryl or heteroaryl being optionally substituted by one or more substituent(s) chosen from :
   - halogen atom; -Alkyl; -Aryl; -OAryl; -Alkyl-OR;
   - Heterocycle optionally substituted by an -Alkyl, -AlkylOR, -AlkylNRR',
   - Alkylaryl, -OR, -Alkyl-Heterocycle, -C(=O)Alkyl or -C(=O)OAlkyl group ;
   - Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl;
   - OR; -NRR'; =O; -C(=O)R; -C(=O)NRR'; -O-Alkyl-C(=O)NRR', -OAlkyl-NRR', -NR-C(=O)R'; -C(=O)OR; -S(O)R -S(O)₂R; -OC(=O)R;
- 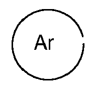 represents an aryl or heteroaryl, and/or
- R1, R2 form together a C=O group with the carbon atom to which they are attached, and/or
- R3 represents a H atom or a group chosen from -alkyl, -aryl, and/or
- R4 represents an aryl optionally fused with an aryl, heteroaryl, or saturated heterocyclic ring and said optionally fused aryl being optionally substituted by one or more substituent(s) chosen from:
   - halogen atom; -Alkyl; -Aryl; -OAryl; -Alkyl-OR;
   - Heterocycle optionally substituted by an -Alkyl, -AlkylOR, -AlkylNRR',
   - Alkylaryl, -OR, -Alkyl-Heterocycle, -C(=O)Alkyl or -C(=O)OAlkyl group ;
   - Alkylaryl; -Alkyl-Heterocycle; perfluoroalkyl-; polyfluoroalkyl-; polyfluoroalkoxy-; -OHeterocycle-Alkyl;
   - OR; -O-Alkyl-C(=O)NRR', -OAlkyl-NRR', -NR-C(=O)R'; -C(=O)OR;
   - OC(=O)R, and/or
- R5 represent a group -Alkyl, -Alkyl-OR, or -Cycloalkyl, each being optionally substituted by one or more substituents chosen from -polyfluoroalkyl groups, and/or
- R6 represents a H atom or a group chosen from -C(=O)NRR', -C(=O)OR, and/or
- R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl;
- C(=O)Aryl; -C(=O)-NRR'; -OAlkylAryl; polyfluoroalkyl-; or 2 R7 form together with the atoms to which they are attached an unsubstituted ring chosen from aryl, heteroaryl, or unsaturated heterocyclic ring fused with 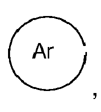 and/or
- X represents a group chosen from -CRR'-, -NT-, -S-, -S(O)-, -C(=O)-., and/or
- Y represents a hydrogen atom, and/or
- 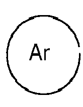 represents an aryl, and/or
- m is 1 or 2, and/or
- n is 2, and/or
- p is 1.

According to a preferred aspect, the compounds of the invention are selected from the group consisting in:
N-benzyl-4-[(4-hydroxyphenyl)thio]-N-methylbutanamide
N,N-dibenzyl-4-[(4-hydroxyphenyl)thio]butanamide
4-[(4-hydroxyphenyl)thio]-N-methyl-N-(pyridin-3-ylmethyl)butanamide
N-ethyl-4-[(4-hydroxyphenyl)thio]-N-(pyridin-4-ylmethyl)butanamide
N-benzyl-4-(4-methoxyphenoxy)-N-methylbutanamide
4-(4-methoxyphenoxy)-N-methyl-N-(pyridin-3-ylmethyl)butanamide
N-benzyl-4-[(3-hydroxyphenyl)thio]-N-methylbutanamide
4-[(3-hydroxyphenyl)thio]-N-methyl-N-(pyridin-3-ylmethyl)butanamide
N-benzyl-4-[(4-methoxyphenyl)thio]-N-methylbutanamide
N,N-dibenzyl-4-[(4-methoxyphenyl)thio]butanamide
4-[(4-methoxyphenyl)thio]-N-methyl-N-(pyridin-3-ylmethyl)butanamide
N,N-dibenzyl-4-[(3-hydroxyphenyl)thio]butanamide
N,N-dibenzyl-4-(4-methoxyphenoxy)butanamide
N-benzyl-4-(4-benzyloxyphenoxy)-N-methylbutanamide
4-[(4-hydroxyphenyl)thio]-N-methyl-N-[1-phenylethyl]butanamide
N-(4-chlorobenzyl)-4-[(4-hydroxyphenyl)thio]-N-methylbutanamide
N-(2-chlorobenzyl)-4-[(4-hydroxyphenyl)thio]-N-methylbutanamide
N-(4-fluorobenzyl)-4-[(4-hydroxyphenyl)thio]-N-methylbutanamide
4-[(4-hydroxyphenyl)thio]-N-(4-methoxybenzyl)-N-methylbutanamide
4-[(4-hydroxyphenyl)thio]-N-methyl-N-[4-(trifluoromethyl)benzyl]butanamide
4-[(4-hydroxyphenyl)thio]-N-methyl-N-(4-methylbenzyl)butanamide
N-benzyl-4-(4-hydroxyphenoxy)-N-methylbutanamide
N-(3-chlorobenzyl)-4-[(4-hydroxyphenyl)thio]-N-methylbutanamide
4-[(4-hydroxyphenyl)thio]-N-methyl-N-phenylbutanamide
N-benzhydryl-4-[(4-hydroxyphenyl)thio]-N-methylbutanamide
4-{[4-oxo-4-(2-phenyl-4,5-dihydro-1H-imidazol-1-yl)butyl]thio}phenol
4-[(4-hydroxyphenyl)thio]-N,N-bis(pyridin-2-ylmethyl)butanamide
4-[(4-hydroxyphenyl)thio]-N-methyl-N-(2-methylbenzyl)butanamide
N-(2,4-dichlorobenzyl)-4-[(4-hydroxyphenyl)thio]-N-methylbutanamide
4-[(4-hydroxyphenyl)thio]-N-(2-methoxybenzyl)-N-methylbutanamide
N-(2,3-dichlorobenzyl)-4-[(4-hydroxyphenyl)thio]-N-methylbutanamide
N-(2-fluorobenzyl)-4-[(4-hydroxyphenyl)thio]-N-methylbutanamide
N-(2,6-dichlorobenzyl)-4-[(4-hydroxyphenyl)thio]-N-methylbutanamide
N-(2-chlorobenzyl)-4-[(4-hydroxyphenyl)sulfinyl]-N-methylbutanamide
N-(2-chloro-6-fluorobenzyl)-4-[(4-hydroxyphenyl)thio]-N-methylbutanamide
N-(2-ethoxybenzyl)-4-[(4-hydroxyphenyl)thio]-N-methylbutanamide
N-(2,3-dimethoxybenzyl)-4-[(4-hydroxyphenyl)thio]-N-methylbutanamide
N-(2-furylmethyl)-4-[(4-hydroxyphenyl)thio]-N-methylbutanamide
4-[(4-hydroxyphenyl)thio]-N-methyl-N-[2-(trifluoromethoxy)benzyl]butanamide
4-[(3-hydroxyphenyl)thio]-N-(2-methoxybenzyl)-N-methylbutanamide
4-[(4-fluorophenyl)thio]-N-(2-methoxybenzyl)-N-methylbutanamide
4-[(4-hydroxyphenyl)thio]-N-methyl-N-(pyridin-2-ylmethyl)butanamide
N-(2-hydroxybenzyl)-4-[(4-hydroxyphenyl)thio]-N-methylbutanamide
N-(2,3-dihydro-1-benzofuran-7-ylmethyl)-4-[(4-hydroxyphenyl)thio]-N-methylbutanamide
4-[(2-hydroxyphenyl)thio]-N-(2-methoxybenzyl)-N-methylbutanamide
4-{[4-(acetylamino)phenyl]thio}-N-(2-methoxybenzyl)-N-methylbutanamide
5-(4-hydroxyphenyl)-N-(2-methoxybenzyl)-N-methylpentanamide
N-(2-methoxybenzyl)-N-methyl-4-{[4-(methylsulfonylamino)phenyl]thio}butanamide
N-[2-(acetylamino)benzyl]-4-[(4-hydroxyphenyl)thio]-N-methylbutanamide
4-[(4-hydroxyphenyl)thio]-N-methyl-N-(1-naphthylmethyl)butanamide
N-(2-methoxybenzyl)-N-methyl-4-(2-naphthylthio)butanamide
N-(2-methoxybenzyl)-N-methyl-4-{[4-(trifluoromethyl)phenyl]thio}butanamide
N-(2-methoxybenzyl)-N-methyl-4-(quinolin-2-ylthio)butanamide
N-(1,3-benzodioxol-4-ylmethyl)-4-[(4-hydroxyphenyl)thio]-N-methylbutanamide
N-(2-methoxybenzyl)-N-methyl-4-{[5-(trifluoromethyl)pyridin-2-yl]thio}butanamide
4-[(4-hydroxyphenyl)thio]-N-methyl-N-[2-(trifluoromethyl)benzyl]butanamide
4-[(4-hydroxyphenyl)thio]-N-(2-isopropoxybenzyl)-N-methylbutanamide
N-(2-methoxybenzyl)-4-[(4-methoxyphenyl)thio]-N-methylbutanamide
4-[(5-chloropyridin-2-yl)thio]-N-(2-methoxybenzyl)-N-methylbutanamide
N-(2-chlorobenzyl)-4-[(4-fluorophenyl)thio]-N-methylbutanamide
Methyl 4-({4-[(2-methoxybenzyl)methylamino]-4-oxobutyl}thio)benzoate
N-(2-methoxybenzyl)-N-methyl-4-(quinoxalin-2-ylthio)butanamide
4-[(4-fluorophenyl)thio]-N-(2-hydroxybenzyl)-N-methylbutanamide
4-[(4-hydroxyphenyl)thio]-N-methyl-N-[2-(4-methylpiperazin-1-yl)benzyl]butanamide
4-({4-[(2-methoxybenzyl)(methyl)amino]-4-oxobutyl}thio)benzoic acid
4-[(4-fluorophenyl)thio]-N-(2-methoxyphenyl)-N-methylbutanamide
4-({4-[(2-methoxybenzyl)(methyl)amino]-4-oxobutyl}thio)-N-methylbenzamide
4-[(4-fluorophenyl)(methyl)amino]-N-(2-methoxybenzyl)-N-methylbutanamide
4-[(4-hydroxyphenyl)thio]-N-methyl-N-(2-morpholin-4-ylbenzyl)butanamide
5-[(4-hydroxyphenyl)thio]-N-(2-methoxybenzyl)-N-methylpentanamide
4-[(4-fluorophenyl)thio]-N-[2-(2-methylamino-2-oxoethoxy)benzyl]-N-methylbutanamide
N-[2-(2-aminoethoxy)benzyl]-4-[(4-fluorophenyl)thio]-N-methylbutanamide
4-(1H-indol-5-yloxy)-N-(2-methoxybenzyl)-N-methylbutanamide
N-(2,3-dichlorobenzyl)-4-[(4-fluorophenyl)thio]-N-methylbutanamide
N-{2-[2-(dimethylamino)ethoxy]benzyl}-4-[(4-fluorophenyl)thio]-N-methylbutanamide
Methyl 2-{[{4-[(4-hydroxyphenyl)thio]butanoyl}(methyl)amino]methyl}benzoate
2-{[{4-[(4-hydroxyphenyl)thio]butanoyl}(methyl)amino]methyl}benzoic acid
Ethyl 4-[2-({4-[(4-hydroxyphenyl)thio]butanoyl}methylamino)methylphenyl]piperazine-1-carboxylate
4-[(4-hydroxyphenyl)thio]-N-[2-(4-hydroxypiperidin-1-yl)benzyl]-N-methylbutanamide
4-[(4-hydroxyphenyl)thio]-N-methyl-N-(2-phenoxybenzyl)butanamide
4-[(4-hydroxyphenyl)thio]-N-methyl-N-(2-piperazin-1-ylbenzyl)butanamide
4-[(4-fluorophenyl)thio]-N-(2-isopropoxybenzyl)-N-methylbutanamide
4-[(4-fluorophenyl)thio]-N-methyl-N-(2-morpholin-4-ylbenzyl)butanamide
Methyl 4-[(4-hydroxyphenyl)thio]-2-{[(2-methoxybenzyl)(methyl)amino]-carbonyl}butanoate
4-[(4-hydroxyphenyl)thio]-N-methyl-N-(2-benzylbenzyl)butanamide
4-[(4-hydroxyphenyl)thio]-N-methyl-N-{2-[4-(2-morpholin-4-ylethyl)piperazin-1-yl]benzyl}butanamide
N-[2-(4-acetylpiperazin-1-yl)benzyl]-4-({4-[benzyl(methyl)amino]-4-oxobutyl}thio)phenyl acetate
4-[(4-hydroxyphenyl)thio]-2-{[(2-methoxybenzyl)(methyl)amino]carbonyl}butanoic acid
N-[2-(4-benzylpiperazin-1-yl)benzyl]-4-[(4-hydroxyphenyl)thio]-N-methylbutanamide
5-(4-hydroxyphenyl)-N-methyl-N-(2-morpholin-4-ylbenzyl)pentanamide
5-(4-fluorophenyl)-N-(2-methoxybenzyl)-N-methyl-5-oxopentanamide
N-(1,1'-biphenyl-2-ylmethyl)-4-[(4-hydroxyphenyl)thio]-N-methylbutanamide
4-[(4-hydroxyphenyl)thio]-N-(2-methoxybenzyl)-N-methylbutanethioamide
5-(4-fluorophenyl)-N-(2-methoxybenzyl)-N-methylpentanamide
N-[2-(4-acetylpiperazin-1-yl)benzyl]-4-[(4-hydroxyphenyl)thio]-N-methylbutanamide
4-[(3,4-difluorophenyl)thio]-N-(2-methoxybenzyl)-N-methylbutanamide
4-[(3-fluorophenyl)thio]-N-(2-methoxybenzyl)-N-methylbutanamide
2-{2-[(4-hydroxyphenyl)thio]ethyl}-N-(2-methoxybenzyl)-N,N'-dimethylmalonamide
5-(4-hydroxyphenyl)-N-(2-isopropoxybenzyl)-N-methylpentanamide
N-(2-hydroxyethyl)-4-[(4-hydroxyphenyl)thio]-N-(2-methoxybenzyl)butanamide
4-[(4-fluorophenyl)methylamino]-N-(2-isopropoxybenzyl)-N-methylbutanamide
4-[(4-fluorophenyl)(methyl)amino]-N-methyl-N-[2-(trifluoromethyl)benzyl]butanamide
4-[(2,4-difluorophenyl)thio]-N-(2-methoxybenzyl)-N-methylbutanamide
N-{2-[4-(2-dimethylaminoethyl)piperazin-1-yl]benzyl}-4-[(4-hydroxyphenyl)thio]-N-methylbutanamide
4-[(4-hydroxyphenyl)thio]-N-(2-methoxybenzyl)-N-(2-morpholin-4-ylethyl)butanamide
4-[(4-hydroxyphenyl)thio]-N-[2-(methoxymethyl)benzyl]-N-methylbutanamide
N-cyclopropylmethyl-4-[(4-hydroxyphenyl)thio]-N-(2-methoxybenzyl)butanamide
N-ethyl-4-[(4-hydroxyphenyl)thio]-N-(2-methoxybenzyl)butanamide
N-(3-fluoro-2-methoxybenzyl)-4-[(4-hydroxyphenyl)thio]-N-methylbutanamide
4-[(4-hydroxyphenyl)thio]-N-(2-methoxy-3-methylbenzyl)-N-methylbutanamide
N-(5-chloro-2-methoxybenzyl)-4-[(4-hydroxyphenyl)thio]-N-methylbutanamide
4-[(3-chloro-4-fluorophenyl)thio]-N-(2-methoxybenzyl)-N-methylbutanamide
4-[(2-chloro-4-fluorophenyl)thio]-N-(2-methoxybenzyl)-N-methylbutanamide
N-(5-fluoro-2-methoxybenzyl)-4-[(4-hydroxyphenyl)thio]-N-methylbutanamide
4-[(4-hydroxyphenyl)thio]-N-methyl-N-[2-(morpholin-4-ylmethyl)benzyl]butanamide
N-{2-[4-(2-hydroxyethyl)piperazin-1-yl]benzyl}-4-[(4-hydroxyphenyl)thio]-N-methylbutanamide
N-(3,5-dichloro-2-methoxybenzyl)-4-[(4-hydroxyphenyl)thio]-N-methylbutanamide
4-[(4-hydroxyphenyl)thio]-N-(2-methoxy-4-methylbenzyl)-N-methylbutanamide
4-[(4-fluorophenyl)thio]-N-(2-methoxybenzyl)-N-(2,2,2-trifluoroethyl)butanamide
(3S)-4-[(4-fluorophenyl)thio]-3-hydroxy-N-(2-methoxybenzyl)-N-methylbutanamide
Methyl 4-({4-[methyl-(2-trifluoromethylbenzyl)amino]-4-oxobutyl}thio)benzoate
N-cyclopropyl-4-[(4-hydroxyphenyl)thio]-N-(2-methoxybenzyl)butanamide
4-[(4-{methyl[2-(trifluoromethyl)benzyl]amino}-4-oxobutyl)thio]benzoic acid
Methyl 5-fluoro-2-({4-[methyl-(2-methoxybenzyl)amino]-4-oxobutyl}thio)benzoate
4-[(4-fluorophenyl)methylamino]-N-[2-(4-methylpiperazin-1-yl)benzyl]-N-methylbutanamide
4-[(4-hydroxyphenyl)thio]-N-methyl-N-{2-[(1-methylpiperidin-4-yl)oxy]benzyl}butanamide
5-fluoro-2-({4-[(2-methoxybenzyl)(methyl)amino]-4-oxobutyl}thio)benzoic acid
4-[(4-hydroxyphenyl)thio]-N-methyl-N-[2-(1,2,3,6-tetrahydropyridin-4-yl)benzyl]butanamide
4-[(4-fluorophenyl)sulfonyl]-N-(2-methoxybenzyl)-N-methylbutanamide
4-[(4-fluorophenyl)(2-hydroxyethyl)amino]-N-(2-methoxybenzyl)-N-methylbutanamide
4-[(4-fluorophenyl)methylamino]-N-methyl-N-(2-{4-[5-(trifluoromethyl)pyridin-2-yl]piperazin-1-yl}benzyl)butanamide
4-(5-fluoro-2,3-dihydro-1H-indolyl-1-yl)-N-(2-isopropoxybenzyl)-N-methylbutanamide
4-(3,4-dihydroquinolin-1 (2H)-yl)-N-(2-isopropoxybenzyl)-N-methylbutanamide
4-(5-fluoro-2,3-dihydro-1H-indolyl-1-yl)-N-(2-methoxybenzyl)-N-methylbutanamide
4-[(4-fluorophenyl)(2-morpholin-4-ylethyl)amino]-N-(2-methoxybenzyl)-N-methylbutanamide
4-[cyclopropyl(4-fluorophenyl)amino]-N-(2-methoxybenzyl)-N-methylbutanamide
4-[ethyl(4-fluorophenyl)amino]-N-(2-methoxybenzyl)-N-methylbutanamide
N-(2,5-difluorobenzyl)-4-[(4-fluorophenyl)thio]-N-methylbutanamide
N-3,4-dihydro-2H-chromen-4-yl-4-[(4-fluorophenyl)thio]-N-methylbutanamide
4-[(4-fluorophenyl)thio]-N-methyl-N-1-naphthylbutanamide
4-[(4-fluorophenyl)thio]-N-methyl-N-1,2,3,4-tetrahydronaphthalen-1-ylbutanamide
N-(5-fluoro-2-methoxybenzyl)-4-[(4-fluorophenyl)thio]-N-methylbutanamide
N-(5-fluoro-2-isopropoxybenzyl)-4-[(4-fluorophenyl)thio]-N-methylbutanamide
4-[(4-fluorophenyl)amino]-N-(2-methoxybenzyl)-N-methylbutanamide
4-[(4-fluorophenyl)thio]-N-(1H-indol-7-ylmethyl)-N-methylbutanamide
N-(6-fluoro-3,4-dihydro-2H-chromen-4-yl)-4-[(4-fluorophenyl)thio]-N-methylbutanamide
N-(5-fluoro-2-methoxybenzyl)-4-[(4-methoxyphenyl)thio]-N-methylbutanamide
N-(2,6-dimethoxybenzyl)-4-[(4-fluorophenyl)thio]-N-methylbutanamide
N-(2-fluoro-5-methoxybenzyl)-4-[(4-fluorophenyl)thio]-N-methylbutanamide
N-[2-(allyloxy)benzyl]-4-[(4-fluorophenyl)thio]-N-methylbutanamide
N-(5-allyl-2-hydroxy-3-methoxybenzyl)-4-[(4-fluorophenyl)thio]-N-methylbutanamide
N-[(4-bromothien-2-yl)methyl]-4-[(4-fluorophenyl)thio]-N-methylbutanamide
N-(5-bromo-2-methoxybenzyl)-4-[(4-fluorophenyl)thio]-N-methylbutanamide
4-[(4-bromophenyl)thio]-N-(5-fluoro-2-methoxybenzyl)-N-methylbutanamide
as well as their their racemates, stereoisomers, corresponding free forms or pharmaceutically acceptable salts.

More preferably, the compounds of the invention are chosen from:
N-benzyl-4-[(4-hydroxyphenyl)thio]-N-methylbutanamide
4-[(4-hydroxyphenyl)thio]-N-methyl-N-[(1S)-1-phenylethyl]butanamide
N-(4-chlorobenzyl)-4-[(4-hydroxyphenyl)thio]-N-methylbutanamide
N-(2-chlorobenzyl)-4-[(4-hydroxyphenyl)thio]-N-methylbutanamide
N-(4-fluorobenzyl)-4-[(4-hydroxyphenyl)thio]-N-methylbutanamide
4-[(4-hydroxyphenyl)thio]-N-(4-methoxybenzyl)-N-methylbutanamide
4-[(4-hydroxyphenyl)thio]-N-methyl-N-[4-(trifluoromethyl)benzyl]butanamide
4-[(4-hydroxyphenyl)thio]-N-methyl-N-(4-methylbenzyl)butanamide
N-benzyl-4-(4-hydroxyphenoxy)-N-methylbutanamide
N-(3-chlorobenzyl)-4-[(4-hydroxyphenyl)thio]-N-methylbutanamide
4-[(4-hydroxyphenyl)thio]-N-methyl-N-phenylbutanamide
N-benzhydryl-4-[(4-hydroxyphenyl)thio]-N-methylbutanamide
4-[(4-hydroxyphenyl)thio]-N-methyl-N-(2-methylbenzyl)butanamide
N-(2,4-dichlorobenzyl)-4-[(4-hydroxyphenyl)thio]-N-methylbutanamide
4-[(4-hydroxyphenyl)thio]-N-(2-methoxybenzyl)-N-methylbutanamide
N-(2,3-dichlorobenzyl)-4-[(4-hydroxyphenyl)thio]-N-methylbutanamide
N-(2-fluorobenzyl)-4-[(4-hydroxyphenyl)thio]-N-methylbutanamide
N-(2,6-dichlorobenzyl)-4-[(4-hydroxyphenyl)thio]-N-methylbutanamide
N-(2-chlorobenzyl)-4-[(4-hydroxyphenyl)sulfinyl]-N-methylbutanamide
N-(2-chloro-6-fluorobenzyl)-4-[(4-hydroxyphenyl)thio]-N-methylbutanamide
N-(2-ethoxybenzyl)-4-[(4-hydroxyphenyl)thio]-N-methylbutanamide
N-(2,3-dimethoxybenzyl)-4-[(4-hydroxyphenyl)thio]-N-methylbutanamide
N-(2-furylmethyl)-4-[(4-hydroxyphenyl)thio]-N-methylbutanamide
4-[(4-hydroxyphenyl)thio]-N-methyl-N-[2-(trifluoromethoxy)benzyl]butanamide
4-[(3-hydroxyphenyl)thio]-N-(2-methoxybenzyl)-N-methylbutanamide
4-[(4-fluorophenyl)thio]-N-(2-methoxybenzyl)-N-methylbutanamide
N-(2-hydroxybenzyl)-4-[(4-hydroxyphenyl)thio]-N-methylbutanamide
N-(2,3-dihydro-1-benzofuran-7-ylmethyl)-4-[(4-hydroxyphenyl)thio]-N-methylbutanamide
4-{[4-(acetylamino)phenyl]thio}-N-(2-methoxybenzyl)-N-methylbutanamide
5-(4-hydroxyphenyl)-N-(2-methoxybenzyl)-N-methylpentanamide
N-(2-methoxybenzyl)-N-methyl-4-{[4-(methylsulfonylamino)phenyl]thio}butanamide
N-[2-(acetylamino)benzyl]-4-[(4-hydroxyphenyl)thio]-N-methylbutanamide
4-[(4-hydroxyphenyl)thio]-N-methyl-N-(1-naphthylmethyl)butanamide
N-(2-methoxybenzyl)-N-methyl-4-(2-naphthylthio)butanamide
N-(2-methoxybenzyl)-N-methyl-4-{[4-(trifluoromethyl)phenyl]thio}butanamide
N-(2-methoxybenzyl)-N-methyl-4-(quinolin-2-ylthio)butanamide
N-(1,3-benzodioxol-4-ylmethyl)-4-[(4-hydroxyphenyl)thio]-N-methylbutanamide
N-(2-methoxybenzyl)-N-methyl-4-{[5-(trifluoromethyl)pyridin-2-yl]thio}butanamide
4-[(4-hydroxyphenyl)thio]-N-methyl-N-[2-(trifluoromethyl)benzyl]butanamide
4-[(4-hydroxyphenyl)thio]-N-(2-isopropoxybenzyl)-N-methylbutanamide
N-(2-methoxybenzyl)-4-[(4-methoxyphenyl)thio]-N-methylbutanamide
N-(2-chlorobenzyl)-4-[(4-fluorophenyl)thio]-N-methylbutanamide
Methyl 4-({4-[(2-methoxybenzyl)methylamino]-4-oxobutyl}thio)benzoate
N-(2-methoxybenzyl)-N-methyl-4-(quinoxalin-2-ylthio)butanamide
4-[(4-hydroxyphenyl)thio]-N-methyl-N-[2-(4-methylpiperazin-1-yl)benzyl]butanamide
4-[(4-fluorophenyl)thio]-N-(2-methoxyphenyl)-N-methylbutanamide
4-({4-[(2-methoxybenzyl)(methyl)amino]-4-oxobutyl}thio)-N-methylbenzamide
4-[(4-hydroxyphenyl)thio]-N-methyl-N-(2-morpholin-4-ylbenzyl)butanamide
5-[(4-hydroxyphenyl)thio]-N-(2-methoxybenzyl)-N-methylpentanamide
Methyl 2-{[{4-[(4-hydroxyphenyl)thio]butanoyl}(methyl)amino]methyl}benzoate
Ethyl 4-[2-({4-[(4-hydroxyphenyl)thio]butanoyl}methylamino)methylphenyl]piperazine-1-carboxylate
4-[(4-hydroxyphenyl)thio]-N-[2-(4-hydroxypiperidin-1-yl)benzyl]-N-methylbutanamide
4-[(4-hydroxyphenyl)thio]-N-methyl-N-(2-phenoxybenzyl)butanamide
4-[(4-hydroxyphenyl)thio]-N-methyl-N-(2-piperazin-1-ylbenzyl)butanamide
4-[(4-fluorophenyl)thio]-N-(2-isopropoxybenzyl)-N-methylbutanamide
4-[(4-fluorophenyl)thio]-N-methyl-N-(2-morpholin-4-ylbenzyl)butanamide
4-[(4-hydroxyphenyl)thio]-N-methyl-N-(2-benzylbenzyl)butanamide
4-[(4-hydroxyphenyl)thio]-N-methyl-N-{2-[4-(2-morpholin-4-ylethyl)piperazin-1-yl]benzyl}butanamide
5-(4-hydroxyphenyl)-N-methyl-N-(2-morpholin-4-ylbenzyl)pentanamide
5-(4-fluorophenyl)-N-(2-methoxybenzyl)-N-methyl-5-oxopentanamide
N-(1,1'-biphenyl-2-ylmethyl)-4-[(4-hydroxyphenyl)thio]-N-methylbutanamide
4-[(4-hydroxyphenyl)thio]-N-(2-methoxybenzyl)-N-methylbutanethioamide
5-(4-fluorophenyl)-N-(2-methoxybenzyl)-N-methylpentanamide
N-[2-(4-acetylpiperazin-1-yl)benzyl]-4-[(4-hydroxyphenyl)thio]-N-methylbutanamide
4-[(3,4-difluorophenyl)thio]-N-(2-methoxybenzyl)-N-methylbutanamide
4-[(3-fluorophenyl)thio]-N-(2-methoxybenzyl)-N-methylbutanamide
5-(4-hydroxyphenyl)-N-(2-isopropoxybenzyl)-N-methylpentanamide
N-(2-hydroxyethyl)-4-[(4-hydroxyphenyl)thio]-N-(2-methoxybenzyl)butanamide
4-[(2,4-difluorophenyl)thio]-N-(2-methoxybenzyl)-N-methylbutanamide
4-[(4-hydroxyphenyl)thio]-N-[2-(methoxymethyl)benzyl]-N-methylbutanamide
N-cyclopropylmethyl-4-[(4-hydroxyphenyl)thio]-N-(2-methoxybenzyl)butanamide
N-ethyl-4-[(4-hydroxyphenyl)thio]-N-(2-methoxybenzyl)butanamide
N-(3-fluoro-2-methoxybenzyl)-4-[(4-hydroxyphenyl)thio]-N-methylbutanamide
4-[(4-hydroxyphenyl)thio]-N-(2-methoxy-3-methylbenzyl)-N-methylbutanamide
N-(5-chloro-2-methoxybenzyl)-4-[(4-hydroxyphenyl)thio]-N-methylbutanamide
4-[(3-chloro-4-fluorophenyl)thio]-N-(2-methoxybenzyl)-N-methylbutanamide
4-[(2-chloro-4-fluorophenyl)thio]-N-(2-methoxybenzyl)-N-methylbutanamide
N-(5-fluoro-2-methoxybenzyl)-4-[(4-hydroxyphenyl)thio]-N-methylbutanamide
4-[(4-hydroxyphenyl)thio]-N-methyl-N-[2-(morpholin-4-ylmethyl)benzyl]butanamide
N-(3,5-dichloro-2-methoxybenzyl)-4-[(4-hydroxyphenyl)thio]-N-methylbutanamide
4-[(4-hydroxyphenyl)thio]-N-(2-methoxy-4-methylbenzyl)-N-methylbutanamide
(3S)-4-[(4-fluorophenyl)thio]-3-hydroxy-N-(2-methoxybenzyl)-N-methylbutanamide
4-[(4-hydroxyphenyl)thio]-N-methyl-N-[2-(1,2,3,6-tetrahydropyridin-4-yl)benzyl]butanamide
4-[(4-fluorophenyl)methylamino]-N-methyl-N-(2-{4-[5-(trifluoromethyl)pyridin-2-yl]piperazin-1-yl}benzyl)butanamide
4-(5-fluoro-2,3-dihydro-1H-indolyl-1-yl)-N-(2-isopropoxybenzyl)-N-methylbutanamide
4-(3,4-dihydroquinolin-1(2H)-yl)-N-(2-isopropoxybenzyl)-N-methylbutanamide
4-(5-fluoro-2,3-dihydro-1H-indolyl-1-yl)-N-(2-methoxybenzyl)-N-methylbutanamide
N-(2,5-difluorobenzyl)-4-[(4-fluorophenyl)thio]-N-methylbutanamide
4-[(4-fluorophenyl)thio]-N-methyl-N-1-naphthylbutanamide
N-(5-fluoro-2-methoxybenzyl)-4-[(4-fluorophenyl)thio]-N-methylbutanamide
N-(5-fluoro-2-isopropoxybenzyl)-4-[(4-fluorophenyl)thio]-N-methylbutanamide
N-(5-fluoro-2-methoxybenzyl)-4-[(4-methoxyphenyl)thio]-N-methylbutanamide
N-[2-(allyloxy)benzyl]-4-[(4-fluorophenyl)thio]-N-methylbutanamide
N-(5-bromo-2-methoxybenzyl)-4-[(4-fluorophenyl)thio]-N-methylbutanamide
4-[(4-bromophenyl)thio]-N-(5-fluoro-2-methoxybenzyl)-N-methylbutanamide
as well as their their racemates, stereoisomers, corresponding free forms or pharmaceutically acceptable salts.

As used herein, the term "alkyl" refers to a branched or straight hydrocarbon chain of 1 to 8 carbon atoms, which is formed by the removal of one hydrogen atom. In certain preferred embodiments, the alkyl group contains from 1 to 6 carbon atoms. In other preferred embodiments, the alkyl group contains from 1 to 4 carbon atoms. A designation such as "C₁-C₄ alkyl" refers to an alkyl radical containing from 1 to 4 carbon atoms. Examples include methyl, ethyl, n-propyl, isopropyl, n-butyl, isobutyl, sec-butyl, t-butyl, pentyl, 2-methylpentyl, hexyl, 2-methylhexyl, 2,3-dimethylhexyl, heptyl, octyl, etc.

As used herein, the term "cycloalkyl" refers to an aromatic or non aromatic hydrocarbon mono, bi or multi cyclic ring of 3 to 10 carbon atoms formed by the removal of one hydrogen atom. A designation such as "C₅-C₇ cycloalkyl" refers to a cycloalkyl radical containing from 5 to 7 carbon atoms. Examples include cyclopropyl, cyclopentyl, cyclohexyl, cycloheptyl, adamantyl, etc. as well as the systems formed by their condensation or by the condensation with a phenyl group.

As used herein, the term "aromatic"or "aryl" in aryl or heteroaryl refers to a cyclic carbocyclic aryl or heteroaryl system as defined herein, which satisfies the Hückel (4n+2) rule and/or with a stability due to delocalization significantly greater than that of a hypothetic localized structure.

As used herein, the terms "heterocycle" or "heterocyclic" refer to a saturated, partially unsaturated or unsaturated, aromatic or non aromatic stable 3 to 14, preferably 5 to 10 membered mono, bi or multicyclic rings wherein at least one member of the ring is a hetero atom. Typically, heteroatoms include, but are not limited to, oxygen, nitrogen, sulfur, selenium, and phosphorus atoms. Preferable heteroatoms are oxygen, nitrogen and sulfur. The nitrogen and sulfur heteroatoms may be optionally oxidized, and the nitrogen may be optionally substituted in nonaromatic rings. The bonds connecting the endocyclic atoms of a heterocyclic group may be single, double, triple, or part of a fused aromatic moiety.

Heterocycles are intended to include non aromatic heterocyclic ("heterocyclyl") and aromatic heterocyclic ("heteroaryl") compounds.

Examples of heterocycles include, but are not limited to oxiranyl, aziridinyl, tetrahydrofuranyl, 1,2-dioxolanyl, 1,3-dioxolanyl, 1,4-dioxolanyl, tetrahydro-pyranyl, 1,2-dioxanyl, 1,3-dioxanyl, 1,4-dioxanyl, tetrahydro-thiophenyl, tetrahydrothiopyran, 1,2-di-thiolanyl, 1,3-dithiolanyl, 1,2-dithianyl, 1,3-dithianyl, 1,4-dithianyl, tetrahydrothiopyranyl, thiomorpholinyl, thiazolidinyl, oxiranyl, pyrrolidinyl, 2-pyrrolidinyl, pirazolidinyl, piperidyl, 4-piperidinyl, morpholino, morpholinyl, piperazinyl, imidazolidinyl, pyranyl, dihydrofuranyl, dihydropyranyl, imidazolinyl, pyrrolinyl, pirazolinyl, tetrahydropyridyl, dihydropyridyl, tetrahydropyrimidinyl, dihydrothiophenyl, dihydrothiopyranyl, furanyl, pyrrolyl, 2H-pyrrolyl, imidazolyl, pyrazolyl, thienyl, oxadiazolyl, 1,2,3-oxadiazolyl, 1,2,4-oxadiazolyl, 1,2,5-oxadiazolyl, 1,3,4-oxadiazolyl, oxazolyl, isoxazolyl, furazanyl, 1,2,3-thiadiazolyl, 1,2,4-thiadiazolyl, 1,2,5-thiadiazolyl, 1,3,4-thiadiazolyl, thiazolyl, isothiazolyl, pyridyl, pyrazinyl, pyrimidinyl, pyridazinyl, indolizinyl, indolyl, indazolyl, purinyl, quinolizinyl, quinolyl, isoquinolyl, phthalazinyl, naphthyridinyl, quinoxalinyl, quinazolinyl, cinnolinyl, pteridinyl, oxazolinyl, 6H-1,2,5-thiadiazinyl, 2*H*,6*H*-1,5,2-dithiazinyl, oxazolidinyl, piperidonyl, 6*H*-1,2,5-thiadiazinyl, thienothiazolyl, thienooxazolyl, thienoimidazolyl, triazinyl, 1,2,3-triazolyl, 1,2,4-triazolyl, 1,2,5-triazolyl, 1,3,4-triazolyl, and tetrazole, as well as the systems formed by their condensation or the condensation with a phenyl group. Suitable heterocycles are also disclosed in The Handbook of Chemistry and Physics, 76th Edition, CRC Press, Inc., 1995-1996, pages 2-25 to 2-26, the disclosure of which is hereby incorporated by reference.

Preferred heterocyclic groups formed with a nitrogen atom include, but are not limited to, pyrrolidinyl, pyrrolyl, pirazolyl, pirazolidinyl, piperazinyl, imidazolidinyl, pyrrolinyl, pirazolinyl, pyridyl, piperidyl, piperidino, morpholinyl, morpholino, thiomorpholino, N-methylpiperazinyl, indolyl, isoindolyl, imidazolyl, imidazolinyl, oxazoline, oxazole, triazole, thiazoline, thiazole, isothiazole, thiadiazoles, triazines, isoxazole, oxindole, indoxyl, pyrazole, pyrazolone, pyrimidine, pyrazine, quinoline, iosquinoline, and tetrazole groups.

Preferred heterocyclic groups formed with an oxygen atom include, but are not limited to, furan, tetrahydrofuran, pyran, benzofurans, isobenzofurans, and tetrahydropyran groups. Preferred heterocyclic groups formed with a sulfur atom include, but are not limited to, thiophene, thianaphthene, tetrahydrothiophene, tetrahydrothiapyran, and benzothiophenes.

Preferred non aromatic heterocyclic, herein called heterocyclyl groups include, but are not limited to oxiranyl, tetrahydrofuranyl, dioxolanyl, tetrahydropyranyl, dioxanyl, pyrrolidinyl, piperidyl, morpholinyl, imidazolidinyl, pyranyl, imidazolinyl, pyrrolinyl, pyrazolinyl.

Preferred aromatic heterocyclic, herein called heteroaryl groups include, but are not limited to, pyridyl, pyridyl-N-oxyde, pyrimidinyl, pyrrolyl, furanyl, thienyl, imidazolyl, triazolyl, tetrazolyl, quinolyl, isoquinolyl, benzoimidazolyl, thiazolyl, pyrazolyl, and benzothiazolyl groups.

As used herein, the "heterocyclyl" groups refer to a non aromatic saturated or unsaturated heterocyclic ring which is formed by removal of a hydrogen atom.

As used herein, the term "aryl" refers to an aromatic carbo, mono-, bi-or multicyclic hydrocarbon ring containing from 6 to 14, preferably 6 to 10 carbon atoms, which is formed by removal of one hydrogen atom. Examples include phenyl, naphthyl, indenyl, etc.

As used herein, the term "heteroaryl" refers to a 5 to 14, preferably 5 to 10 membered aromatic hetero, mono-, bi- or multicyclic ring, which is formed by removal of one hydrogen atom. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, etc.

"Alkyl", "cycloalkyl", "aryl", "heteroaryl", "heterocycle" refers also to the corresponding "alkylene", "cycloalkylene", "arylene", "heteroarylene", "heterocyclene" which are formed by the removal of two hydrogen atoms.

As used herein, "Hal" refers to a halogen atom, including fluoro, chloro, iodo, bromo.

As used herein, the term "subject" refers to a warm blooded animal such as a mammal, preferably a human, or a human child, which is afflicted with, or has the potential to be afflicted with one or more diseases and conditions described herein.

As used herein, a "therapeutically effective amount" refers to an amount of a compound of the present invention which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, sulfuric, sulfamic, phosphoric, nitric and the like; and the salts prepared from organic acids such as acetic, propionic, succinic, tartaric, citric, glutamic, benzoic, salicylic, toluenesulfonic, oxalic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propanoic, succinic, tartaric, citric, methanesulfonic, benzenesulfonic, glucuronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium. Hydrochloride and oxalate salts are preferred.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

The compounds of the general formula (I) having geometrical and stereomers are also a part of the invention.

According to a further object, the present invention is also concerned with the process of preparation of the compounds of formula (I).

The compounds and process of the present invention may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, such methods can be found in R.C. Larock, Comprehensive Organic Transformations, VCH publishers, 1989.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, John Wiley and Sons, 1991; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, methylcyclohexane, toluene and xylene; amides, such as *N*,*N*-dimethylformamide; alcohols such as ethanol and methanol and ethers, such as diethyl ether, methyl *tert-*butyl ether, methyl cyclopentyl ether and tetrahydrofuran.

The reactions can take place over a wide range of temperatures. In general, we find it convenient to carry out the reaction at a temperature of from -78°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of up to 20 hours will usually suffice.

The compound thus prepared may be recovered from the reaction mixture by conventional means. For example, the compounds may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

The process of preparation of a compound of formula (I) of the invention is another object of the present invention.

According to a first aspect, compounds of the invention of the formula (I) where R1 and R2 form together with the C atom to which they are attached a C=O group, can be obtained by reacting corresponding compounds of formula (II) with corresponding compounds of formula (III), or a precursor thereof : 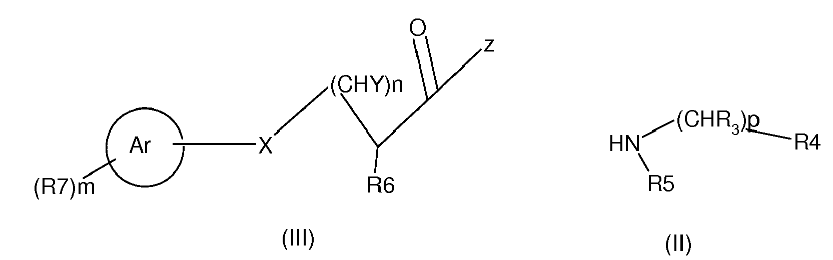 where R7, Ar, X, Y, R6, R5, R3, R4, m, n, p are defined as in formula (I) and Z is either a halogen atom or a OH group, optionally followed by the functionalization of the obtained compound, if appropriate.

Generally, the coupling reaction is carried out in an organic, aprotic solvent, such as dichloromethane or DMF, at room temperature, in the presence of coupling agents, such as DMAP and/or DCI.

Precursor thereof is used herein to refer to compounds which differ from the indicated or desired compounds by the presence and/or absence of functions. Such functions may be introduced, transformed and/or omitted by common functionalization reactions, known from the skilled person.

The functionalization reaction may be carried out by application or adaptation of known methods.

According to a second aspect, compounds of the invention of the formula (I) where X represents a -NT- group can be obtained by reacting corresponding compounds of formula (IV) with corresponding compounds of formula (V), or a precursor thereof: 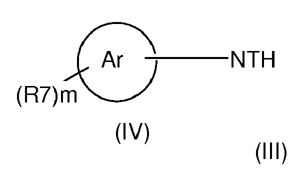 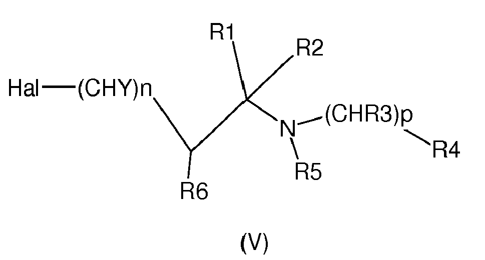 where R7, Ar, X, Y, R6, R5, R1, R2, R3, R4, T, m, n, p are defined as in formula (I) and Hal is a halogen atom, optionally followed by the functionalization of the obtained compound, if appropriate.

Generally, the coupling reaction is carried out in a solvent, such as acetonitrile, at a temperature comprised between 50°C and 150°C in a microwave, in the presence of a reagent, such as KI.

Said compounds of formula (II), (III), (IV) or (V) are commercially available or may be synthesized by applying or adaptating any known method, such as those described in the examples.

The process of the invention also comprises the additional step of isolating said desired compound of formula (I).

According to a still further aspect, the present invention also provides pharmaceutical compositions comprising at least one compound of the present invention of formula (I) as defined below, 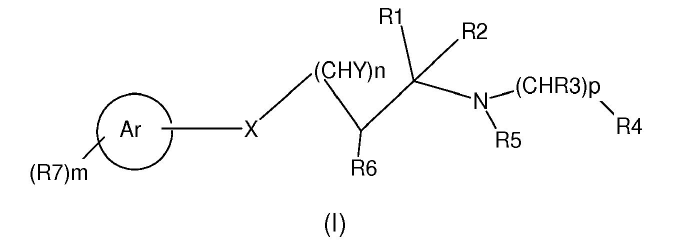 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O) R;
Or a R3 form with a substituent of R4 a cycloalkyl, aryl, heteroaryl or heterocycle fused with said R4;
Or a R3 form with R5 a N-containing heterocyle or heteroaryl;
R4 represents an aryl, heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
   - halogen atom; -Alkyl; -Aryl; -OAryl; -Alkyl-OR;
   - Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
   - C(=O)R; -C(=O)NRR'; -O-Alkyl-C(=O)NRR', -O-Alkyl-C(=O)OR, -OAlkyl-NRR', -NR-C(=O)R'; -C(=O)OR; -S(O)R, -S(O)₂R; -OC(=O)R; -OAlkenyl;
   - Alkenyl;
   - Heterocycle optionally substituted by an -Alkyl, -AlkylOR, -AlkylNRR',
   - Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally comprising one or more further heteroatom and optionally substituted by a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 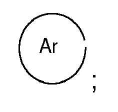
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 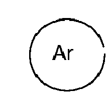
represents an aryl, heteroaryl or unsaturated heterocycle;
m is an integer comprised between 1 and 5, provided that when 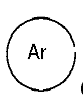 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts, with the exception of the following compounds :
- the compounds where Ar is a tetrazol, X is S, n is 2, R6 is H, R1 and R2 form together a C=O with the carbon atom to which they are attached; and
- the compounds of formulae : 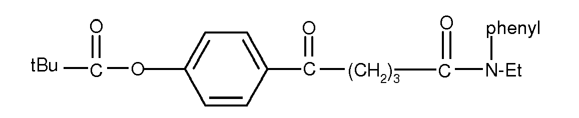 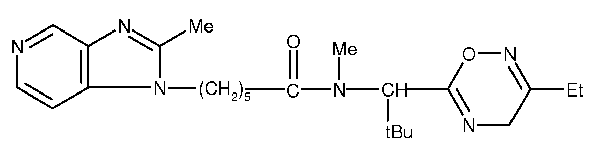 and a pharmaceutically acceptable carrier.

According to a further object, the present invention also provides a compound of formula (I) 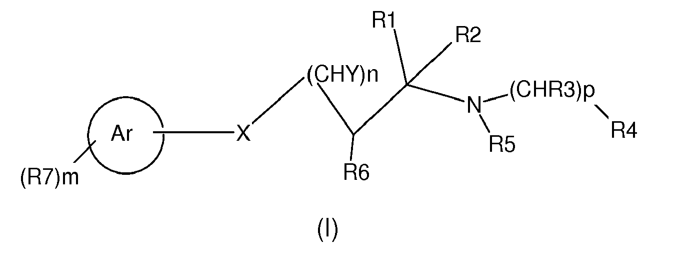 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O) R;
Or a R3 form with a substituent of R4 a cycloalkyl, aryl, heteroaryl or heterocycle fused with said R4;
Or a R3 form with R5 a N-containing heterocyle or heteroaryl;
R4 represents an aryl, heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
   - halogen atom; -Alkyl; -Aryl; -OAryl; -Alkyl-OR;
   - Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
   - C(=O)R; -C(=O)NRR'; -O-Alkyl-C(=O)NRR', -O-Alkyl-C(=O)OR, -OAlkyl-NRR', -NR-C(=O)R'; -C(=O)OR; -S(O)R, -S(O)₂R; -OC(=O)R; -OAlkenyl;
   - Alkenyl;
   - Heterocycle optionally substituted by an -Alkyl, -AlkylOR, -AlkylNRR',
   - Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally comprising one or more further heteroatom and optionally substituted by a group chosen from halogen atom,
-alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 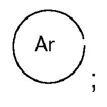
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 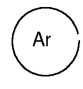 represents an aryl, heteroaryl or unsaturated heterocycle;
m is an integer comprised between 1 and 5, provided that when 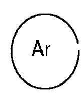 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts.
for inhibiting integration of viruses into the host genom.

According to a further object, the present invention also provides a compound of formula (I) as defined above for treating and/or preventing viral infections and/or disorders, caused by retroviruses such as HIV and AIDS-related complex, persistent generalised lymphadenopathy (PGL), Kaposi's sarcoma, AIDS dementia complex (or AIDS related disorders), Avian sarcoma and leukosis viral group, Mammalian B-type viral group, Murine leukemia-related viral group, Human T-cell leukemia and bovine leukemia viral group, D-type viral group, Lentiviruses and Spumaviruses; and for example Rous sarcoma virus, mouse mammary tumor virus, Moloney murine leukemia virus, human T-cell leukemia virus, Mason-Pfizer monkey virus, human immunodeficiency virus, human foamy virus.

The present invention also concerns the corresponding methods for inhibiting integration, treating and/or preventing viral infections and/or disorders, such as in a subject in need thereof, said method comprising administering to said subject a therapeutically effective amount of a compound of formula (I) as defined above.

The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect will vary depending upon a number of factors, including the dosage of the drug to be administered, the chemical characteristics (e.g., hydrophobicity) of the compounds employed, the potency of the compounds, the type of disease, the diseased state of the patient, and the route of administration.

In general terms, the compounds of this invention may be provided in an aqueous physiological buffer solution containing about 0.1 to 10% w/v compound for parenteral administration. Typical dose ranges are from about 1 µg/kg to about 1 g/kg of body weight per day; a preferred dose range is from about 0.01 mg/kg to 100 mg/kg of body weight per day. A preferred daily dose for adult humans includes about 25, 50, 100, 200 and 400 mg, and an equivalent dose in a human child. The preferred dosage of drug to be administered is likely to depend on such variables as the type and extent of progression of the disease or disorder, the overall health status of the particular patient, the relative biological efficacy of the compound selected, and formulation of the compound excipient, and its route of administration.

The compounds of the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical daily dose ranges are from about 0.1 to 100 mg/kg of body weight. By way of general guidance, unit doses for humans range from about 0.1 mg to about 1000 mg per day. Preferably the unit dose range is from about 1 to about 500 mg administered one to four times a day, and even more preferably from about 10 mg to about 300 mg, two times a day. Compounds provided herein can be formulated into pharmaceutical compositions by admixture with one or more pharmaceutically acceptable excipients. Such compositions may be prepared for use in oral administration, particularly in the form of tablets or capsules; or parenteral administration, particularly in the form of liquid solutions, suspensions or emulsions; or intranasally, particularly in the form of powders, nasal drops, or aerosols; or dermally, for example, topically or via trans-dermal patches.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents, and/or adjuvant materials can be included as part of the composition. Oral compositions will generally include an inert diluent carrier or an edible carrier.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Preferred formulations include pharmaceutical compositions in which a compound of the present invention is formulated for oral or parenteral administration, or more preferably those in which a compound of the present invention is formulated as a tablet. Preferred tablets contain lactose, cornstarch, magnesium silicate, croscarmellose sodium, povidone, magnesium stearate, or talc in any combination. It is also an aspect of the present disclosure that a compound of the present invention may be incorporated into a food product or a liquid.

Liquid preparations for administration include sterile aqueous or nonaqueous solutions, suspensions, and emulsions. The liquid compositions may also include binders, buffers, preservatives, chelating agents, sweetening, flavoring and coloring agents, and the like. Nonaqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The compounds of the current invention can be employed as the sole active ingredient in a pharmaceutical composition. Alternatively, they can be used in combination or combined with other pharmaceutical agents associated with the same or other disease states. In particular, the compounds of formula (I), can be combined with agents that are useful for the treatment of HIV, including reverse transcriptase or protease inhibitors. The present invention encompasses, therefore, combinations of the compounds of the current invention with agents or pharmaceutical compositions known to be prescribed or effective with regard to such conditions.

Said ingredients can be administered simultaneously or separately.

The compounds of the current invention and their pharmaceutically acceptable derivatives may be employed in combination with other therapeutic agents, but not limited to, such as :
(1-alpha,2-beta,3-alpha)-9-[2,3-bis(hydroxymethyl)cyclobutyl]guanine [(-)BHCG, SQ-34514, lobucavir]; 9-[(2R,3R,4S)-3,4-bis(hydroxyl methyl)-2-oxetanosyl]-adenine (oxetanocin-G); acyclic nucleosides, for example acyclovir, valaciclovir, famciclovir, ganciclovir, and penciclovir; acyclic phosphonates, for example (S)-1-(3-hydroxy-2-phosphonyl-methoxypropyl) cytosine (HPMPC, cidofovir), [[[2-(6-amino-9H-purin-9-yl)ethoxy]methyl]phosphinylidene]bis(oxymethylene)-2,2-dimethyl propanoic acid (bis-POM PMEA, adefovir dipivoxil), [[(1R)-2-(6-amino-9H-purin-9-yl)-1-methylethoxy]methyl]phosphonic acid (tenofovir), and (R)-[[2-6-amino-9H-purin-9-yl)-1-methylethoxy]methyl] phosphonic acid bis-(isopropoxycarbonyloxymethyl)ester (bis-POC-PMPA); ribonucleotide reductase inhibitors, for example 2-acetylpyridine-5-[(2-chloranilino)thiocarbonyl] thiocarbonohydrazone and hydroyurea; nucleoside reverse transcriptase inhibitors, for example 3'-azido-3'-deoxythymidine (AZT, zidovudine), 2',3'-dideoxycytidine (ddC, zalcitabine), 2',3'-dideoxyadenosine, 2',3'-didexyinosine (ddl, didanosine), 2',3'-didehydrothymidine (d4T, stavudine), (-)-beta-D-2,6-diaminopurine dioxolane (DAPD), 3'-azido-2',3'-dideoxythymidine-5'-H-phosphophonate (phosphonovir), 2'-deoxy-5-iodo-uridine (idoxuridine), (-)-cis-1-(2-hydroxymethyl)-1,3-oxathiolane-5-yl)-cytosine (lamivudine), cis-1-(2-(hydroxymethyl)-1,3-oxathiolan-5-yl)-5-fluorocytosine (FTC), 3'-deoxy-3'-fluorothymidine, 5-chloro-2',3'-dideoxy-3'-fluorouridine, (-)-cis-4-[2-amino-6-(cyclopropylamino)-9H-purin-9-yl] -2-cyclopentene-1-methanol (abacavir), 9-[4-hydroxy-2-(hydroxymethyl)but-1-yl]-guanine (H2G), ABT-606 (H2G progrug, valomaciclovir) and ribavirin; protease inhibitors, for example indinavir, ritonavir, nelfinavir, amprenavir, saquinavir, fosamprenavir, (R)-N-tert-butyl-3-[(2S,3S)-2-hydroxy-3-N-[(R)-2-N-(isoquinolin-5-yloxyacetyl)amino-3-methylthio-propanoyl]-amino-4-phenylbutanoyl]-5,5-dimethyl-1,3- thiazolidine-4-carboxamide (KNI-272), (4R,5S,6S,7R)-1,3-bis(3-aminobenzyl)-4,7-dibenzylhexahydro-5,6-dihydroxy-2H-1,3-diazepin-2-one dimethanesulfonate (mozenavir, DMP-450), methyl N-[(2S)-1-[[(2S,3S)-3-hydroxy-4-[[[(2S)-2-(methoxycarbonylamino)-3,3-dimethylbutanoyl] amino]-[(4-pyridin-2-ylphenyl)methyl]amino]-1-phenylbutan-2-yl]amino]-3,3-dimethyl-1-oxobutan-2-yl]carbamate (BMS-232632, atazanavir), 3-(2(S)-Hydroxy-3(S)-(3-hydroxy-2-methylbenzamido)-4-phenylbutanoyl)-5,5-dimethyl-N-(2-methylbenzyl)thiazolidine-4(R)-carboxamide (AG-1776), N-(2(R)- hydroxy- 1(S)-indanyl)-2(R)-phenyl-methyl-4(S)-hydroxy-5-(1 -(1-(4- benzo[b]furanylmethyl)-2(S)-N'-(tert-butyl carboxamido)piperazinyl)pentanamide (MK-944A); interferons such as α-interferon; renal excretion inhibitors such as probenecid; nucleoside transport inhibitors such as dipyridamole, pentoxifylline, N-acetylcysteine (NAC), Procysteine, α-trichosanthin, phosphonoformic acid; as well as immunomodulators such as interleukin II or thymosin, granulocyte macrophage colony stimulating factors, erythropoetin, soluble CD₄ and genetically engineered derivatives thereof; non-nucleoside reverse transcriptase inhibitors (NNRTIs), for example nevirapine (BI-RG-587), alpha-((2-acetyl-5- methylphenyl)amino)-2,6-dichloro-benzeneacetamide (loviride), 1-[3-(isopropyl amino)-2-pyridyl]-4-[5-(methanesulfonamido)-1H-indol-2-ylcarbonyl]piperazine monomethanesulfonate (delavirdine), (10R, 11S, 12S)-12-Hydroxy-6, 6, 10, 11-tetramethyl-4-propyl-11,12-dihydro-2H, 6H, 10H-benzo(1, 2-b:3, 4-b':5, 6-b")tripyran-2-one ((+)-calanolide A), (4S)-6-Chloro-4-[1-(E)-cyclopropyl ethenyl)-3,4- dihydro-4-(trifluoromethyl)-2(1H)-quinazolinone (DPC-083), (S)-6-chloro-4-(cyclopropyl ethynyl)-1,4-dihydro-4-(trifluoromethyl)-2H-3,1-benzoxazin-2-one (efavirenz, DMP 266), 1-(ethoxy methyl)-5-(1- methylethyl)-6-(phenylmethyl)-2,4(1H,3H)-pyrimidinedione (MKC-442), and 5-(3,5-dichloro phenyl)thio-4-isopropyl-1-(4-pyridyl)methyl-(1H)-imidazol-2-ylmethyl carbamate (capravirine); glycoprotein 120 antagonists, for example PRO-2000, PRO-542 and 1,4-bis[3-[(2,4-dichlorophenyl)carbonylamino]-2-oxo-5,8-disodiumsulfanyl]-naphthalyl-2,5-dimethoxyphenyl-1,4-dihydrazone (FP-21399); cytokine antagonists, for example reticulose (Product-R), 1,1'-azobis-formamide (ADA), 1,11-(1,4-phenylenebis(methylene))bis-1,4,8,11-tetraazacyclotetradecane octahydrochloride (AMD-3100); integrase inhibitors, for example raltegravir and elvitegravir; and fusion inhibitors, for example T-20 and T- 1249.

### EXAMPLES

The following examples are given for illustration of the invention and are not intended to be limited thereof.
s : singlet
brs : broad singlet
d : doublet
brd : broad doublet
t : triplet
q : quadruplet
quint : quintuplet
dd : doubled doublet
dt : doubled triplet
dq : doubled quadruplet
sept : septuplet
m : massif

Commercial compounds were purchased from Acros Organics, Sigma-Aldrich, Alfa Aesar, Chembridge and Maybridge.

CAS numbers of other building blocks are indicated in brackets.

### General procedure X : typical synthesis of acids :

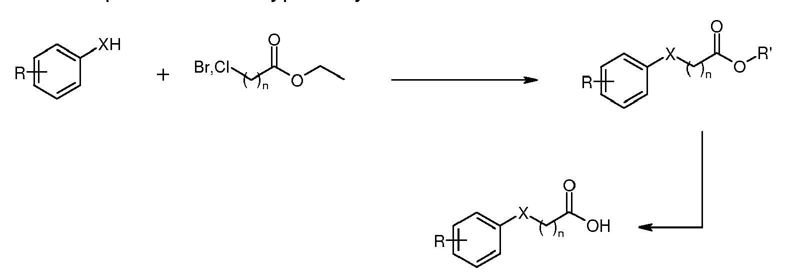

*Step 1 : To* a solution of the starting material (1 eq) in acetonitrile (1.3 mL / mmol) were added anhydrous potassium carbonate (1.5eq) and the halogeno-ester (1.1eq). The mixture was refluxed overnight (the reaction was monitored by TLC), then water was added. The mixture was extracted 3 times with ethyl acetate, the organic layers were combined, dried on anhydrous MgSO₄, filtered and concentrated in vacuum to give the ester which was possibly purified by flash chromatography on silica gel.
*Step 2 :* To a solution of the ester (1eq) in dioxane (2 mL / mmol) was added NaOH 3N (4eq). The mixture was stirred at room temperature or reflux ; the reaction was monitored by TLC. Then the mixture was evaporated in vacuum and acidified to pH=1 by addition of HCl 4N. The precipitated was filtered and dried to give the acid.

### General procedure Y : typical synthesis of benzylamines using reductive amination

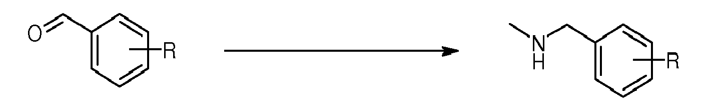

To a solution of the aldehyde (1 eq) in methanol (2 mL / mmol) were added a solution of methylamine in methanol (2eq) and titanium (IV) isopropoxide (1.3eq). The mixture was stirred 5 hours at room temperature and sodium borohydride (1 eq) was added. The mixture was then stirred 2 hours and water was added. Salts were filtered and the filtrate was extracted 3 times with diethyl ether. The organic layers were combined, washed with brine, dried on anhydrous MgSO₄, filtered and concentrated in vacuum to give the benzylamine which was used without purification.

### General procedure Z : typical synthesis of amines by nucleophilic substitution of 2-fluorobenzaldehyde

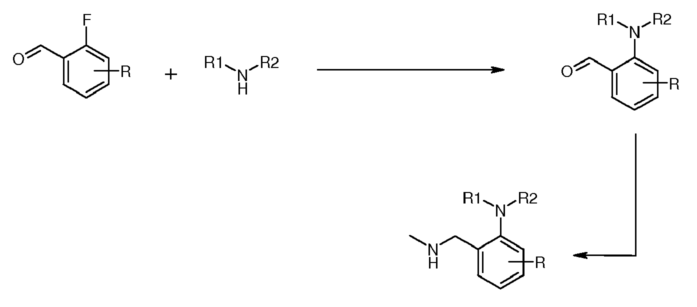

To a solution of the amine (1eq) in N,N-dimethylformamide were added 2-fluorobenzaldehyde (1eq) and anhydrous potassium carbonate (1.5eq). The mixture were refluxed overnight (the reaction was monitored by TLC), then water was added. The mixture was extracted 3 times with ethyl acetate, the organic layers were combined, dried on anhydrous MgSO₄, filtered and concentrated in vacuum. The residue was purified by flash chromatography on silica gel to give the 2-aminobenzaldehyde.

The benzylamine derivative was then prepared by reductive amination of the aldehyde according general procedure Y.

### General procedure A

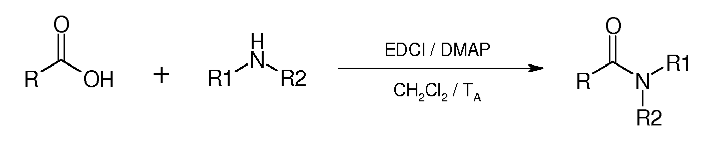

To a solution of acid (1eq) and amine (1.1eq) in dichloromethane were added 1-(3-dimethylaminopropyl)-3-ethylcarbodiimide hydrochloride (1.1eq) and N,N-dimethylaminopyridine (1.1eq). The solution was stirred 24 hours at room temperature and a saturated solution of NaHCO₃ was added. The mixture was extracted 3 times with ethyl acetate, the organic layers were combined and washed with HCl 1N, dried over anhydrous MgSO₄ and concentrated under vacuum. The residue was purified by flash chromatography (silica gel) with the appropriate gradient determined by TLC.

The following compounds were prepared according general procedure A :
***Example 1 :***
   N-(3-Chlorobenzyl)-4-[(4-hydroxyphenyl)thio]-N-methylbutanamide
   Prepared from 4-[(4-hydroxyphenyl)thio]butanoic acid [85896-82-8] and commercial 3-chloro-N-methylbenzylamine in 35% yield.
   NMR-¹H (CDCl₃) : δ (ppm) 7.34-7.17 (m, 3H) ; 7.17-6.99 (m, 1H) ; 6.83-6.67 (m, 2H) ; 4.53 (d, 2H) ; 2.94 (d, 3H) ; 2.86 (dt, 2H) ; 2.53 (q, 2H) ; 1.97 (quint, 2H)
   MS (ESI+) : *m*/*z* = 350 [M+H]⁺
***Example 2 :***
   N-(4-Fluorobenzyl)-4-[(4-hydroxyphenyl)thio]-N-methylbutanamide
   Prepared from 4-[(4-hydroxyphenyl)thio]butanoic acid [85896-82-8] and commercial 4-fluoro-N-methylbenzylamine in 46% yield.
   NMR-¹H (CDCl₃) : δ (ppm) 7.29-6.90 (m, 6H) ; 6.77-6.65 (m, 2H) ; 4.49 (d, 2H) ; 2.94-2.76 (m, 5H) ; 2.50 (t, 2H) ; 2.01-1.85 (m, 2H)
   MS (ESI+) : *m*/*z* = 334 [M+H]⁺
***Example 3 :***
   N-(2-Chlorobenzyl)-4-[(4-hydroxyphenyl)thio]-N-methylbutanamide
   Prepared from 4-[(4-hydroxyphenyl)thio]butanoic acid [85896-82-8] and commercial 2-chloro-N-methylbenzylamine in 81 % yield.
   NMR-¹H (CDCl₃) : δ (ppm) 7.47-7.00 (m, 6H) ; 6.81-6.68 (m, 2H) ; 4.66 (d, 2H) ; 3.05-2.77 (m, 5H) ; 2.51 (dt, 2H) ; 2.06-1.86 (m, 2H)
   MS (ESI+) : *m*/*z* = 350 [M+H]⁺
***Example 4 :***
   N-(4-Chlorobenzyl)-4-[(4-hydroxyphenyl)thio]-N-methylbutanamide
   Prepared from 4-[(4-hydroxyphenyl)thio]butanoic acid [85896-82-8] and commercial 4-chloro-N-methylbenzylamine in 59% yield.
   NMR-¹H (CDCl₃) : δ (ppm) 7.41-7.02 (m, 6H) ; 6.75 (dd, 2H) ; 4.52 (d, 2H) ; 2.99-2.76 (m, 5H) ; 2.59-2.42 (m, 2H) ; 2.05-1.87 (m, 2H)
   MS (ESI+) : *m*/*z* = 350 [M+H]⁺
***Example 5 :***
   4-[(4-Hydroxyphenyl)thio]-N-methyl-N-[(1S)-1-phenylethyl]butanamide
   Prepared from 4-[(4-hydroxyphenyl)thio]butanoic acid [85896-82-8] and commercial (R)-(+)-N-methyl-1-phenylethylamine in 11 % yield.
   NMR-¹H (CDCl₃) : δ (ppm) 7.33-7.13 (m, 7H) ; 6.73-6.69 (m, 2H) ; 5.99 (q, 1H) ; 2.88-2.81 (m, 2H) ; 2.60-2.57 (m, 3H) ; 2.42 (t, 2H) ; 1.98-1.87 (m, 2H) ; 1.51 (d, 3H)
   MS (ESI+) : *m*/*z* = 330 [M+H]⁺
***Example 6 :***
   4-[(4-Hydroxyphenyl)thio]-N-(4-methoxybenzyl)-N-methylbutanamide
   Prepared from 4-[(4-hydroxyphenyl)thio]butanoic acid [85896-82-8] and commercial 4-methoxy-N-methylbenzylamine in 57% yield.
   NMR-¹H (CDCl₃) : δ (ppm) 7.22-6.99 (m, 3H) ; 6.84-6.68 (m, 5H) ; 4.42 (d, 2H) ; 3.73 (d, 3H) ; 2.85-2.65 (m, 5H) ; 2.47 (q, 2H) ; 1.96-1.82 (m, 2H)
   MS (ESI+) : *m*/*z* = 346 [M+H]⁺
***Example 7 :***
   4-[(4-Hydroxyphenyl)thio]-N-methyl-N-[4-(trifluoromethyl)benzyl]butanamide
   Prepared from 4-[(4-hydroxyphenyl)thio]butanoic acid [85896-82-8] and commercial N-methyl-4-(trifluoromethyl)benzylamine in 50% yield.
   NMR-¹H (CDCl₃) : δ (ppm) 7.60-7.48 (m, 2H) ; 7.27-7.14 (m, 4H) ; 6.77-6.65 (m, 2H) ; 4.53 (d, 2H) ; 2.88-2.78 (m, 5H) ; 2.50-2.39 (m, 2H) ; 1.97-1.84 (m, 2H)
   MS (ESI+) : *m*/*z* = 384 [M+H]⁺
***Example 8 :***
   4-[(4-Hydroxyphenyl)thio]-N-methyl-N-(4-methylbenzyl)butanamide
   Prepared from 4-[(4-hydroxyphenyl)thio]butanoic acid [85896-82-8] and commercial 4,N-dimethylbenzylamine in 50% yield.
   NMR-¹H (CDCl₃) : δ (ppm) 7.30-7.02(m, 6H) ; 6.78-6.73 (m, 2H) ; 4.51 (d, 2H) ; 2.93-2.82 (m, 5H) ; 2.55-2.48 (m, 2H) ; 2.34 (d, 2H) ; 2.03-1.88 (m, 2H)
   MS (ESI+) : *m*/*z* = 330 [M+H]⁺
***Example 9 :***
   4-[(4-Hydroxyphenyl)thio]-N-methyl-N-phenylbutanamide
   Prepared from 4-[(4-hydroxyphenyl)thio]butanoic acid [85896-82-8] and commercial N-methylaniline in 50% yield.
   NMR-¹H (CDCl₃) : δ (ppm) 7.39- 7.29 (m, 3H) ; 7.17- 7.08 (m, 4H) ; 6.69 (d, 2H) ; 3.19 (s, 3H) ; 2.67 (t, 2H) ; 2.13 (t, 2H) ; 1.78 (m, 2H)
   MS (ESI+) : *m*/*z* = 302 [M+H]⁺
***Example 10 :***
   N-Benzhydryl-4-[(4-hydroxyphenyl)thio]-N-methylbutanamide
   Prepared from 4-[(4-hydroxyphenyl)thio]butanoic acid [85896-82-8] and commercial N-methylbenzhydrylamine in 5% yield.
   NMR-¹H (CDCl₃) : δ (ppm) 7.37-7.15 (m, 13H) ; 6.74 (d, 2H) ; 2.94-2.72 (m, 5H) ; 2.63-2.54 (m, 2H) ; 2.06-1.93 (m, 2H)
   MS (ESI+) : *m*/*z* = 392 [M+H]⁺

### General procedure B

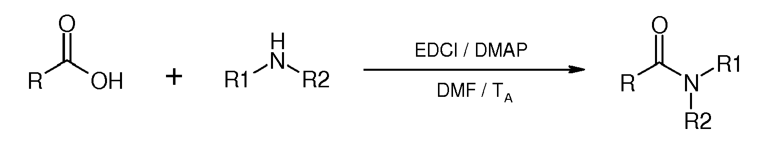

To a solution of the acid (1eq) and the amine (1eq) in N,N-dimethylformamide were added 1-(3-dimethylaminopropyl)-3-ethylcarbodiimide hydrochloride (1 eq) and N,N-dimethylaminopyridine (1 eq). The solution was stirred overnight at room temperature and a saturated solution of NaHCO₃ was added. The mixture was extracted 3 times with ethyl acetate, the organic layers were combined and washed with HCl 1N, dried over anhydrous MgSO₄ and concentrated under vacuum. The residue was purified by flash chromatography (silica gel) with the appropriate gradient determined by TLC.

The following compounds were prepared according general procedure B :
***Example 11 :***
   4-[(4-Hydroxyphenyl)thio]-N-methyl-N-(2-morpholin-4-ylbenzyl)butanamide
   Prepared from 4-[(4-hydroxyphenyl)thio]butanoic acid [85896-82-8] and N-methyl-2-(morpholin-4-yl)benzylamine [871217-44-6] in 50% yield.
   NMR-¹H (CDCl₃) : δ (ppm) 7.29-7.08 (m, 6H) ; 6.77 (t, 2H) ; 4.67 (d, 2H) ; 3.93-3.78 (m, 4H) ; 2.99 (d, 3H) ; 2.96-2.82 (m, 4H) ; 2.80 (t, 2H) ; 2.53 (dt, 2H) ; 2.05-1.89 (m, 2H)
   MS (ESI+) : *m*/*z* = 401 [M+H]⁺
***Example 12 :***
   5-[(4-Hydroxyphenyl)thio]-N-(2-methoxybenzyl)-N-methylpentanamide
   Prepared from 4-[(4-hydroxyphenyl)thio]pentanoic acid (see below) and commercial 2-methoxy-N-methylbenzylamine in 65% yield.
   NMR-¹H (CDCl₃) : δ (ppm) 7.31-7.13 (m, 3H) ; 7.01-6.76 (m, 5H) ; 4.55 (d, 2H) ; 3.82 (d, 3H) ; 2.94 (s, 3H) ; 2.83-2.72 (m, 2H) ; 2.36 (t, 2H) ; 1.83-1.68 (m, 2H) ; 1.66-1.48 (m, 4H)
   MS (ESI+) : *m*/*z* = 360 [M+H]⁺
   4-[(4-Hydroxyphenyl)thio]pentanoic acid was prepared according general procedure X from commercial 4-hydroxythiophenol and methyl 5-pentanoic acid.
***Example 13 :***
   4-[(4-Hydroxyphenyl)thio]-N-methyl-N-[2-(4-methylpiperazin-1-yl)benzyl]-butanamide
   Prepared from 4-[(4-hydroxyphenyl)thio]butanoic acid [85896-82-8] and N-methyl-2-(4-methylpiperazin-1-yl)benzylamine (see below) in 58% yield.
   NMR-¹H (CDCl₃) : δ (ppm) 7.31-7.01 (m, 6H) ; 6.75 (dd, 2H) ; 4.64 (d, 2H) ; 3.03-2.85 (m, 7H) ; 2.81 (t, 2H) ; 2.66-2.53 (m, 4H) ; 2.51 (dt, 2H) ; 2.37 (d, 3H) ; 2.09-1.81 (m, 2H)
   MS (ESI+) : *m*/*z* = 414 [M+H]⁺
   N-Methyl-2-(4-methylpiperazin-1-yl)benzylamine was prepared according general procedure Y from commercial 2-(4-methylpiperazino)benzaldehyde.
***Example 14 :***
   N-(2-Chlorobenzyl)-4-[(4-fluorophenyl)thio]-N-methylbutanamide
   Prepared from 4-[(4-fluorophenyl)thio]butanoic acid [18850-56-1] and commercial 2-chloro-N-methylbenzylamine in 82% yield.
   NMR-¹H (CDCl₃) : δ (ppm) 7.45-7.13 (m, 4H) ; 7.10-6.89 (m, 2H) ; 4.65 (d, 2H) ; 3.07-2.86 (m, 5H) ; 2.55 (t, 1H) ; 2.43 (t, 1H) ; 2.10-1.86 (m, 2H).
   MS (ESI+) : *m*/*z* = 352 [M+H]⁺
***Example 15 :***
   4-[(4-Hydroxyphenyl)thio]-N-methyl-N-[2-(trifluoromethyl)benzyl]butanamide
   Prepared from 4-[(4-hydroxyphenyl)thio]butanoic acid [85896-82-8] and N-methyl-2-(trifluoromethyl)benzylamine [296276-41-0] in 38% yield.
   NMR-¹H (CDCl₃) : δ (ppm) 7.73-7.16 (m, 6H) ; 6.75 (t, 2H) ; 4.78 (d, 2H) ; 2.97 (d, 3H) ; 2.91 (t, 1H) ; 2.81 (t, 1H) ; 2.61 (t, 1H) ; 2.45 (t, 1H) ; 2.06-1.87 (m, 2H)
   MS (ESI+) : *m*/*z* = 384 [M+H]⁺
***Example 16 :***
   N-(2-Methoxybenzyl)-N-methyl-4-{[5-(trifluoromethyl)pyridin-2-yl]thio}butanamide
   Prepared from 4-[[5-(trifluoromethyl)-2-pyridinyl]thio]butanoic acid [1019352-70-5] and commercial 2-methoxy-N-methylbenzylamine in 79% yield.
   NMR-¹H (CDCl₃) : δ (ppm) 8.59 (br s, 1H) ; 7.62 (br d, 1H) ; 7.33-7.13 (m, 2H) ; 7.06-6.81 (m, 3H) ; 4.55 (d, 2H) ; 3.82 (d, 3H) ; 3.26 (dt, 2H) ; 2.93 (s, 3H) ; 2.52 (t, 2H) ; 2.09 (m, 2H)
   MS (ESI+) : *m*/*z* = 399 [M+H]⁺
***Example 17:***
   N-(2-Methoxybenzyl)-N-methyl-4-(2-naphthylthio)butanamide
   Prepared from 4-(2-naphthalenylthio)butanoic acid [5324-80-1] and commercial 2-methoxy-N-methylbenzylamine in 69% yield.
   NMR-¹H (CDCl₃) : δ (ppm) 7.84-7.67 (m, 4H) ; 7.52-7.33 (m, 3H) ; 7.34-7.08 (m, 1H) ; 7.05-6.82 (m, 3H) ; 4.54 (d, 2H) ; 3.82 (d, 3H) ; 3.13 (dt, 2H) ; 2.92 (s, 3H) ; 2.56 (t, 2H) ; 2.15-1.99 (m, 2H)
   MS (ESI+) : *m*/*z* = 380 [M+H]⁺
***Example 18 :***
   4-[(4-Hydroxyphenyl)thio]-N-methyl-N-(1-naphthylmethyl)butanamide
   Prepared from 4-[(4-hydroxyphenyl)thio]butanoic acid [85896-82-8] and N-methyl-1-naphthylmethylamine [14489-75-9] in 20% yield.
   NMR-¹H (DMSO) : δ (ppm) 9.53 (s, 1H) ; 8.09-7.82 (m, 3H) ; 6.62-7.41 (m, 3H) ; 7.31 (br d, 1H) ; 7.20 (d, 1H) ; 7.09 (br d, 1H) ; 6.76-6.63 (m, 2H) ; 5.00 (d, 2H) ; 3.87 (d, 3H) ; 2.79 (dt, 2H) ; 2.45 (dt, 2H) ; 1.84-1.63 (m, 2H)
   MS (ESI+) : *m*/*z* = 366 [M+H]⁺
***Example 19 :***
   5-(4-Hydroxyphenyl)-N-(2-methoxybenzyl)-N-methylpentanamide
   Prepared from commercial 5-(4-hydroxyphenyl)pentanoic acid and commercial 2-methoxy-N-methylbenzylamine in 27% yield.
   NMR-¹H (CDCl₃) : δ (ppm) 7.36-7.10 (m, 1H) ; 7.07-6.66 (m, 7H) ; 4.56 (d, 2H) ; 3.82 (d, 3H) ; 2.94 (s, 3H) ; 2.54 (dt, 2H) ; 2.39 (t, 2H) ; 1.81-1.51 (m, 4H)
   MS (ESI+) : *m*/*z* = 328 [M+H]⁺
***Example 20 :***
   4-{[4-(Acetylamino)phenyl]thio}-N-(2-methoxybenzyl)-N-methylbutanamide
   Prepared from 4-[[4-(acetylamino)phenyl]thio]butanoic acid [1016762-61-0] and commercial 2-methoxy-N-methylbenzylamine in 69% yield.
   NMR-¹H (CDCl₃) : δ (ppm) 8.54 (d, 1H) ; 7.43 (dd, 2H) ; 7.35-7.15 (m, 2H) ; 7.08 (d, 1H) ; 7.04-6.76 (m, 3H) ; 4.53 (d, 2H) ; 3.80 (d, 3H) ; 3.09-2.76 (m, 5H) ; 2.51 (t, 2H) ; 2.09 (s, 3H) ; 2.02-1.90 (m, 2H)
   MS (ESI+) : *m*/*z* = 387 [M+H]⁺
***Example 21 :***
   4-[(2-Hydroxyphenyl)thio]-N-(2-methoxybenzyl)-N-methylbutanamide
   Prepared from 4-[(2-hydroxyphenyl)thio]butanoic acid [1004781-54-7] and commercial 2-methoxy-N-methylbenzylamine in 41% yield.
   NMR-¹H (CDCl₃) : δ (ppm) 7.42 (ddd, 1H) ; 7.33-7.11 (m, 2H) ; 7.05-6.76 (m, 5H) ; 4.55 (d, 2H) ; 3.82 (d, 3H) ; 2.93 (d, 3H) ; 2.8 (dt, 2H) ; 2.48 (t, 2H) ; 2.10-1.78 (m, 2H) ;
   MS (ESI+) : *m*/*z* = 346 [M+H]⁺
***Example 22 :***
   N-(2,3-Dihydro-1-benzofuran-7-ylmethyl)-4-[(4-hydroxyphenyl)thio]-N-methylbutanamide
   Prepared from 4-[(4-hydroxyphenyl)thio]butanoic acid [85896-82-8] and (2,3-dihydro-benzofuran-7-ylmethyl)methylamine [389845-43-6] in 48% yield.
   NMR-¹H (CDCl₃) : δ (ppm) 7.2 (dd, 2H) ; 7.1 (ddd, 1H) ; 6.99 (d, 1H) ; 6.89-6.69 (m, 3H) ; 4.73-4.34 (m, 4H ),3.20 (q, 2H) ; 2.94 (d, 3H) ; 2.84 (dt, 2H) ; 2.55 (dt, 2H) ; 2.05-1.82 (m, 2H)
   MS (ESI+) : *m*/*z* = 358 [M+H]⁺
***Example 23 :***
   N-(2-hydroxybenzyl)-4-[(4-hydroxyphenyl)thio]-N-methylbutanamide
   Prepared from 4-[(4-hydroxyphenyl)thio]butanoic acid [85896-82-8] and 2-hydroxy-N-methylbenzylamine [60399-02-2] in 31% yield.
   NMR-¹H (CDCl₃) : δ (ppm) 7.30-7.19 (m, 3H) ; 7.11 (dd, 1H) ; 6.94 (dd, 1H) ; 6.83 (dt, 1H) ; 6.78-6.70 (m, 2H) ; 4.42 (s, 3H) ; 3.03 (s, 3H) ; 2.85 (t, 2H) ; 2.47 (t, 2H) ; 1.91 (quint, 2H)
   MS (ESI+) : *m*/*z* = 332 [M+H]⁺
***Example 24 :***
   4-[(4-hydroxyphenyl)thio]-N-methyl-N-(pyridin-2-ylmethyl)butanamide
   Prepared from 4-[(4-hydroxyphenyl)thio]butanoic acid [85896-82-8] and N-methyl-N-(2-pyridylmethyl)amine [21035-59-6] in 23% yield.
   NMR-¹H (CDCl₃) : δ (ppm) 8.52 (dd, 1H) ; 7.70 (ddt, 1H) ; 7.36-7.08 (m, 4H) ; 6.87-6.62 (m, 2H) ; 4.67 (d, 2H) ; 3.01 (d, 3H) ; 2.83 (dt, 2H) ; 2.59-2.45 (m, 2H) ; 2.09-1.79 (m, 2H)
   MS (ESI+) : *m*/*z* = 317 [M+H]⁺
***Example 25 :***
   4-[(4-fluorophenyl)thio]-N-(2-methoxybenzyl)-N-methylbutanamide
   Prepared from 4-[(4-fluorophenyl)thio]butanoic acid [18850-56-1] and commercial 2-methoxy-N-methylbenzylamine in 79% yield.
   NMR-¹H (CDCl₃) : δ (ppm) 7.47-7.07 (m, 3H) ; 7.08-6.77 (m, 5H) ;4.54 (d, 2H) ; 3.84 (d, 3H) ; 3.05-2.85 (m, 5H) ; 2.50 (dt, 2H) ; 2.07-1.87 (m, 2H)
   MS (ESI+) : *m*/*z* = 348 [M+H]⁺
***Example 26 :***
   4-[(3-hydroxyphenyl)thio]-N-(2-methoxybenzyl)-N-methylbutanamide
   Prepared from 4-[(3-hydroxyphenyl)thio]butanoic acid [1004781-69-4] and commercial 2-methoxy-N-methylbenzylamine in 94% yield.
   NMR-¹H (CDCl₃) : δ (ppm) 7.43-6.52 (m, 8H) ; 4.56 (d, 2H) ; 3.81 (d, 3H) ; 3.96-2.80 (m, 5H) ; 2.46 (q, , 2H) ; 2.03-1.85 (m, 2H)
   MS (ESI+) : *m*/*z* = 346 [M+H]⁺
***Example 27 :***
   4-[(4-hydroxyphenyl)thio]-N-methyl-N-[2-(trifluoromethoxy)benzyl]butanamide
   Prepared from 4-[(4-hydroxyphenyl)thio]butanoic acid [85896-82-8] and N-methyl-N-[2-(trifluoromethoxy)benzyl]amine [823188-82-5] in 41 % yield.
   NMR-¹H (CDCl₃) : δ (ppm) 7.8 (s large, 1H) ; 7.42-7.07 (m, 6H) ; 6.91-6.64 (m, 2H) ; 4.65 (d, 2H) ; 2.96 (s, 3H) ; 2.85 (dt, 2H) ; 2.53 (dt, 2H) ; 2.93-2.76 (m, 2H)
   MS (ESI+) : *m*/*z* = 400 [M+H]⁺
***Example 28 :***
   N-(2-furylmethyl)-4-[(4-hydroxyphenyl)thio]-N-methylbutanamide
   Prepared from 4-[(4-hydroxyphenyl)thio]butanoic acid [85896-82-8] and commercial N-methylfurfurylamine in 67% yield.
   NMR-¹H (CDCl₃) : δ (ppm) 7.33 (d, 1H) ; 7.24 (dd, 2H) ; 6.78 (d, 2H) ; 6.31 (d, 1H) ; 6.26-6.14 (m, 1H) ; 4.48 (d, 2H) ; 2.96 (d, 3H) ; 2.84 (dt, 2H) ; 2.64 (t, 1H) ; 2.48 (t, 1H) ; 2.07-1.80 (m, 2H) ;
   MS (ESI+) : *m*/*z* = 306 [M+H]⁺
***Example 29 :***
   N-(2,3-dimethoxybenzyl)-4-[(4-hydroxyphenyl)thio]-N-methylbutanamide
   Prepared from 4-[(4-hydroxyphenyl)thio]butanoic acid [85896-82-8] and 2,3-dimethoxy-N-methylbenzylamine [53663-28-8] in 47% yield.
   NMR-¹H (CDCl₃) : δ (ppm) 7.29-7.23 (m, 2H) ; 6.07-6.61 (m, 5H) ; 4.60 (d, 2H) ; 3.85 (dd, 6H) ; 2.92 (s, 3H) ; 2.90-2.81 (m, 2H) ; 2.67-2.39 (m, 2H) ; 2.09-1.83 (m, 2H)
   MS (ESI+) : *m*/*z* = 376 [M+H]⁺
***Example 30 :***
   N-(2-ethoxybenzyl)-4-[(4-hydroxyphenyl)thio]-N-methylbutanamide
   Prepared from 4-[(4-hydroxyphenyl)thio]butanoic acid (CAS [85896-82-8]) and 2-ethoxy-N-methylbenzylamine [709651-39-8] in 40% yield.
   NMR-¹H (CDCl₃) : δ (ppm) 7.32-7.11 (m, 3H) ; 7.05-6.74 (m, 5H) ; 4.58 (d, 2H) ; 4.04 (quint, 2H) ; 2.95 (d, 3H) ; 2.85 (dt, 2H) ; 2.67-2.49 (m, 2H) ; 2.06-1.88 (m, 2H) ; 1.41 (q, 3H)
   MS (ESI+) : *m*/*z* = 360 [M+H]⁺
***Example 31 :***
   N-(2-chloro-6-fluorobenzyl)-4-[(4-hydroxyphenyl)thio]-N-methylbutanamide
   Prepared from 4-[(4-hydroxyphenyl)thio]butanoic acid [85896-82-8] and 2-chloro-6-fluoro-N-methylbenzylamine [62924-64-5] in 36% yield.
   NMR-¹H (CDCl₃) : δ (ppm) 7.7 (s, 1H) ; 7.3-7.2 (m, 4H) ; 7.04-6.90 (m, 1H) ; 6.77 (dd, 2H) ; 4.77 (d, 2H) ; 2.92 -2.7 ( m, 5H) ; 2.5 (t, 2H) ; 2.02-1.85 (m, 2H)
   MS (ESI+) : *m*/*z* = 368 [M+H]⁺
***Example 32 :***
   N-(2,6-Dichlorobenzyl)-4-[(4-hydroxyphenyl)thio]-N-methylbutanamide
   Prepared from 4-[(4-hydroxyphenyl)thio]butanoic acid [85896-82-8] and 2,6-dichloro-N-methylbenzylamine [15205-19-3] in 28% yield.
   NMR-¹H (CDCl₃) : δ (ppm) 7.54-7.06 (m, 5H) ; 6.78 (dd, 2H) ; 4.88 (d, 2H) ; 2.90 (t, 2H) ; 2.72 (d, 3H) ; 2.51 (t, 2H) ; 2.08-1.87 (m, 2H)
   MS (ESI+) : *m*/*z* = 384/386 [M+H]⁺
***Example 33 :***
   N-(2-Fluorobenzyl)-4-[(4-hydroxyphenyl)thio]-N-methylbutanamide
   Prepared from 4-[(4-hydroxyphenyl)thio]butanoic acid [85896-82-8] and commercial 2-fluoro-N-methylbenzylamine in 30% yield.
   NMR-¹H (CDCl₃) : δ (ppm) 7.77 (s, 1H) ; 7.35-6.97 (m, 6H) ; 6.90-6.66 (m, 2H) ; 4.61 (d, 2H) ; 2.96 (d, 3H) ; 2.85 (q, 2H) ; 2.55 (q, 2H) ; 2.03-1.85 (m, 2H)
   MS (ESI+) : *m*/*z* = 334 [M+H]⁺
***Example 34 :***
   N-(2,3-Dichlorobenzyl)-4-[(4-hydroxyphenyl)thio]-N-methylbutanamide
   Prepared from 4-[(4-hydroxyphenyl)thio]butanoic acid [85896-82-8] and 2,3-dichloro-N-methylbenzylamine [731827-07-9] in 62% yield.
   NMR-¹H (CDCl₃) : δ (ppm) 7.57-6.89 (m, 5H) ; 6.87-6.58 (m, 2H) ; 5.96 (br s, 1H) ; 4.66 (d, 2H) ; 2.98 (s, 3H) ; 2.87 (dt, 2H) ; 2.55 (dt, 2H) ; 2.11-1.83 (m, 2H)
   MS (ESI+) : *m*/*z* = 384/386 [M+H]⁺
***Example 35 :***
   4-[(4-Hydroxyphenyl)thio]-N-(2-methoxybenzyl)-N-methylbutanamide
   Prepared from 4-[(4-hydroxyphenyl)thio]butanoic acid [85896-82-8] and commercial 2-methoxy-N-methylbenzylamine in 32% yield.
   NMR-¹H (CDCl₃) : δ (ppm) 7.33-6.82 (m, 6H) ; 6.80-6.71 (m, 2H) ; 4.56 (d, 2H) ; 3.83 (d, 3H) ; 2.94 (d, 3H) ; 2.85 (dd, 2H) ; 2.52 (t, 2H) ; 2.04-1.85 (m, 2H)
   MS (ESI+) : *m*/*z* = 346 [M+H]⁺
***Example 36 :***
   N-(2,4-Dichlorobenzyl)-4-[(4-hydroxyphenyl)thio]-N-methylbutanamide
   Prepared from 4-[(4-hydroxyphenyl)thio]butanoic acid [85896-82-8] and 2,4-dichloro-N-methylbenzylamine [5013-77-4] in 64% yield.
   NMR-¹H (CDCl₃) : δ (ppm) 7.52-6.91 (m, 5H) ; 6.89-6.60 (m, 2H) ; 5.73 (br s, 1H) ; 4.61 (d, 2H) ; 2.96 (s, 1H) ; 2.89 (dt, 2H) ; 2.49 (dt, 2H) ; 2.09-1.81 (m, 2H)
   MS (ESI+) : *m*/*z* = 384/386 [M+H]⁺
***Example 37 :***
   4-[(4-Hydroxyphenyl)thio]-N-methyl-N-(2-methylbenzyl)butanamide
   Prepared from 4-[(4-hydroxyphenyl)thio]butanoic acid [85896-82-8] and N,2-dimethylbenzylamine [874-33-9] in 35% yield.
   NMR-¹H (CDCl₃) : δ (ppm) 7.44-7.12 (m, 5H) ; 7.06 (d, 1H) ; 6.90-6.69 (m, 2H) ; 4.55 (d, 2H) ; 2.93 (d, 3H) ; 2.87 (dt, 2H) ; 2.51 (dt, 2H) ; 2.27 (d, 3H) ; 2.06-1.86 (m, 2H)
   MS (ESI+) : *m*/*z* = 330 [M+H]⁺
***Example 38 :***
   N-(1,3-Benzodioxol-4-ylmethyl)-4-[(4-hydroxyphenyl)thio]-N-methylbutanamide
   Prepared from 4-[(4-hydroxyphenyl)thio]butanoic acid [85896-82-8] and benzo[1,3]dioxol-4-ylmethyl-methylamine [110931-73-2] in 56% yield.
   NMR-¹H (CDCl₃) : δ (ppm) 7.37-7.17 (m, 2H) ; 6.95-6.54 (m, 5H) ; 5.93 (d, 2H) ; 4.53 (d, 2H) ; 2.95 (d, 3H) ; 2.86 (q, 2H) ; 2.55 (dt, 2H) ; 2.06-1.84 (m, 2H)
   MS (ESI+) : *m*/*z* = 360 [M+H]⁺
***Example 39 :***
   N-(2-Methoxybenzyl)-N-methyl-4-(quinolin-2-ylthio)butanamide
   Prepared from 4-[(2-quinolinyl)thio]butanoic acid (see below) and commercial 2-methoxy-N-methylbenzylamine in 65% yield.
   NMR-¹H (CDCl₃) : δ (ppm) 7.96-7.82 (m, 2H) ; 7.75-7.56 (m, 2H) ; 7.47-7.36 (m, 1H) ; 7.31-7.14 (m, 2H) ; 7.05-6.82 (m, 3H) ; 4.56 (d, 2H) ; 3.81 (d, 3H) ; 3.51-3.35 (m, 2H) ; 2.93 (s, 3H) ; 2.60 (t, 2H) ; 2.19 (m, 2H)
   MS (ESI+) : *m*/*z* = 381 [M+H]⁺
   4-[(2-Quinolinyl)thio]butanoic acid was prepared according general procedure X from commercial 2-quinolinethiol and commercial ethyl 4-bromobutanoic acid.
***Example 40 :***
   N-(2-Methoxybenzyl)-N-methyl-4-{[4-(trifluoromethyl)phenyl]thio}butanamide
   Prepared from 4-{[4-(trifluoromethyl)phenyl]thio}butanoic acid (see below) and commercial 2-methoxy-N-methylbenzylamine in 71 % yield.
   NMR-¹H (CDCl₃) : δ (ppm) 7.52-7.13 (m, 5H) ; 7.05-6.83 (m, 3H) ; 4.55 (d, 2H) ; 3.83 (d, 3H) ; 3.15-3.00 (dt, 2H) ; 2.94 (d, 3H) ; 2.53 (t, 2H) ; 2.14-1.96 (m, 2H)
   MS (ESI+) : *m*/*z* = 398 [M+H]⁺
   4-{[4-(trifluoromethyl)phenyl]thio}butanoic acid was prepared according general procedure X from commercial 4-(trifluoromethyl)thiophenol and commercial ethyl 4-bromobutanoic acid.
***Example 41 :***
   4-[(4-Hydroxyphenyl)thio]-N-(2-isopropoxybenzyl)-N-methylbutanamide
   Prepared from 4-[(4-hydroxyphenyl)thio]butanoic acid [85896-82-8] and 2-isopropoxy-N-methylbenzylamine (see below) in 53% yield.
   NMR-¹H (CDCl₃) : δ (ppm) 7.32-7.12 (m, 3H) ; 7.03-6.72 (m, 5H) ; 4.59 (sept ; 1H) ; 4.55 (d, 2H) ; 2.94 (d, 3H) ; 2.83 (dd, 2H) ; 2.53 (q, 2H) ; 1.94 (quint ,2H) ; 1.33 (t, 6H)
   MS (ESI+) : *m*/*z* = 374 [M+H]⁺
   2-Isopropoxy-N-methylbenzylamine was prepared according general procedure Y from commercial 2-isopropoxybenzaldehyde.
***Example 42 :***
   N-(2-Methoxybenzyl)-4-[(4-methoxyphenyl)thio]-N-methylbutanamide
   Prepared from 4-[(4-methoxyphenyl)thio]butanoic acid [52872-94-3] and commercial 2-methoxy-N-methylbenzylamine in 42% yield.
   NMR-¹H (CDCl₃) : δ (ppm) 7.38-7.10 (m, 3H) ; 7.05-6.77 (m, 5H) ; 4.55 (d, 2H) ; 3.83 (d, 3H) ; 3.79 (d, 3H) ; 3.04-2.75 (m, 5H) ; 2.51 (dt, 2H) ; 2.10-1.81 (m, 2H)
   MS (ESI+) : *m*/*z* = 360 [M+H]⁺
***Example 43 :***
   4-[(4-Fluorophenyl)thio]-N-(2-hydroxybenzyl)-N-methylbutanamide
   Prepared from 4-[(4-fluorophenyl)thio]butanoic acid [18850-56-1] and 2-hydroxy-N-methylbenzylamine [60399-02-2] in 65% yield.
   NMR-¹H (CDCl₃) : δ (ppm) 9.55 (s, large, 1H) ; 7.41-7.19 (m, 3H) ; 7.11 (dd, 1H) ; 7.02-6.88 (m, 3H) ; 6.82 (dt, 1H) ; 4.42 (s, 2H) ; 3.02 (d, 3H) ; 2.95 (t, 2H) ; 2.48 (t, 2H) ; 1.96 ( p, 2H)
   MS (ESI+) : *m*/*z* = 334 [M+H]⁺
***Example 44 :***
   4-(1H-indol-5-yloxy)-N-(2-methoxybenzyl)-N-methylbutanamide
   Prepared from 4-(1H-indol-5-yloxy]butanoic acid (see below) and commercial 2-methoxy-N-methylbenzylamine in 56% yield.
   NMR-¹H (CDCl₃) : δ (ppm) 8.16 (br s, 1H) ; 7.33-7.21 (m, 2H) ; 7.21-6.99 (m, 3H) ; 6.97-6.74 (m, 3H) ; 6.46 (dd, 1H) ; 4.59 (d, 2H) ; 4.07 (dt, 2H) ; 3.82 (d, 3H) ; 2.97 (d, 3H) ; 2.63 (t, 2H) ; 2.27-2.12 (m, 2H)
   MS (ESI+) : *m*/*z* = 353 [M+H]⁺
   4-(1H-Indol-5-yloxy]butanoic acid was prepared according general procedure X from commercial 5-hydroxyindole and commercial ethyl 4-bromobutanoic acid.
***Example 45 :***
   N-(2,3-Dichlorobenzyl)-4-[(4-fluorophenyl)thio]-N-methylbutanamide
   Prepared from 4-[(4-fluorophenyl)thio]butanoic acid [18850-56-1] and 2,3-dichloro-N-methylbenzylamine [731827-07-9] in 67% yield.
   NMR-¹H (CDCl₃) : δ (ppm) 7.54-6.83 (m, 7H) ; 4.65 (d, 2H) ; 3.13-2.81 (m, 5H) ; 2.48 (dt, 2H) ; 2.06-1.86 ( m, 2H)
   MS (ESI+) : *m*/*z* = 386/388 [M+H]⁺
***Example 46 :***
   Methyl 2-{[{4-[(4-hydroxyphenyl)thio]butanoyl}(methyl)amino]methyl}benzoate
   Prepared from 4-[(4-hydroxyphenyl)thio]butanoic acid [85896-82-8] and methyl 2-[(methylamino)methyl]benzoate in 11 % yield.
   NMR-¹H (CDCl₃) : δ (ppm) 8.00 (ddd, 1H) ; 7.67-7.03 (m, 5H) ; 6.88-6.62 (m, 2H) ; 4.98 (d, 2H) ; 3.90 (d, 3H) ; 2.98 (d, 3H) ; 2.84 (dt, 2H) ; 2.50 (dt, 2H) ; 2.04-1.82 (m, 2H)
   MS (ESI+) : *m*/*z* = 374 [M+H]⁺
   Methyl 2-[(methylamino)methyl]benzoate was prepared by esterification of 2-[(methylamino)methyl]benzoic acid [527705-23-3].
***Example 47 :***
   Ethyl 4-[2-({4-[(4-hydroxyphenyl)thio]butanoyl}methylamino)methylphenyl]-piperazine-1-carboxylate
   Prepared from 4-[(4-hydroxyphenyl)thio]butanoic acid [85896-82-8] and ethyl 4-[2-(methylamino)methylphenyl]piperazine-1-carboxylate (see below) in 71% yield.
   NMR-¹H (CDCl₃) : δ (ppm) 7.74 (s, large 1H) ; 7.38-7.00 (m, 6H) ; 6.76 (t, 2H) ; 4.66 (d, 2H) ; 4.27-4.09 (m, 2H) ; 3.71-3.50 (m, 4H) ; 3.04-2.69 (m, 9H) ; 2.52 (dt, 2H) ; 2.08-1.81 (m, 3H) ; 1.29 (dt, 4H)
   MS (ESI+) : *m*/*z* = 472 [M+H]⁺
   Ethyl 4-[2-(methylamino)methylphenyl]piperazine-1-carboxylate was prepared according general procedure Y from ethyl 4-(2-formylphenyl)piperazine-1-carboxylate [204078-77-3].
***Example 48 :***
   4-[(4-Hydroxyphenyl)thio]-N-[2-(4-hydroxypiperidin-1-yl)benzyl]-N-methylbutanamide
   Prepared from 4-[(4-hydroxyphenyl)thio]butanoic acid [85896-82-8] and 2-(4-hydroxypiperidin-1-yl)-N-methylbenzylamine (see below) in 41% yield.
   NMR-¹H (CDCl₃) : δ (ppm) 7.28-7.02 (m, 6H) ; 6.79-6.73 (m, 2H) ; 4.59 (d, 2H) ; 3.01-2.67 (m, 8H) ; 2.55 (t, 1H) ; 2.44 (t, 1H) ; 2.03-1.86 (m, 5H) ; 1.78-1.60 (m, 4H)
   MS (ESI+) : *m*/*z* = 415 [M+H]⁺
   2-(4-Hydroxypiperidin-1-yl)-N-methylbenzylamine was prepared according general procedure Y from 2-(4-Hydroxy-piperidin-1-yl)benzaldehyde [291545-00-1].
***Example 49 :***
   4-[(4-Hydroxyphenyl)thio]-N-methyl-N-(2-phenoxybenzyl)butanamide
   Prepared from 4-[(4-hydroxyphenyl)thio]butanoic acid [85896-82-8] and N-methyl-2-phenoxybenzylamine [361394-74-3] in 31% yield.
   NMR-¹H (CDCl₃) : δ (ppm) 7.36-6.86 (m, 11H) ; 6.76-6.71 (m, 2H) ; 4.61 (d, 2H) ; 2.95-2.94 (m, 3H) ; 2.86-2.74 (m, 2H) ; 2.53-2.41 (m, 2H) ; 1.97-1.82 (m, 2H)
   MS (ESI+) : *m*/*z* = 408 [M+H]⁺
***Example 50 :***
   4-[(4-Fluorophenyl)thio]-N-(2-isopropoxybenzyl)-N-methylbutanamide
   Prepared from 4-[(4-fluorophenyl)thio]butanoic acid [18850-56-1] and 2-isopropoxy-N-methylbenzylamine (see example 41) in 18% yield.
   NMR-¹H (CDCl₃) : δ (ppm) 7.38-7.12 (m, 3H) ; 7.01-6.84 (m, 5H) ; 4.59 (sept, 1H) ; 4.53 (d, 2H) ; 3.01-2.90 (m, 5H) ; 2.50 (t, 2H) ; 2.04-1.89 (m, 2H) ; 1.33 (t, 6H)
   MS (ESI+) : *m*/*z* = 376 [M+H]⁺
***Example 51 :***
   4-[(4-fluorophenyl)thio]-N-methyl-N-(2-morpholin-4-ylbenzyl)butanamide
   Prepared from 4-[(4-fluorophenyl)thio]butanoic acid [18850-56-1] and N-methyl-2-(morpholin-4-yl)benzylamine [871217-44-6] in 44% yield.
   NMR-¹H (CDCl₃) : δ (ppm) 7.40-6.92 (m, 8H) ; 4.67 (s, 2H) ; 3.87-3.82 (m, 4H) ; 3.04-2.86 (m, 9H) ; 2.50 (dt, 2H) ; 2.08-1.92 (m, 2H)
   MS (ESI+) : *m*/*z* = 403 [M+H]⁺
***Example 52 :***
   4-[(4-Hydroxyphenyl)thio]-N-methyl-N-(2-benzylbenzyl)butanamide
   Prepared from 4-[(4-hydroxyphenyl)thio]butanoic acid [85896-82-8] and 2-benzyl-N-methylbenzylamine [381237-13-4] in 50% yield.
   NMR-¹H (CDCl₃) : δ (ppm) 7.31-7.01 (m, 11H) ; 6.79-6.71 (m, 2H) ; 4.49 (d, 2H) ; 4.00 (s, 2H) ; 2.90-2.65 (m, 5H) ; 2.27-2.15 (m, 2H) ; 1.93-1.73 (m, 2H)
   MS (ESI+) : *m*/*z* = 406 [M+H]⁺
***Example 53 :***
   4-[(4-Hydroxyphenyl)thio]-N-methyl-N-{2-[4-(2-morpholin-4-ylethyl)piperazin-1-yl]benzyllbutanamide
   Prepared from 4-[(4-hydroxyphenyl)thio]butanoic acid [85896-82-8] and N-methyl-2-[4-(2-morpholin-4-ylethyl)piperazin-1-yl]benzylamine in 39% yield.
   NMR-¹H (CDCl₃) : δ (ppm) 7.29-7.00 (m, 6H) ; 6.77-6.70 (m, 2H) ; 4.62 (d, 2H) ; 3.74-3.71 (m, 4H) ; 2.97-2.73 (m, 9H) ; 2.69-2.36 (m, 14H) ; 2.02-1.84 (m, 2H)
   MS (ESI+) : *m*/*z* = 513 [M+H]⁺
   N-Methyl-2-[4-(2-morpholin-4-ylethyl)piperazin-1-yl]benzylamine was prepared according general procedure Z from commercial 2-fluorobenzaldehyde and commercial 1-(2-morpholinoethyl)piperazine.
***Example 54 :***
   N-[2-(4-Benzylpiperazin-1-yl)benzyl]-4-[(4-hydroxyphenyl)thio]-N-methylbutanamide
   Prepared from 4-[(4-hydroxyphenyl)thio]butanoic acid [85896-82-8] and 2-(4-benzylpiperazin-1-yl)-N-methylbenzylamine in 49% yield.
   NMR-¹H (CDCl₃) : δ (ppm) 7.35-7.00 (m, 11H) ; 6.77-6.70 (m, 2H) ; 4.64 (d, 2H) ; 3.61-3.59 (m, 2H) ; 2.95-2.43 (m, 15H) ; 2.01-1.85 (m, 2H)
   MS (ESI+) : *m*/*z* = 490 [M+H]⁺
   2-(4-Benzylpiperazin-1-yl)-N-methylbenzylamine was prepared according general procedure Y from commercial 2-(4-benzylpiperazin-1-yl)benzaldehyde.
***Example 55 :***
   5-(4-Hydroxyphenyl)-N-methyl-N-(2-morpholin-4-ylbenzyl)pentanamide
   Prepared from commercial 5-(4-hydroxyphenyl)pentanoic acid [85896-82-8] and N-methyl-2-(morpholin-4-yl)benzylamine [871217-44-6] in 75% yield.
   NMR-¹H (CDCl₃) : δ (ppm) 7.31-6.96 (m, 6H) ; 6.77-6.70 (m, 2H) ; 4.66 (d, 2H) ; 3.86-3.82 (m, 4H) ; 2.95-2.85 (m, 7H) ; 2.62-2.30 (m, 4H) ; 1.81-1.48 (m, 4H)
   MS (ESI+) : *m*/*z* = 383 [M+H]⁺
***Example 56 :***
   5-(4-Fluorophenyl)-N-(2-methoxybenzyl)-N-methyl-5-oxopentanamide
   Prepared from commercial 4-(4-fluorobenzoyl)butyric acid commercial 2-methoxy-N-methylbenzylamine in 46% yield.
   NMR-¹H (CDCl₃) : δ (ppm) 8.04-7.98 (m, 2H) ; 7.30-6.85 (m, 6H) ; 4.57 (d, 2H) ; 3.83-3.82 (m, 3H) ; 3.12-3.02 (m, 2H) ; 2.96-2.94 (m, 3H) ; 2.50 (t, 2H) ; 2.17-2.03 (m, 2H)
   MS (ESI+) : *m*/*z* = 344 [M+H]⁺
***Example 57 :***
   N-(1,1'-Biphenyl-2-ylmethyl)-4-[(4-hydroxyphenyl)thio]-N-methylbutanamide
   Prepared from 4-[(4-hydroxyphenyl)thio]butanoic acid [85896-82-8] and commercial N-methyl-2-biphenylmethylamine in 37% yield.
   NMR-¹H (CDCl₃) : δ (ppm) 7.48-7.15 (m, 11H) ; 6.77-6.72 (m, 2H) ; 4.52 (d, 2H) ; 2.89-2.69 (m, 5H) ; 2.27 (td, 2H) ; 1.96-1.79 (m, 2H)
   MS (ESI+) : *m*/*z* = 392 [M+H]⁺
***Example 58 :***
   5-(4-Fluorophenyl)-N-(2-methoxybenzyl)-N-methylpentanamide
   Prepared from commercial 5-(4-fluorophenyl)pentanoic acid and commercial 2-methoxy-N-methylbenzylamine in 67% yield.
   NMR-¹H (CDCl₃) : δ (ppm) 7.31-6.84 (m, 8H) ; 4.55 (d, 2H) ; 3.83-3.82 (m, 3H) ; 2.94 (s, 3H) ; 2.65-2.54 (m, 2H) ; 2.41-2.33 (m, 2H) ; 1.70-1.56 (m, 4H)
   MS (ESI+) : *m*/*z* = 330 [M+H]⁺
***Example 59 :***
   5-(4-Hydroxyphenyl)-N-(2-isopropoxybenzyl)-N-methylpentanamide
   Prepared from commercial 5-(4-hydroxyphenyl)pentanoic acid and 2-isopropoxy-N-methylbenzylamine (see example 41) in 53% yield.
   NMR-¹H (CDCl₃) : δ (ppm) 7.25-6.72 (m, 8H) ; 4.64-4.47 (m, 3H) ; 2.93 (s, 3H) ; 2.54 (d, 2H) ; 2.39 (t, 2H) ; 1.79-1.50 (m, 4H) ; 1.35-1.25 (m, 6H)
   MS (ESI+) : *m*/*z*= 356 [M+H]⁺
***Example 60 :***
   N-(2-Hydroxyethyl)-4-[(4-hydroxyphenyl)thio]-N-(2-methoxybenzyl)butanamide
   Prepared from 4-[(4-hydroxyphenyl)thio]butanoic acid [85896-82-8] and N-(2-hydroxyethyl)-2-methoxybenzylamine [109926-15-0] in 32% yield.
   NMR-¹H (CDCl₃) : δ (ppm) 7.33-7.12 (m, 3H) ; 7.04-6.72 (m, 5H) ; 4.61 (d, 2H) ; 3.83-3.41 (m, 8H) ; 2.82 (t, 2H) ; 2.54 (t, 2H) ; 1.91 (quint, 2H)
   MS (ESI+) : *m*/*z* = 376 [M+H]⁺
***Example 61 :***
   4-[(4-Fluorophenyl)methylamino]-N-(2-isopropoxybenzyl)-N-methylbutanamide
   Prepared from 4-[(4-fluorophenyl)methylamino]butanoic acid (see below) and 2-isopropoxy-N-methylbenzylamine (see example 41) in 53% yield.
   NMR-¹H (CDCl₃) : δ (ppm) 7.28-7.17 (m, 2H) ; 7.07-6.85 (m, 4H) ; 6.69-6.62 (m, 2H) ; 4.64-4.44 (m, 3H) ; 3.39-3.27 (m, 2H) ; 2.94-2.85 (m, 6H) ; 2.41-2.35 (m, 2H) ; 2.00-1.84 (m, 2H) ; 1.35-1.32 (m, 6H)
   MS (ESI+) : *m*/*z* = 373 [M+H]⁺
   4-[(4-fluorophenyl)methylamino]butanoic acid was prepared according general procedure X from commercial 4-fluoro-N-methylaniline and commercial ethyl 4-bromobutanoic acid.
***Example 62 :***
   4-[(4-Fluorophenyl)methylamino]-N-methyl-N-[2-(trifluoromethyl)benzyl]butanamide
   Prepared from 4-[(4-fluorophenyl)methylamino]butanoic acid (see example 61) and N-methyl-2-(trifluoromethyl)benzylamine [296276-41-0] in 15% yield.
   NMR-¹H (CDCl₃) : δ (ppm) 7.71-7.64 (m, 1H) ; 7.58-7.16 (m, 3H) ; 7.05-6.84 (m, 2H) ; 6.72-6.60 (m, 2H) ; 4.75 (d, 2H) ; 3.27 (dt, 2H) ; 3.01-2.82 (m, 6H) ; 2.30 (dt, 2H) ; 2.05-1.81 (m, 2H)
   MS (ESI+) : *m*/*z* = 383 [M+H]⁺
***Example 63 :***
   N-{2-[4-(2-Dimethylaminoethyl)piperazin-1-yl]benzyl}-4-[(4-hydroxyphenyl)thio]-N-methylbutanamide
   Prepared from 4-[(4-hydroxyphenyl)thio]butanoic acid [85896-82-8] and 2-[4-(2-dimethylaminoethyl]piperazin-1-yl)-N-methylbenzylamine (see below) in 45% yield.
   NMR-¹H (CDCl₃) : δ (ppm) 7.30-7.18(m, 3H) ; 7.13-7.03 (m, 3H) ; 6.73 (t,2H) ; 4.59 (d, 2H) ; 2.92-2.65 (m, 9H) ; 2.56-2.32 (m, 16H) ; 1.99-1.85 (m, 2H)
   MS (ESI+) : *m*/*z* = 471 [M+H]⁺
   2-[4-(2-dimethylaminoethyl]piperazin-1-yl)-N-methylbenzylamine was prepared according general procedure Z from commercial 2-fluorobenzaldehyde and commercial 1-[4-(2-dimethylaminoethyl)]piperazine.
***Example 64 :***
   4-[(4-Hydroxyphenyl)thio]-N-(2-methoxybenzyl)-N-(2-morpholin-4-ylethyl)butanamide
   Prepared from 4-[(4-hydroxyphenyl)thio]butanoic acid [85896-82-8] and 2-methoxy-N-(2-morpholin-4-ylethyl)benzylamine [626209-57-2] in 35% yield.
   NMR-¹H (CDCl₃) : δ (ppm) 7.38-7.19(m, 3H) ; 7.10 (d, 1H) ; 7.03-6.89 (m, 2H) ; 6.83-678 (m, 2H) ; 4.67 (d, 2H) ; 3.90-3.86 (d, 3H) ; 3.80-3.71 (m, 4H) ; 3.51 (dt, 2H) ; 2.96-2.84 (m, 2H) ; 2.67-2.51 (m, 8H) ; 2.09-1.92 (m, 2H)
   MS (ESI+) : *m*/*z* = 445 [M+H]⁺
***Example 65 :***
   4-[(4-Hydroxyphenyl)thio]-N-[2-(methoxymethyl)benzyl]-N-methylbutanamide
   Prepared from 4-[(4-hydroxyphenyl)thio]butanoic acid [85896-82-8] and 2-(methoxymethyl)-N-methylbenzylamine (see below) in 19% yield.
   NMR-¹H (CDCl₃) : δ (ppm) 7.32-7.05 (m, 6H) ; 6.74 (t, 2H) ; 4.66 (t, 2H) ; 4.44 (d, 2H) ; 3.37 (d, 3H) ; 2.93 (d, 3H) ; 2.86 (dt, 2H) ; 2.50 (dt, 2H) ; 2.04-1.86 (m, 2H)
   MS (ESI+) : *m*/*z* = 360 [M+H]⁺
   2-(Methoxymethyl)-N-methylbenzylamine was prepared according general procedure Y from 2-(methoxymethyl)benzaldehyde [106020-70-6].
***Example 66 :***
   N-Cyclopropylmethyl-4-[(4-hydroxyphenyl)thio]-N-(2-methoxybenzyl)butanamide
   Prepared from 4-[(4-hydroxyphenyl)thio]butanoic acid [85896-82-8] and N-cyclopropylmethyl-2-methoxybenzylamine [1019561-08-0] in 22% yield.
   NMR-¹H (CDCl₃) : δ (ppm) 7.34-6.77 (m, 8H) ; 4.72 (d, 2H) ; 3.89-3.83 (m, 3H) ; 3.26 (dd, 2H) ; 2.87 (dt, 2H) ; 2.59 (dt, 2H) ; 2.08-1.90 (m, 2H)
   MS (ESI+) : *m*/*z* = 386 [M+H]⁺
***Example 67 :***
   N-Ethyl-4-[(4-hydroxyphenyl)thio]-N-(2-methoxybenzyl)butanamide
   Prepared from 4-[(4-hydroxyphenyl)thio]butanoic acid [85896-82-8] and N-ethyl-2-methoxybenzylamine [62924-83-8] in 57% yield.
   NMR-¹H (CDCl₃) : δ (ppm) 7.31-7.18 (m, 3H) ; 713-6.82 (m, 3H) ; 6.78-6.73 (m, 2H) ; 4.55 (d, 2H) ; 3.85-3.80 (m, 3H) ; 3.44-3.27 (m, 2H) ; 2.89-2.76 (m, 2H) ; 2.58-2.44 (m, 2H) ; 2.03-1.85 (m, 2H) ; 1.18-1.05 (m, 3H)
   MS (ESI+) : *m*/*z* = 360 [M+H]⁺
***Example 68 :***
   N-(3-Fluoro-2-methoxybenzyl)-4-[(4-hydroxyphenyl)thio]-N-methylbutanamide
   Prepared from 4-[(4-hydroxyphenyl)thio]butanoic acid [85896-82-8] and 3-fluoro-2-methoxy-N-methylbenzylamine (see below) in 36% yield.
   NMR-¹H (CDCl₃) : δ (ppm) 7.26-7.21 (m, 3H) ; 7.07-6.94 (m; 3H) ; 6.82-6.74 (m; 2H) ; 4.59 (d, 2H) ; 3.96-3.90 (m, 3H) ; 2.95-2.80 (m, 5H) ; 2.54 (t, 2H) ; 2.03-1.87 (m, 2H)
   MS (ESI+) : *m*/*z* = 364 [M+H]⁺
   3-Fluoro-2-methoxy-N-methylbenzylamine was prepared according general procedure Y from 3-fluoro-2-methoxybenzaldehyde [74266-68-5].
***Example 69 :***
   4-[(4-Hydroxyphenyl)thio]-N-(2-methoxy-3-methylbenzyl)-N-methylbutanamide
   Prepared from 4-[(4-hydroxyphenyl)thio]butanoic acid [85896-82-8] and 2-methoxy-3,N-dimethylbenzylamine (see below) in 46% yield.
   NMR-¹H (CDCl₃) : δ (ppm) 7.26-6.74 (m, 7H) ; 4.63 (d,2H) ; 3.73-3.70 (m, 3H) ; 2.94-2.79 (m, 5H) ; 2.55 (t, 2H) ; 2.32-2.29 (m, 3H) ; 2.03-1.88 (m, 2H)
   MS (ESI+) : *m*/*z* = 360 [M+H]⁺
   2-Methoxy-3,N-dimethylbenzylamine was prepared according general procedure Y from 2-methoxy-3-methylbenzaldehyde [67639-61-6].
***Example 70 :***
   N-(5-Chloro-2-methoxybenzyl)-4-[(4-hydroxyphenyl)thio]-N-methylbutanamide
   Prepared from 4-[(4-hydroxyphenyl)thio]butanoic acid [85896-82-8] and 5-chloro-2-methoxy-N-methylbenzylamine [823188-85-8] in 55% yield.
   NMR-¹H (CDCl₃) : δ (ppm) 7.29-7.12 (m, 3H) ; 7.01 (dd, 1H) ; 6.85-6.71 (m, 3H) ; 4.50 (d, 2H) ; 3.78 (d, 3H) ; 2.94 (d, 3H) ; 2.83 (dt, 2H) ; 2.58-2.46 (m, 2H) ; 2.03-1.84 (m, 2H)
   MS (ESI+) : *m*/*z* = 380 [M+H]⁺
***Example 71 :***
   N-(5-Fluoro-2-methoxybenzyl)-4-[(4-hydroxyphenyl)thio]-N-methylbutanamide
   Prepared from 4-[(4-hydroxyphenyl)thio]butanoic acid [85896-82-8] and 5-fluoro-2-methoxy-N-methylbenzylamine [823188-87-0] in 28% yield.
   NMR-¹H (CDCl₃) : δ (ppm) 7.31-7.19 (m, 2H) ; 7.02-6.70 (m, 5H) ; 4.53 (d, 2H) ; 3.80 (d, 3H) ; 2.96 (d, 3H) ; 2.92-2.78 (m, 2H) ; 2.53 (q, 2H) ; 2.02-1.85 (m, 2H)
   MS (ESI+) : *m*/*z* = 364 [M+H]⁺
***Example 72 :***
   4-[(4-Hydroxyphenyl)thio]-N-methyl-N-[2-(morpholin-4-ylmethyl)benzyl]butanamide
   Prepared from 4-[(4-hydroxyphenyl)thio]butanoic acid [85896-82-8] and 2-(morpholin-4-ylmethyl)-N-methylbenzylamine [871825-58-0] in 62% yield.
   NMR-¹H (CDCl₃) : δ (ppm) 7.28-7.01 (m, 6H) ; 6.75 (t, 2H) ; 4.79 (s, 2H) ; 3.67-3.65 (m, 4H) ; 3.47 (s, 2H) ; 3.00-2.75 (m, 5H) ; 2.61-2.41 (m, 6H) ; 1.99-1.79 (m, 2H)
   MS (ESI+) : *m*/*z* = 415 [M+H]⁺
***Example 73 :***
   N-{2-[4-(2-hydroxyethyl)piperazin-1-yl]benzyl}-4-[(4-hydroxyphenyl)thio]-N-methylbutanamide
   Prepared from 4-[(4-hydroxyphenyl)thio]butanoic acid [85896-82-8] and 2-[4-(2-hydroxyethyl]piperazin-1-yl)-N-methylbenzylamine (see below) in 57% yield.
   NMR-¹H (CDCl₃) : δ (ppm) 7.47-7.26 (m, 6H) ; 6.96 (t, 2H) ; 4.89-4.76 (m, 2H) ; 3.89-3.86 (m, 2H) ; 3.13-2.60 (m, 15H) ; 2.20-2.02 (m, 2H) ; 1.53-1.13 (m, 2H)
   MS (ESI+) : *m*/*z* = 444 [M+H]⁺
   2-[4-(2-Hydroxyethyl]piperazin-1-yl)-N-methylbenzylamine was prepared according general procedure Y from 2-[4-(2-hydroxyethyl)piperazin-1-yl]benzaldehyde [628325-98-4].
***Example 74 :***
   N-(3,5-Dichloro-2-methoxybenzyl)-4-[(4-hydroxyphenyl)thio]-N-methylbutanamide
   Prepared from 4-[(4-hydroxyphenyl)thio]butanoic acid [85896-82-8] and 3,5-dichloro-2-methoxy-N-methylbenzylamine [869945-80-2] in 65% yield.
   NMR-¹H (CDCl₃) : δ (ppm) 7.36-7.22 (m, 3H) ; 6.98 (dd, 1H) ; 6.79-6.72 (m, 2H) ; 4.58 (d ,2H) ; 3.85-3.82 (m, 3H) ; 2.95-2.83 (m, 5H) ; 2.58-2.47 (m, 2H) ; 2.03-1.91 (m, 2H)
   MS (ESI+) : *m*/*z* = 414/416 [M+H]⁺
***Example 75 :***
   4-[(4-Hydroxyphenyl)thio]-N-(2-methoxy-4-methylbenzyl)-N-methylbutanamide
   Prepared from 4-[(4-hydroxyphenyl)thio]butanoic acid [85896-82-8] and 2-methoxy-4,N-dimethylbenzylamine (see below) in 51% yield.
   NMR-¹H (CDCl₃) : δ (ppm) 7.28-7.22 (m, 2H) ; 7.05-6.67 (m, 5H) ; 4.52 (d, 2H) ; 3.82-3.79 (m, 3H) ; 2.93-2.80 (m, 5H) ; 2.57-2.48 (m, 2H) ; 2.36-2.33 (m, 3H) ; 2.01-1.87 (m, 2H)
   MS (ESI+) : *m*/*z* = 360 [M+H]⁺
   2-Methoxy-4,N-dimethylbenzylamine was prepared according general procedure Y from 2-methoxy-4-methylbenzaldehyde [57415-35-7].
***Example 76 :***
   (3S)-4-[(4-Fluorophenyl)thio]-3-hydroxy-N-(2-methoxybenzyl)-N-methylbutanamide
   Prepared from (3S)-4-[(4-fluorophenyl)thio]-3-hydroxybutanoic acid (see below) and commercial 2-methoxy-N-methylbenzylamine in 55% yield.
   NMR-¹H (CDCl₃) : δ (ppm) 7.41-7.13 (m, 4H) ; 7.01-6.86 (m, 4H) ; 4.68-4.37 (m, 2H) ; 4.20-4.08 (m, 1H) ; 3.84-3.83 (m, 3H) ; 3.18-2.71 (m, 8H) ; 2.58-2.49 (m, 1H)
   MS (ESI+) : *m*/*z* = 364 [M+H]⁺
   (3S)-4-[(4-Fluorophenyl)thio]-3-hydroxybutanoic acid was prepared according general procedure X from commercial 4-fluorothiophenol and commercial ethyl (S)-(-)-4-chloro-3-hydroxybutanoate.
***Example 77 :***
   N-Cyclopropyl-4-[(4-hydroxyphenyl)thio]-N-(2-methoxybenzyl)butanamide
   Prepared from 4-[(4-hydroxyphenyl)thio]butanoic acid [85896-82-8] and N-cyclopropyl-2-methoxy-benzylamine [625437-49-2] in 31 % yield.
   NMR-¹H (CDCl₃) : δ (ppm) 7.34-7.13 (m, 3H) ; 7.05-6.96 (m, 1H) ; 6.94-6.8 (m, 2H) ; 6.79-6.70 (m, 2H) ; 4.62 (s, 2H), 3.87-3.76 (m, 3H) ; 2.83 (dt, 4H) ; 2.65-2.52 (m, 1H) ; 1.96 (t, 2H) ; 0.84-0.72 (m, 4H)
   MS (ESI+) : *m*/*z* = 372 [M+H]⁺
***Example 78 :***
   4-[(4-Fluorophenyl)methylamino]-N-[2-(4-methylpiperazin-1-yl)benzyl]-N-methylbutanamide
   Prepared from 4-[(4-fluorophenyl)methylamino]butanoic acid (see example 61) and N-methyl-2-(4-methylpiperazin-1-yl)benzylamine (see example 13) in 4% yield.
   NMR-¹H (CDCl₃) : δ (ppm) 7.35-6.97 (m, 4H) ; 6.98-6.82 (m, 2H) ; 6.73-6.51 (m, 2H) ; 4.60 (d, 2H) ; 3.32 (dt, 2H) ; 3.02-2.83 (m, 7H) ; 2.80 (s, 3H) ; 2.69-2.49 (m, 4H) ; 2.48-2.24 (m, 5H) ; 2.03-1.78 (m, 2H)
   MS (ESI+) : *m*/*z* = 413 [M+H]⁺
***Example 79 :***
   4-[(4-Hydroxyphenyl)thio]-N-methyl-N-{2-[(1-methylpiperidin-4-yl)oxy]benzyl}butanamide
   Prepared from 4-[(4-hydroxyphenyl)thio]butanoic acid [85896-82-8] and N-methyl-2-[(1-methylpiperidin-4-yl)oxy]benzylamine (see below) in 15% yield.
   NMR-¹H (CDCl₃) : δ (ppm) 7.30-7.1 (m, 3H) ; 6.98-6.62 (m, 5H) ; 4.57 (s, 1H) ; 4.39 (s, 1H) ; 4.03-2.63 (m, 7H) ; 2.52-2.13 (m, 7H) ; 2.09-1.54 (m, 6H)
   MS (ESI+) : m/z = 429 [M+H]⁺

### Synthesis of N-methyl-2-[(1-methylpiperidin-4-yl)oxy]benzylamine :

2-[(1-Methylpiperidin-4-yl)oxy]benzonitrile [870062-43-4] (480 mg, 2.22 mmol) were charged in 20 mL of 50% aqueous formic acid, then Raney Nickel® (761 mg, 8.88 mmol) were added. The mixture was refluxed 3 days and salts were filtered after cooling. The filtrate was basified with NaOH 1 N, extracted 3 times with ethyl acetate. The combined organic layers were dried on anhydrous MgSO₄, filtered and concentrated in vacuo to give 415 mg (yield 85%) of 2-[(1-methylpiperidin-4-yl)oxy]benzaldehyde which was converted into N-methyl-2-[(1-methylpiperidin-4-yl)oxy]benzylamine with general procedure Y (yield 79%).
***Example 80 :***
   4-[(4-Fluorophenyl)methylamino]-N-methyl-N-(2-{4-[5-(trifluoromethyl)pyridin-2-yl]piperazin-1-yl}benzyl)butanamide
   Prepared from 4-[(4-fluorophenyl)methylamino]butanoic acid (see example 61) and N-methyl-2-{4-[5-(trifluoromethyl)pyridin-2-yl]piperazin-1-yl}benzylamine (see below) in 70% yield.
   NMR-¹H (CDCl₃) : δ (ppm) 8.43 (s large,1H) ; 7.77-7.60 (m, 1H) ; 7.39-7.02 (m, 4H) ; 7.00-6.80 (m, 2H) ; 6.79-6.55 (m, 3H) ; 4.69 (d, 2H) ; 3.92-3.63 (m, 4H) ; 3.34 (dt, 2H) ; 3.15-2.73 (m, 10H) ; 2.40 (dt, 2H) ; 2.07-1.83 (m, 2H)
   MS (ESI+) : *m*/*z* = 544 [M+H]⁺
   N-Methyl-2-{4-[5-(trifluoromethyl)pyridin-2-yl]piperazin-1-yl}benzylamine was prepared according general procedure Y from commercial 2-(4-[5-(trifluoromethyl)-2-pyridyl]piperazino)benzaldehyde.
***Example 81 :***
   4-(5-Fluoro-2,3-dihydro-1H-indolyl-1-yl)-N-(2-isopropoxybenzyl)-N-methylbutanamide
   Prepared from 4-(5-fluoro-2,3-dihydro-1H-indolyl-1-yl)butanoic acid (see below) and 2-isopropoxy-N-methylbenzylamine (see example 41) in 32% yield.
   NMR-¹H (CDCl₃) : δ (ppm) 7.40-6.60 (m, 6H) ; 6.55-6.24 (m, 1H) ; 4.55 (d, 2H) ; 3.43-3.20 (m, 2H) ; 3.18-2.83 (m, 7H) ; 2.48 (t, 2H) ; 2.06-1.87 (m, 2H) ; 1.38-1.20 (m, 7H)
   MS (ESI+) : *m*/*z* = 385 [M+H]⁺
   4-(5-Fluoro-2,3-dihydro-1H-indolyl-1-yl)butanoic acid was prepared according general procedure X from 5-fluoroindoline [2343-22-8] and commercial ethyl 4-bromobutanoic acid.
***Example 82 :***
   4-(5-Fluoro-2,3-dihydro-1H-indolyl-1-yl)-N-(2-methoxybenzyl)-N-methylbutanamide
   Prepared from 4-(5-fluoro-2,3-dihydro-1H-indolyl-1-yl)butanoic acid (see example 81) and commercial 2-methoxy-N-methylbenzylamine in 55% yield.
   NMR-¹H (CDCl₃) : δ (ppm) 7.38-7.10 (m, 1H) ; 7.07-6.62 (m, 5H) ; 6.51-6.28 (m, 1H) ; 4.56 (d, 2H) ; 3.82 (d, 3H) ; 3.41-3.22 (m, 2H) ; 3.17-2.73 (m, 7H) ; 2.53-2.40 (m, 2H) ; 2.12-1.85 (m, 2H) ;
   MS (ESI+) : *m*/*z* = 357 [M+H]⁺
***Example 83 :***
   N-(2,5-Difluorobenzyl)-4-[(4-fluorophenyl)thio]-N-methylbutanamide
   Prepared from 4-[(4-fluorophenyl)thio]butanoic acid (see below) and 2,5-difluoro-N-methylbenzylamine [392691-70-2] in 68% yield.
   NMR-¹H (CDCl₃) : δ (ppm) 7.38-7.28 (m, 2H) ; 7.02-6.78 (m, 5H) ; 4.56 (d, 2H) ; 3.00-2.91 (m, 5H) ; 2.54-2.48 (m, 2H) ; 2.04 (m, 2H) ; 2.04-1.93 (m, 2H)
   MS (ESI+) : *m*/*z* = 354 [M+H]⁺
   4-[(4-Fluorophenyl)thio]butanoic acid was prepared according general procedure X from commercial 4-fluorothiophenol and commercial ethyl 4-butanoic acid.
***Example 84 :***
   N-(5-Fluoro-2-methoxybenzyl)-4-[(4-fluorophenyl)thio]-N-methylbutanamide
   Prepared from 4-[(4-fluorophenyl)thio]butanoic acid (see example 83) and 5-fluoro-2-methoxy-N-methylbenzylamine [823188-87-0] in 69% yield.
   NMR-¹H (CDCl₃) : δ (ppm) 7.46-7.23 (m, 2H) , 7.09-6.68 (m, 5H) ; 4.48 (d, 2H) ; 3.73 (d, 3H) ; 2.97-2.77 (m, 5H) ; 2.50 (quint, 2H) ; 1.98 (sept, 2H)
   MS (ESI+) : *m*/*z* = 366 [M+H]⁺
***Example 85 :***
   N-(5-Fluoro-2-isopropoxybenzyl)-4-[(4-fluorophenyl)thio]-N-methylbutanamide
   Prepared from 4-[(4-fluorophenyl)thio]butanoic acid (see example 83) and 5-fluoro-2-isopropoxy-N-methylbenzylamine (see below) in 74% yield.
   NMR-¹H (CDCl₃) : δ (ppm) 7.40-7.25 (m, 2H) ; 7.04-6.69 (m, 5H) ; 4.50 (d+sept, 3H) ; 2.96 (dt, 2H) ; 2.93 (d, 3H) ; 2.50 (dt, 2H) ; 2.07-1.88 (m, 2H) ; 1.32 (t, 6H)
   MS (ESI+) : *m*/*z* = 394 [M+H]⁺
   5-Fluoro-2-isopropoxy-N-methylbenzylamine was prepared according general procedure Y from 5-fluoro-2-isopropoxybenzaldehyde [610797-48-3].
***Example 86 :***
   4-[(4-Fluorophenyl)thio]-N-(H-indol-7-ylmethyl)-N-methylbutanamide
   N-(5-Fluoro-2-isopropoxybenzyl)-4-[(4-fluorophenyl)thio]-N-methylbutanamide
   Prepared from 4-[(4-fluorophenyl)thio]butanoic acid (see example 83) and N-methyl-1H-indole-7-methanamine [709649-74-1] in 62% yield.
   NMR-¹H (CDCl₃) : δ (ppm) 10.13 (s large, 1H) ; 7,66-7.62 (m, 1H) ; 7.32-7.21 (m, 3H) ; 7,05-6.91 (m, 4H) ; 6.55-6.53 (m, 1H) ;4.74 (s, 2H) ; 2.97-2.91 (m, 5H) ; 2.50-2.44 (t, 2H) ; 2.05-1.89 (m, 2H)
   MS (ESI+) : *m*/*z* = 357 [M+H]⁺
***Example 87 :***
   N-(5-Fluoro-2-methoxybenzyl)-4-[(4-methoxyphenyl)thio]-N-methylbutanamide
   Prepared from 4-[(4-methoxyphenyl)thio]butanoic acid [52872-94-3] and 5-fluoro-2-methoxy-N-methylbenzylamine [823188-87-0] in 53% yield.
   NMR-¹H (CDCl₃) : δ (ppm) 7.40-7.24 (m, 2H) ; 7.02-6.70 (m, 5H) ; 4.51 (d, 2H) ; 3.87-3.72 (m, 6H) ; 3.00-2.88 (m, 5H) ; 2.59-2.40 (m, 2H) ; 2.05-1.83 (m, 2H)
   MS (ESI+) : *m*/*z* = 378 [M+H]⁺
***Example 88 :***
   N-(2,6-Dimethoxybenzyl)-4-[(4-fluorophenyl)thio]-N-methylbutanamide
   Prepared from 4-[(4-fluorophenyl)thio]butanoic acid (see example 83) and 2,6-dimethoxy-N-methylbenzylamine [958863-63-3] in 49% yield.
   NMR-¹H (CDCl₃) : δ (ppm) 7.40-7.21 (m, 3H) ; 7.02-6.95 (m, 2H) ; 6.57-6.54 (m, 2H) ; 4.62 (d, 2H) ; 3.81-3.78 (m, 6H) ; 3.03-2.98 (m, 2H) ; 2.80-2.72 (m, 5H) ; 2.07-1.95 (m, 2H)
   MS (ESI+) : *m*/*z* = 378 [M+H]⁺
***Example 89 :***
   N-(2-Fluoro-5-methoxybenzyl)-4-[(4-fluorophenyl)thio]-N-methylbutanamide
   Prepared from 4-[(4-fluorophenyl)thio]butanoic acid (see example 83) and 2-fluoro-5-methoxy-N-methylbenzylamine (see below) in 62% yield.
   NMR-¹H (CDCl₃) : δ (ppm) 7.37-7.28 (m, 2H) ; 7.04-6.92 (m, 2H) ; 6.80-6.58 (m, 2H) ; 4.56 (d, 2H) ; 3.75-3.74 (m, 3H) ; 3.00-2.91 (m, 5H) ; 2.54-2.47 (m, 2H) ; 2.04-1.90 (m, 2H)
   MS (ESI+) : *m*/*z* = 366 [M+H]⁺
   2-Fluoro-5-methoxy-N-methylbenzylamine was prepared according general procedure Y from commercial 2-fluoro-5-methoxybenzaldehyde.
***Example 90 :***
   N-[2-(Allyloxy)benzyl]-4-[(4-fluorophenyl)thio]-N-methylbutanamide
   Prepared from 4-[(4-fluorophenyl)thio]butanoic acid (see example 83) and 2-allyloxy-N-methylbenzylamine [869941-98-0] in 51% yield.
   NMR-¹H (CDCl₃) : δ (ppm) 7.41-7.13 (m, 4H) ; 7.06-6.81 (m, 4H) ; 6.16-5.96 (m, 1H) ; 5.49-5.24 (m, 2H) ; 4.70-4.47 (m, 4H) , 3.05-2.85 (m, 5H) ; 2.58-2.43 (m, 2H) ; 2.08-1.86 (m, 2H)
   MS (ESI+) : *m*/*z* = 374 [M+H]⁺
***Example 91 :***
   N-(5-Allyl-2-hydroxy-3-methoxybenzyl)-4-[(4-fluorophenyl)thio]-N-methylbutanamide
   Prepared from 4-[(4-fluorophenyl)thio]butanoic acid (see example 83) and 5-allyl-2-hydroxy-3-methoxy-N-methylbenzylamine (see below) in 25% yield.
   NMR-¹H (CDCl₃) : δ (ppm) 8.94 (s, 1H) ; 7.34-7.28 (m, 2H) ; 6.99-6.90 (m, 2H) ; 6.98-6.44 (m, 2H) ; 6.02-5.86 (m, 1H) ; 5.10-5.03 (m, 2H) ; 5.49-4.43 (m, 2H) ; 3.88-3.85 (m, 3H) ; 3.31-3.28 (m, 2H) ; 2.99-2.89 (m, 5H) ; 2.57-2.43 (m, 2H)
   MS (ESI+) : m/z= 404 [M+H]⁺
   5-Allyl-2-hydroxy-3-methoxy-N-methylbenzylamine was prepared according general procedure Y from commercial 5-allyl-2-hydroxy-3-methoxy-N-methylbenzaldehyde.
***Example 92 :***
   N-(5-Bromo-2-methoxybenzyl)-4-[(4-fluorophenyl)thio]-N-methylbutanamide
   Prepared from 4-[(4-fluorophenyl)thio]butanoic acid (see example 83) and 5-bromo-2-methoxy-N-methylbenzylamine [137469-70-6] in 7% yield.
   NMR-¹H (CDCl₃) : δ (ppm) 7.40-7.28 (m, 3H) ; 7.21-7.09 (m, 1H) ; 7.02-6.92 (m, 2H) ; 6.78-6.71 (m, 1H) ; 4.50 (d, 2H) ; 3.82-3.80 (m, 3H) ; 3.01-2.90 (m, 5H) ; 2.55-2.45 (m, 2H) ; 2.05-1.89 (m, 2H)
   MS (ESI+) : *m*/*z* = 426/428 [M+H]⁺
***Example 93 :***
   N-(2-Methoxybenzyl)-N-methyl-4-{[4-(methylsulfonylamino)phenyl]thio}butanamide
   Prepared from 4-{[4-(methylsulfonylamino)phenyl]thio}butanoic acid (see below) and commercial 2-methoxy-N-methylbenzylamine in 72% yield.
   NMR-¹H (CDCl₃) : δ (ppm) 7.50-6.90 (m, 8H) ; 4.65 (d, 2H) ; 3.93 (d, 3H) ; 3.21-2.96 (m, 8H) ; 2.62 (t, 2H) ; 2.19-1.99 (m, 2H)
   MS (ESI+) : *m*/*z* = 423 [M+H]⁺
   Synthesis of 4-{[4-(methylsulfonylamino)phenyl]thio}butanoic acid :
   *Step 1 :* ethyl 4-[(4-aminophenyl)thio]butanoate
      Prepared from commercial 4-aminothiophenol and commercial ethyl 4-bromobutanoate according general procedure X step 1 in 5% yield.
   *Step 2 :* ethyl 4-1[4-(methylsulfonylamino)phenyl]thiolbutanoate
      To a solution of ethyl 4-[(4-aminophenyl)thio]butanoate (100 mg, 0.418 mmol) in 2 mL of dichloromethane were added methanesulfonyl chloride (48 mg, 0.418 mmol) and triethylamine (58.3 µL, 0.418 mmol). The mixture was stirred overnight at room temperature, then refluxed 5 hours. After cooling, water was added and the mixture was extracted 3 times with ethyl acetate, the combined organic layers were washed with brine, dried with anhydrous MgSO₄, filtered and concentrated in vacuo. The residue was purified by flash chromatography on silica gel (cyclohexane / ethyl acetate 8/2) to give 63 mg (yield 47%) of the sulfonamide.
   *Step 3 :* 4-{[4-(methylsulfonylamino)phenyl]thio}butanoic acid
      Prepared from ethyl 4-[(4-aminophenyl)thio]butanoate according general procedure X step 2 in 80% yield.
***Example 94 :***
   4-[(4-Fluorophenyl)methylamino]-N-(2-methoxybenzyl)-N-methylbutanamide
   Prepared from 4-[(4-fluorophenyl)methylamino]butanoic acid (see below) and commercial 2-methoxy-N-methylbenzylamine in 42% yield.
   NMR-¹H (CDCl₃) : δ (ppm) 7.33-7.14 (m, 2H) ; 7.04-6.84 (m, 4H) ; 6.72-6.59 (m, 2H) ; 4.55 (d, 2H) ; 3.83 (s, 3H) ; 3.41-3.24 (m, 2H) ; 2.90 (dd, 6H) ; 2.43-2.32 (m, 2H) ; 2.02-1.82 (m, 2H)
   MS (ESI+) : *m*/*z* = 345 [M+H]⁺
   4-[(4-Fluorophenyl)methylamino]butanoic acid was prepared according general procedure X from commercial 4-fluoro-N-methylaniline and commercial ethyl 4-bromobutanoic acid.

### General procedure C

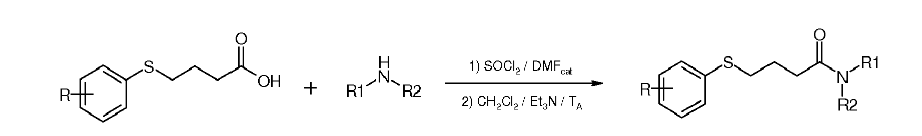

To a solution of the acid (1 eq) in dichloromethane (3 mL / mmol) was added thionyl chloride (2eq). The mixture was refluxed 3 hours then concentrated in vacuo and co-evaporated 3 times with toluene. Dichloromethane (3 mL / mmol) was added to the residue then triethylamine (1eq) and the amine (1eq) were added. The mixture was stirred overnight at room temperature and water was added. The mixture was extracted 3 times with dichloromethane, the combined organic layers were dried with anhydrous MgSO₄, filtered and concentrated in vacuo. The residue was purified by flash chromatography (silica gel) with the appropriate gradient determined by TLC to give the amide.

The following compounds were prepared according general procedure C :
***Example 95 :***
   4-[(4-Fluorophenyl)thio]-N-(2-methoxyphenyl)-N-methylbutanamide
   Prepared from 4-[(4-fluorophenyl)thio]butanoic acid [18850-56-1 and commercial 2-methoxy-N-methylaniline in 31% yield.
   NMR-¹H (CDCl₃) : δ (ppm) 7.39-7.20 (m, 3H) ; 7.12 (br d, 1H) ; 7.03-6.89 (m, 4H) ; 3.80 (s, 3H) ; 3.16 (s, 3H) ; 2.82 (t, 2H) ; 2.18-2.05 (m, 2H) ; 1.84 (quint, 2H)
   MS (ESI+) : *m*/*z* = 334 [M+H]⁺
***Example 96 :***
   4-[(4-Fluorophenyl)thio]-N-(2-methoxybenzyl)-N-(2,2,2-trifluoroethyl)butanamide
   Prepared from 4-[(4-fluorophenyl)thio]butanoic acid (see example 83) and 2-methoxy-N-(2,2,2-trifluoroethyl)benzylamine [1016737-83-9] in 15% yield.
   NMR-¹H (CDCl₃) : δ (ppm) 7.35-7.21 (m, 4H) ; 7.00-6.86 (m, 4H) ; 4.67 (d, 2H) ; 4.06-3.82 (m, 5H) ; 2.92 (t, 2H) ; 2.61-2.49 (m, 2H) ; 2.03-1.92 (m, 2H)
   MS (ESI+) : *m*/*z* = 416 [M+H]⁺

### General procedure D

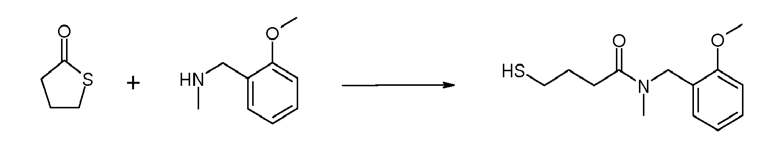

In a three-neck round-bottom flask equipped with a Dean-Stard trap, commercial 2-methoxy-N-methylbenzylamine (4.88 g, 32.4 mmol) and camphorsulfonic acid (1.5 g, 6.46 mmol) were added to a solution of thiobutyrolactone (3.3 g, 32.4 mmol) in 50 mL of toluene. The mixture was refluxed 24 hours then concentrated in vacuo. The residue was purified by flash chromatography on silica gel (cyclohexane / ethyl acetate 9/1 to 7/3) to give 6.4 g (78% yield) of 4-mercapto-N-(2-methoxybenzyl)-N-methylbutanamide.

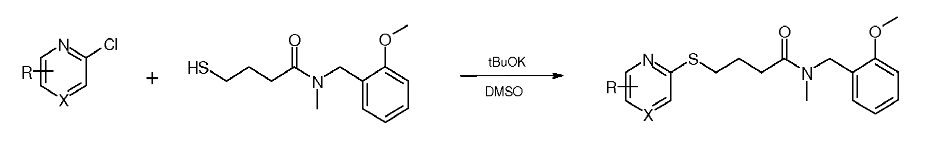

Potassium tert-butoxide (1.5eq) and the chloro derivative (1 eq) were added to a solution of 4-mercapto-N-(2-methoxybenzyl)-N-methylbutanamide (1,5eq) in dry DMSO. The mixture was heated 2.5 hours at 110°C, then aqueous NaOH 1 N was added. The mixture was extracted 3 times with ethyl acetate, the combined organic layers were washed with brine, dried on anhydrous MgSO₄, filtered and concentrated in vacuo. The residue was purified by flash chromatography (silica gel) with the appropriate gradient determined by TLC to give the required compound.

The following compounds were prepared according general procedure D :
***Example 97 :***
   N-(2-Methoxybenzyl)-N-methyl-4-(quinoxalin-2-ylthio)butanamide
   Prepared from commercial 2-chloroquinoxaline in 56% yield.
   NMR-¹H (CDCl₃) : δ (ppm) 8.56 (d, 1H) ; 8.08-7.77 (m, 2H) ; 7.75-7.51 (m, 2H) ; 7.33-7.12 (m, 1H) ; 6.99 (dd, 1H) ; 6.88 (q, 2H) ; 4.55 (d, 2H) ; 3.81 (d, 3H) ; 3.48-3.37 (m, 2H) ; 2.94 (s, 3H) ; 2.58 (t, 2H) ; 2.31-2.04 (m, 2H)
   MS (ESI+) : *m*/*z*= 382 [M+H]⁺
***Example 98 :***
   4-[(5-Chloropyridin-2-yl)thio]-N-(2-methoxybenzyl)-N-methylbutanamide
   Prepared from commercial 2,5-dichloropyridine in 49% yield.
   NMR-¹H (CDCl₃) : δ (ppm) 8.31 (ddd, 1H) ; 7.44 - 7.38 (m, 1H) ; 7.31-7.05 (m, 2H) ; 7.04-6.79 (m, 3H) ; 4.53 (d, 2H) ; 3.81 (d, 3H) ; 3.19 (dt, 2H) ; 2.92 (s, 3H) ; 2.51 (t, 2H) ; 2.15-1.91 (m, 2H).
   MS (ESI+) : *m*/*z*= 365 [M+H]⁺

### General procedure E

### Step 1 : synthesis of substituted N-benzyl-4-chloro-N-methylbutanamide :

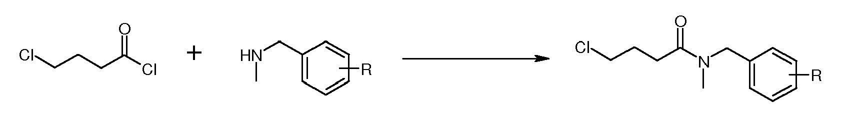

Triethylamine (1eq) and 4-chlorobutyryl chloride (1eq) were added to a solution of the substituted N-methylbenzylamine (1 eq) in dichloromethane (1.3 mL/mmol). The mixture was stirred 3 hours at room temperature then water was added. The resulting mixture was extracted 3 times with dichloromethane, the combined organic layers were dried with anhydrous MgSO₄, filtered and concentrated in vacuo. The residue was purified by flash chromatography (silica gel) with the appropriate gradient determined by TLC to give the substituted N-benzyl-4-chloro-N-methylbutanamide.

### Step 2 : alkylation of thiols with substituted N-benzyl-4-chloro-N-methylbutanamide:

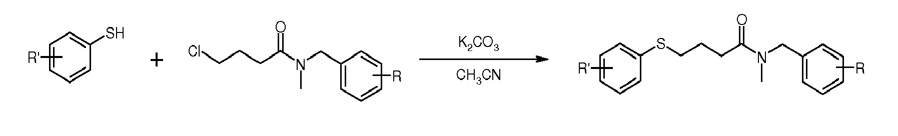

Anhydrous potassium carbonate (1.5eq) and substituted N-benzyl-4-chloro-N-methylbutanamide (1 eq) were added to a solution of the thiol derivative (1 eq) in acetonitrile (6 mL / mmol). The mixture was refluxed overnight then water and ethyl acetate was added after cooling. The mixture was extracted 3 times with ethyl acetate, the combined organic layers were washed with brine, dried with anhydrous MgSO₄, filtered and concentrated in vacuo. The residue was purified by flash chromatography (silica gel) with the appropriate gradient determined by TLC to give the thio-ether.

The following compounds were prepared according general procedure E :
***Example 99 :***
   4-[(3,4-Difluorophenyl)thio]-N-(2-methoxybenzyl)-N-methylbutanamide
   Prepared from commercial 3,4-difluorothiophenol and commercial 2-methoxy-N-methylbenzylamine in 27% yield.
   NMR-¹H (CDCl₃) : δ (ppm) 7.32-6.85 (m, 7H) ; 4.55 (d, 2H) ; 3.84-3.82 (m, 3H) ; 3.03-2.92 (m, 5H) ; 2.50 (t, 2H) ; 2.04-1.94 (m, 2H)
   MS (ESI+) : *m*/*z* = 366 [M+H]⁺
***Example 100 :***
   4-[(3-Fluorophenyl)thio]-N-(2-methoxybenzyl)-N-methylbutanamide
   Prepared from commercial 3-fluorothiophenol and commercial 2-methoxy-N-methylbenzylamine in 37% yield.
   NMR-¹H (CDCl₃) : δ (ppm) 7.32-6.80 (m, 8H) ; 4.55 (d, 2H) ; 3.84-3.82 (m, 3H) ; 3.08-2.94 (m, 5H) ; 2.53 (t, 2H) ; 2.08-1.98 (m, 2H)
   MS (ESI+) : *m*/*z* = 348 [M+H]⁺
***Example 101 :***
   4-[(2,4-Difluorophenyl)thio]-N-(2-methoxybenzyl)-N-methylbutanamide
   Prepared from commercial 2,4-difluorothiophenol and commercial 2-methoxy-N-methylbenzylamine in 15% yield.
   NMR-¹H (CDCl₃) : δ (ppm) 7.44-7.13 (m, 2H) ; 7.02-6.77 (m, 5H) ; 4.55 (d, 2H) ; 3.85-3.82 (m, 3H) ; 2.99-2.88 (m, 5H) ; 2.51 (t, 2H) ; 1.97-1.89 (m, 2H)
   MS (ESI+) : *m*/*z*= 366 [M+H]⁺
***Example 102 :***
   4-[(3-Chloro-4-fluorophenyl)thio]-N-(2-methoxybenzyl)-N-methylbutanamide
   Prepared from commercial 3-chloro-4-fluorothiophenol and commercial 2-methoxy-N-methylbenzylamine in 4% yield.
   NMR-¹H (CDCl₃) : δ (ppm) 7.43-7.09 (m, 4H) ; 7.07-6.76 (m, 3H) ; 4.52 (d, 2H) ; 3.81 (d, 3H) ; 3.04-2.86 (m, 5H) ; 2.48 (t, 2H) ; 2.06-1.86 (m, 2H)
   MS (ESI+) : *m*/*z=* 382/384 [M+H]⁺
***Example 103 :***
   4-[(2-Chloro-4-fluorophenyl)thio]-N-(2-methoxybenzyl)-N-methylbutanamide
   Prepared from commercial 2-chloro-4-fluorothiophenol and commercial 2-methoxy-N-methylbenzylamine in 5% yield.
   NMR-¹H (CDCl₃) : δ (ppm) 7.41-6.85 (m, 7H) ; 4.56 (d, 2H) ; 3.84-3.82 (m, 3H) ; 3.05-2.94 (m, 5H) ; 2.54 (t, 2H) ; 2.08-1.93 (m, 2H)
   MS (ESI+) : *m*/*z* = 382 [M+H]⁺
***Example 104 :***
   Methyl 4-({4-[methyl-(2-trifluoromethylbenzyl)amino]-4-oxobutyl}thio)benzoate
   Prepared from methyl 4-mercaptobenzoate [6302-65-4] and N-methyl-2-(trifluoromethyl)benzylamine [296276-41-0] in 46% yield.
   NMR-¹H (CDCl₃) : δ (ppm) 7.98-7.85 (m, 2H) ; 7.75-7.16 (m, 6H) ; 4.76 (d, 2H) ; 3.90 (d, 3H) ; 3.15 (t, 1H) ; 3.05 (t, 1H) ; 3.00 (s, 1H) ; 2.92 (s, 2H) ; .,61 (t, 1H) ; 2.44 (t, 1H) ; 2.20-1.96 (m, 2H)
   MS (ESI+) : *m*/*z* = 426 [M+H]⁺
***Example 105 :***
   4-(3,4-Dihydroquinolin-1 (2H)-yl)-N-(2-isopropoxybenzyl)-N-methylbutanamide
   Prepared from commercial 1,2,3,4-tetrahydroquinoline and 2-isopropoxy-N-methylbenzylamine (see below) in 63% yield.
   NMR-¹H (CDCl₃) : δ (ppm) 7.38-7.08 (m, 1H) ; 7.10-6.77 (m, 5H) ; 6.66-6.46 (m, 2H) ; 4.53 (d, 2H) ; 3.39-3.17 (m, 4H) ; 2.94 -2.92 (m, 2H) ; 2.77-2.64 (m, 2H) ; 2.40 (t, 2H) ; 2.07-1.77 (m, 4H)
   MS (ESI+) : *m*/*z*= 381 [M+H]⁺
   2-Isopropoxy-N-methylbenzylamine was prepared according general procedure Y from commercial 2-isopropoxybenzaldehyde.
***Example 106 :***
   4-[(4-Bromophenyl)thio]-N-(5-fluoro-2-methoxybenzyl)-N-methylbutanamide
   Prepared from commercial 4-bromothiophenol and 5-fluoro-2-methoxy-N-methylbenzylamine [823188-87-0] in 51% yield.
   NMR-¹H (CDCl₃) : δ (ppm) 7.40-7.36 (m, 2H) ; 7.28-7.15 (m, 2H) ; 7.02-6.72 (m, 3H) ; 4.52 (d, 2H) ; 3.82-3.80 (m, 3H) ; 3.06-2.95 (m, 5H) ; 2.56-2.45 (m, 2H) ; 2.09-1.93 (m, 2H)
   MS (ESI+) : *m*/*z* = 426/428 [M+H]⁺
***Example 107:***
   Methyl 4-({4-[(2-methoxybenzyl)methylamino]-4-oxobutyl}thio)benzoate
   Prepared from methyl 4-mercaptobenzoate [6302-65-4] and commercial 2-methoxy-N-methylbenzylamine in 15% yield.
   NMR-¹H (CDCl₃) : δ (ppm) 7.94-7.88 (m, 2H) ; 7.35-7.13 (m, 3H) ; 7.05-6.85 (m, 3H) ; 4.55 (d, 2H) ; 3.9 (s, 3H) ; 3.83 (d, 3H) ; 3.09 (dt, 2H) ; 2.94 (d, 3H) ; 2.53 (t, 2H) ; 2.19-1.90 (m, 2H).
   MS (ESI+) : *m*/*z* = 388 [M+H]⁺

### General procedure F

### Alkylation of anilines with 4-chloro-N-(2-methoxybenzyl)-N-methylbutanamide :

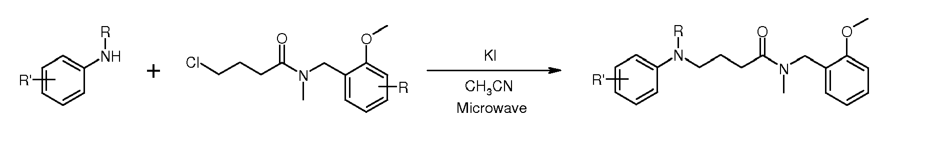

Potassium iodide (20 mg/mmol), 4-chloro-N-(2-methoxybenzyl)-N-methylbutanamide and acetonitrile (4 mL / mmol) were charged in a sealed vial and heated 15 to 30 minutes at 120-140°C in a microwave apparatus. After cooling, the mixture was concentrated in vacuo and purified by semi-preparative HPLC (acetonitrile / H₂O) to give the alkylaniline.

The following compounds were prepared according general procedure F :
***Example 108 :***
   4-[Cyclopropyl(4-fluorophenyl)amino]-N-(2-methoxybenzyl)-N-methylbutanamide
   Prepared from N-cyclopropyl-4-fluoroaniline [136005-64-6] in 16% yield.
   NMR-¹H (CDCl₃) : δ (ppm) 7.39-7.08 (m, 1H) ; 7.08-6.77 (m, 7H) ; 4.53 (d, 2H) ; 3.83 (d, 3H) ; 3.49-3.29 (m, 2H) ; 2.95 (d, 2H) ; 2.88 (s, 1H) ; 2.49-2.17 (m, 3H) ; 2.02-1.79 (m, 2H) ; 0.84-0.72 (m, 2H) ; 0.61-0.48 (m, 2H)
   MS (ESI+) : *m*/*z*= 371 [M+H]⁺
***Example 109 :***
   4-[Ethyl(4-fluorophenyl)amino]-N-(2-methoxybenzyl)-N-methylbutanamide
   Prepared from N-ethyl-4-fluoroaniline [405-67-4] in 17% yield.
   NMR-¹H (CDCl₃) : δ (ppm) 7.34-7.13 (m, 2H) ; 7.05-6.83 (m, 4H) ; 6.71-6.56 (m, 2H) ; 4.56 (d, 2H) ; 3.83 (s, 3H) ; 3.39-3.17 (m, 4H) ; 2.94 (d, 3H) ; 2.45-2.32 (m, 2H) ; 2.03-1.82 (m, 2H) ; 1.18-1.02 (q, 3H)
   MS (ESI+) : *m*/*z*= 359 [M+H]⁺
***Example 110 :***
   4-[(4-Fluorophenyl)amino]-N-(2-methoxybenzyl)-N-methylbutanamide
   Prepared from 4-fluoroaniline in 36% yield.
   NMR-¹H (CDCl₃) : δ (ppm) 7.31-6.80 (m, 6H) ; 6.54-6.46 (m, 2H) ; 4.56 (d, 2H) ; 3.82 (s, 3H) ; 3.18-3.06 (m, 3H) ; 2.96-2.94 (m, 3H) ; 2.52-2.44 (m, 2H) ; 2.04-1.94 (m, 2H)
   MS (ESI+) : *m*/*z*= 331 [M+H]⁺
***Example 111 :***
   4-[(4-Fluorophenyl)(2-hydroxyethyl)amino]-N-(2-methoxybenzyl)-N-methylbutanamide
   Prepared from 4-fluoro-N-(2-hydroxyethyl)aniline [702-17-0] in 7% yield.
   NMR-¹H (CDCl₃) : δ (ppm) 7.35-7.08 (m, 2H) ; 7.03-6.81 (m, 6H) ; 4.53 (d, 2H) ; 3.82 (d, 3H) ; 3.73 (t, 2H) ; 3.50-3.27 (m, 4H) ; 2.99-2.87 (m, 3H) ; 2.39 (t, 2H) ; 2.02-1.80 (m, 2H)
   MS (ESI+) : *m*/*z*= 375 [M+H]⁺

### Other examples

***Example 112 :***
   4-({4-[(2-methoxybenzyl)(methyl)amino]-4-oxobutyl}thio)benzoic acid
   Prepared by saponification of compound of example 107.
   NMR-¹H (CDCl₃) : δ (ppm) 7.96 (d, 2H) ; 7.44-7.13 (m, 3H) ; 7.09-6.79 (m, 3H) ; 4.57 (d, 2H) ; 3.82 (d, 3H) ; 3.10 (dt, 2H) ; 2.96 (d, 3H) ; 2.58 (t, 2H) ; 2.14-1.98 (m, 2H) ;
   MS (ESI+) : *m*/*z*= 374 [M+H]⁺
***Example 113 :***
   4-({4-[(2-Methoxybenzyl)(methyl)amino]-4-oxobutyl}thio)-N-methylbenzamide
   Prepared from compound of example 112 as follows :
      To a solution of compound of example 112 (90 mg, 0.241 mmol) and methylamine 2M in THF (120 µL, 0.241 mmol) in N,N-dimethylformamide were added 1-(3-dimethylaminopropyl)-3-ethylcarbodiimide hydrochloride (46.2 mg, 0.241 mmol) and N,N-dimethylaminopyridine (29.4 mg, 0.241 mmol). The solution was stirred overnight at room temperature and a saturated solution of NaHCO₃ was added. The mixture was extracted 3 times with ethyl acetate, the organic layers were combined and washed with HCl 1N, dried over anhydrous MgSO₄ and concentrated under vacuum. The residue was purified by flash chromatography on silica gel (cyclohexane / ethyl acetate 5/5 to 3/7) to give 17.5 mg (yield 19%) of 4-({4-[(2-methoxybenzyl)(methyl)amino]-4-oxobutyl}thio)-N-methylbenzamide.
   NMR-¹H (CDCl₃) : δ (ppm) 7.64(t, 2H) ; 7.30 (q, 2H) ; 7.23-7.14 (m, 1H) ; 7.02-6.85 (m, 3H) ; 6.13 (s, 1H) ; 4.55 (d, 2H) ; 3.83 (d, 3H) ; 3.19-3.03 (m, 2H) ; 3.02 (d, 3H) ; 2.94 (s, 3H) ; 2.53 (t, 2H) ; 2.12-1.96 (m, 2H)
   MS (ESI+) : *m*/*z* = 387 [M+H]⁺
***Example 114 :***
   N-(2-Chlorobenzyl)-4-[(4-hydroxyphenyl)sulfinyl]-N-methylbutanamide
   Prepared by oxidation of compound of example 3 as follows :
      To a solution of compound of example 3 (200 mg, 0.572 mmol) in 3 mL of dichloromethane was added m-chloroperbenzoic acid (197.3 mg, 1.143 mmol). The mixture was stirred overnight at room temperature, then the precipitate was filtered. The filtrate was washed with a saturated NaHCO₃ solution, dried with anhydrous MgSO₄, filtered and concentrated in vacuo. The residue was purified by flash chromatography on silica gel (cyclohexane / ethyl acetate 5/5 to 0/10) to give 80 mg (yield 38%) of N-(2-chlorobenzyl)-4-[(4-hydroxyphenyl)sulfinyl]-N-methylbutanamide.
   NMR-¹H (CDCl₃) : δ (ppm) 7.48-6.96 (m, 6H) ; 6.89 (dd, 2H) ; 4.63 (d, 2H) ; 3.04-2.80 (m, 5H) ; 2.52 (dt, 2H) ; 2.15-1.85 (m, 2H)
   MS (ESI+) : *m*/*z*= 366 [M+H]⁺
***Example 115 :***
   N-Benzyl-4-(4-hydroxyphenoxy)-N-methylbutanamide
      To a solution of N-benzyl-4-(4-benzyloxyphenoxy)-N-methylbutanamide (see below, 200 mg, 0.513 mmol) in 3 mL of anhydrous dichloromethane under argon was added BCl₃ 1M in dichloromethane (1 mL, 1 mmol), then the mixture was stirred 2 hours at room temperature. The mixture was quenched with a saturated NaHCO₃ solution, extracted 2 times with dichloromethane. The combined organic layers were washed with brine, dried with anhydrous MgSO₄, filtered and concentrated in vacuo. The residue was purified by flash chromatography on silica gel (cyclohexane / ethyl acetate 7/3 to 5/5) to give 119 mg (yield 76%) of N-benzyl-4-(4-hydroxyphenoxy)-N-methylbutanamide.
   NMR-¹H (CDCl₃) : δ (ppm) ppm 7.52-7.02 (m, 5H) ; 6.96-6.49 (m, 4H) ; 4.62(d, 2H) ; 3.95 (dd, 2H) ; 2.96 (d, 3H) ; 2.61 (t, 2H) ; 2.24-2.05 (m, 2H)
   MS (ESI+) : *m*/*z* = 300 [M+H]⁺
   N-Benzyl-4-(4-benzyloxyphenoxy)-N-methylbutanamide was prepared according general procedure A from 4-[4-(benzyloxyphenoxy)]butanoic acid [202126-58-7] and commercial N-methylbenzylamine in 88% yield.
***Example 116 :***
   N-[2-(Acetylamino)benzyl]-4-[(4-hydroxyphenyl)thio]-N-methylbutanamide
   *Step 1 :* 4-[(4-hydroxyphenyl)thio]-N-methyl-N-(2-nitrobenzyl)butanamide
      Prepared from 4-[(4-hydroxyphenyl)thio]butanoic acid [85896-82-8] and N-methyl-2-nitrobenzylamine [56222-08-3] in 62% yield.
   *Step 2 :* N-(2-aminobenzyl)-4-[(4-hydroxyphenyl)thio]-N-methylbutanamide
      To a solution of 4-[(4-hydroxyphenyl)thio]-N-methyl-N-(2-nitrobenzyl)butanamide (200 mg, 0.555 mmol) in a mixture of acetic acid (2 mL), water (8 mL) and ethyl acetate (6 mL) were added iron powder (155 mg, 2.775 mmol). The mixture was stirred 10 minutes at 65°C, then filtered on Celite®. The filtrate was extracted 3 times with ethyl acetate, the combined organic layers were washed with brine, dried with anhydrous MgSO₄, filtered and concentrated in vacuo to give 148 mg of the amine which was used without purification.
   *Step 3 :* N-[2-(acetylamino)benzyl]-4-[(4-hydroxyphenyl)thio]-N-methylbutanamide acetate
      To a solution of N-(2-aminobenzyl)-4-[(4-hydroxyphenyl)thio]-N-methylbutanamide (148 mg, 0.448 mmol) in 3 mL of dichloromethane were added acetic anhydride (101 mg, 0.985 mmol) and pyridine (158 µL, 1.792 mmol). The mixture was refluxed 4 hours, cooled to room temperature, washed with brine, dried with anhydrous MgSO₄ and concentrated in vacuo. The residue was purified by flash chromatography on silica gel (cyclohexane / ethyl acetate 5/5) to give 123 mg (yield 66%) of the acetamide.
   *Step 4 :* N-[2-(acetylamino)benzyl]-4-[(4-hydroxyphenyl)thio]-N-methylbutanamide
      The compound was prepared by saponification (NaOH, methanol) of N-[2-(acetylamino)benzyl]-4-[(4-hydroxyphenyl)thio]-N-methylbutanamide acetate and purified by semi-preparative HPLC.
   NMR-¹H (CDCl₃): δ (ppm) 9.80 (br s, 1H) ; 8.40 (d, 1H) ; 7.41-7.15 (m, 4H) ; 7.04 (dt, 1H) ; 6.76 (d, 2H) ; 6.03 (br s, 1H) ; 4.50 (s, 2H) ; 2.94 (s, 3H) ; 2.87 (t, 2H) ; 2.49 (t, 2H) ; 2.16 (s, 3H) ; 2.00-1.81 (m, 2H)
   MS (ESI+) : *m*/*z* = 373 [M+H]⁺
***Example 117:***
   4-[(4-Fluorophenyl)thio]-N-[2-(2-methylamino-2-oxoethoxy)benzyl]-N-methylbutanamide
   *Step 1:* methyl [2-(1[4-(4-(fluorophenyl)thio)-butyryl]-methylaminol-methyl)-phenoxy]acetate
      To a solution of compound of example 43 (300 mg, 0.9 mmol) in 5 mL of acetonitrile were added anhydrous potassium carbonate (187 mg, 1.35 mmol) and methyl bromoacetate (151 mg, 0.99 mmol). The mixture was refluxed overnight then water was added. The resulting mixture was extracted 3 times with ethyl acetate, the combined organic layers were washed with brine, dried with anhydrous MgSO₄, filtered and concentrated in vacuo. The residue was purified by flash chromatography on silica gel (cyclohexane / ethyl acetate 8/2) to give 300 mg (yield 82%) of the ester.
   *Step 2 :* [2-({[4-(4-(fluorophenyl)thio)-butyryl]-methylamino}-methyl)-phenoxy]acetic acid
      The compound was prepared by saponification (NaOH, methanol) of the ester (step 1) and used without purification.
   *Step* 3: 4-[(4-fluorophenyl)thio]-N-[2-(2-methylamino-2-oxoethoxy)benzyl]-N-methylbutanamide
      The compound was prepared according the procedure described for example 113 in 18% yield.
   NMR-¹H (CDCl₃) : δ (ppm) 8.26 (s, large 1H) ; 7.38-7.27 (m, 3H) ; 7.22 (dd, 1H) ; 7.04-6.91 (m, 3H) ; 6.81 (d, 1H) ; 4.69 (s, 2H) ; 4.41 (s, 2H) ; 3.00-2.84 (m, 5H) ; 2.81 (s, 3H) ; 2.47 (t, 2H) ; 1.95 (p, 2H)
   MS (ESI+) : *m*/*z* = 405 [M+H]⁺
***Example 118 :***
   N-[2-(2-Aminoethoxy)benzyl]-4-[(4-fluorophenyl)thio]-N-methylbutanamide
   *Step 1 :* to a solution of compound of example 43 (200 mg, 0.6 mmol) in 3 mL of acetonitrile were added anhydrous potassium carbonate (124 mg, 0.9 mmol) and commercial 2-(boc-amino)ethyl bromide (148 mg, 0.66 mmol). The mixture was refluxed overnight then water and ethyl acetate were added. The resulting mixture was extracted 3 times with ethyl acetate, the combined organic layers were washed with brine, dried with anhydrous MgSO₄, filtered and concentrated in vacuo. The residue was purified by flash chromatography on silica gel (cyclohexane / ethyl acetate 9/1 to 8/2) to give 100 mg (yield 35%) of the boc derivative.
   *Step 2 :* to a solution of the boc derivative (100 mg, 0.21 mmol) in 3 mL of dichloromethane was added 162 µL of trifluoroacetic acid. The mixture was stirred 3 hours at room temperature, then concentrated in vacuo. Ethyl acetate was added to the residue, and the solution was washed with a saturated solution of NaHCO₃, dried with anhydrous MgSO₄, filtered and concentrated in vacuo. The residue was purified by flash chromatography on reversed-phase silica gel (acetonitrile / water) to give 67 mg (84% yield) of N-[2-(2-aminoethoxy)benzyl]-4-[(4-fluorophenyl)thio]-N-methylbutanamide.
   NMR-¹H (CDCl₃) : δ (ppm) 7.50-6.70 (m, 8H) ; 4.57 (d, 2H) ; 4.17-3.84 (m, 2H) ; 3.16-3.00 (m, 2H) ; 3.03-2.84 (m, 5H) ; 2.49 (dt, 2H) ; 1.96 (quint, 2H)
   MS (ESI+) : *m*/*z* = 377 [M+H]⁺
***Example 119 :***
   N-{2-[2-(Dimethylamino)ethoxy]benzyl}-4-[(4-fluorophenyl)thio]-N-methylbutanamide
      To a solution of compound of example 43 (90 mg, 0.27 mmol) in 2 mL of anhydrous DMF were added sodium hydride (60% in mineral oil, 22 mg, 0.54 mmol). The mixture was stirred 30 minutes at room temperature and 2-dimethylaminoethyl chloride hydrochloride (38.9 mg, 0.27 mmol) was added. The mixture was stirred at 80°C overnight then water was added. The resulting mixture was extracted 3 times with ethyl acetate, the combined organic layers were washed with brine, dried with anhydrous MgSO₄, filtered and concentrated in vacuo. The residue was purified by flash chromatography on reversed-phase silica gel (acetonitrile / water) to give 51 mg (yield 47%) of N-{2-[2-(dimethylamino)ethoxy]benzyl}-4-[(4-fluorophenyl)thio]-N-methylbutanamide.
   NMR-¹H (CDCl₃) : δ (ppm) 7.47-7.06 (m, 4H) ; 7.06-6.80 (m, 4H) ; 4.56 (d, 2H) ; 4.11 (dt, 2H) ; 3.08-2.68 (m, 7H) ; 2.56-2.44 (m, 2H) ; 2.37 (d, 2H) ; 2.07-1.85 (m, 2H)
   MS (ESI+) : *m*/*z* = 405 [M+H]⁺
***Example 120 :***
   2-{[{4-[(4-Hydroxyphenyl)thio]butanoyl}(methyl)amino]methyl}benzoic acid
   Prepared by saponification (NaOH, methanol) of compound of example 46.
   NMR-¹H (DMSO-D6) : δ (ppm) 13.06 (br s, 1H) ; 9.53 (br s, 1H) ; 8.09-7.74 (m, 1H) ; 7.72-6.96 (m, 5H) ; 6.70 (dd, 2H) ; 4.85 (d, 2H) ; 3.00-2.66 (m, 5H) ; 2.32 (t, 1H) ; 1.84-1.58 (m, 2H)
   MS (ESI+) : *m*/*z* = 360 [M+H]⁺
   ***Example 121 :***
   4-[(4-Hydroxyphenyl)thio]-N-methyl-N-(2-piperazin-1-ylbenzyl)butanamide
   *Step 1 :* tert-butyl 4-[2-({4-[(4-hydroxyphenyl)thio]butanoyl}methylamino)-methylphenyl]piperazine-1-carboxylate
   Prepared from 4-[(4-hydroxyphenyl)thio]butanoic acid [85896-82-8] and tert-butyl 4-[2-(methylamino)methylphenyl]piperazine-l-carboxylate (see below) according general procedure B in 67% yield.
   Tert-Butyl 4-[2-(methylamino)methylphenyl]piperazine-1-carboxylate was prepared according general procedure Y from commercial tert- butyl 4-(2-formylphenyl)piperazine-1-carboxylate [174855-57-3].
   *Step 2 :* to a solution of the Boc derivative (158 mg, 0.316 mmol) in 3 mL of dichloromethane was added 244 µL of trifluoroacetic acid. The mixture was stirred 3 hours at room temperature, then water was added. The mixture was extracted with dichloromethane, the combined organic layers were washed with a saturated solution of NaHCO₃, dried with anhydrous MgSO₄, filtered and concentrated in vacuo to give 61 mg (48% yield) of 4-[(4-hydroxyphenyl)thio]-N-methyl-N-(2-piperazin-1-ylbenzyl)butanamide.
   NMR-¹H (CDCl₃) : δ (ppm) 7.29-7.05 (m, 6H) ; 6.78-6.71 (m, 2H) ; 4.62 (d, 2H) ; 3.05-2.78 (m, 13H) ; 2.58-2.53 (t, 1H) ; 2.47-2.42 (t, 1H) ; 2.01-1.86 (m, 2H)
   MS (ESI+) : *m*/*z* = 400 [M+H]⁺
***Example 122 :***
   N-[2-(4-Acetylpiperazin-1-yl)benzyl]-4-({4-[benzyl(methyl)amino]-4-oxobutyl}thio)phenyl acetate
   To a solution of compound of example 121 (60 mg, 0.15 mmol) in 3 mL of dichloromethane were added 49 µL of pyridine and acetic anhydride (31.2 µL, 0.33 mmol). The mixture was refluxed 4 hours then water was added. The mixture was extracted with dichloromethane, the combined organic layers were washed with brine, dried with anhydrous MgSO₄, filtered and concentrated in vacuo. The residue was purified by flash chromatography on silica gel (cyclohexane / ethyl acetate 5/5 to 0/10) to give 56 mg (77% yield) of N-[2-(4-acetylpiperazin-1-yl)benzyl]-4-({4-[benzyl(methyl)amino]-4-oxobutyl}thio)phenyl acetate.
   NMR-¹H (CDCl₃) : δ (ppm) 7.38-6.95 (m, 8H) ; 4.66 (d, 2H) ; 3.82-3.67 (m, 2H) ; 3.64-3.53 (m, 2H) ; 3.09-2.78 (m, 9H) ; 2.51 (dt, 2H) ; 2.28 (s, 3H) ; 2.14 (d, 3H) ; 2.13-1.90 (m, 2H)
   MS (ESI+) : *m*/*z* = 484 [M+H]⁺
***Example 123 :***
   4-[(4-Hydroxyphenyl)thio]-N-(2-methoxybenzyl)-N-methylbutanethioamide
      To a solution of compound of example 35 (300 mg, 0.868 mmol) in 5 mL of toluene was added Lawesson's Reagent (351 mg, 0.868 mmol). The mixture was refluxed 2 hours then concentrated in vacuo. The residue were co-evaporated 3 times with dichloromethane then purified by flash chromatography on reversed-phase silica gel (acetonitrile / water) to give 24 mg (8% yield) of 4-[(4-hydroxyphenyl)thio]-N-(2-methoxybenzyl)-N-methylbutanethioamide.
   NMR-¹H (CDCl₃) : δ (ppm) 7.33-7.14 (m, 4H) ; 6.97-6.85 (m, 2H) ; 6.79-6.68 (m, 2H) ; 5.04 (d, 2H) ; 3.82 (d, 3H) ; 3.27 (d, 3H) ; 2.98-2.82 (m, 4H) ; 2.14-1.99 (m, 2H)
   MS (ESI+) : *m*/*z* = 362 [M+H]⁺
***Example 124 :***
   N-[2-(4-Acetylpiperazin-1-yl)benzyl]-4-[(4-hydroxyphenyl)thio]-N-methylbutanamide
   Prepared by saponification (NaOH, methanol) of compound of example 122 in 34% yield.
   NMR-¹H (CDCl₃) : δ (ppm) 7.34-7.05 (m, 6H) ; 6.80-6.73 (m, 2H) ; 4.66 (d, 2H) ; 3.74-3.55 (m, 4H) ; 2.95-2.79 (m, 9H) ; 2.51 (dt, 2H) ; 2.16-2.13 (m, 3H) ; 2.03-1.89 (m, 2H)
   MS (ESI+) : *m*/*z*= 442 [M+H]⁺
***Example 125 :***
   4-[(4-{Methyl[2-(trifluoromethyl)benzyl]amino}-4-oxobutyl)thio]benzoic acid
   Prepared by saponification (NaOH, methanol) of compound of example 104 in 99% yield.
   NMR-¹H (CDCl₃) : δ (ppm) 8.03-7.89 (m, 2H) ; 7.73-7.15 (m, 6H) ; 4.76 (d, 2H) ; 3.14 (t, 1H) ; 3.04 (t, 1H) ; 2.96 (d, 3H) ; 2.62 (t, 1H) ; 2.45 (t, 1H) ; 2.20-1.96 (m, 2H)
   MS (ESI+) : *m*/*z* = 442 [M+H]⁺
***Example 126 :***
   4-[(4-Fluorophenyl)sulfonyl]-N-(2-methoxybenzyl)-N-methylbutanamide
      To a solution of compound of example 25 (79 mg, 0.227 mmol) in 2.4 mL of acetic acid were added 65 µL of hydrogen peroxide (35 wt.% solution in water). The mixture was stirred 2 hours at room temperature then refluxed 1 hour. Water was added and the mixture was extracted with dichloromethane. The combined organic layers were washed with H₂O then brine, dried with anhydrous MgSO₄, filtered and concentrated in vacuo. The residue was purified by flash chromatography on silica gel (dichloromethane / ethanol 100/0 to 95/5) to give 49 mg (57% yield) of 4-[(4-fluorophenyl)sulfonyl]-N-(2-methoxybenzyl)-N-methylbutanamide.
   NMR-¹H (CDCl₃) : δ (ppm) 8.03-7.77 (m, 2H) ; 7.34-7.05 (m, 3H) ; 7.03-6.76 (m, 3H) ; 4.50 (d, 2H) ; 3.83 (d, 3H) ; 3.30-3.15 (m, 2H) ; 2.92 (d, 3H) ; 2.60-2.44 (m, 2H) ; 2.16-1.92 (m, 2H) ;
   MS (ESI+) : *m*/*z* = 380 [M+H]⁺
***Example 127:***
   4-[(4-Hydroxyphenyl)thio]-N-methyl-N-[2-(1,2,3,6-tetrahydropyridin-4-yl)benzyl]butanamide
   *Step 1 :* N-(2-bromobenzyl)-4-[(4-hydroxyphenyl)thio]-N-methylbutanamide
      Prepared from 4-[(4-fluorophenyl)thio]butanoic acid [18850-56-1] and commercial 2-bromo-N-methylbenzylamine in 64% yield.
   *Step 2 :* N-(2-bromobenzyl)-4-[(4-hydroxyphenyl)thio]-N-methylbutanamide (200 mg, 0.507 mmol), commercial 3,6-dihydro-2H-pyridine-1-tert-butoxycarbonyl-4-boronic acid pinacol ester (156.8 mg, 0.507 mmol), Pd(PPh₃)₄ (29.3 mg, 0.025 mmol), LiCl (64.5 mg, 1.521 mmol), 1.2 mL of an aqueous solution 1 M of Na₂CO₃, 1 mL of toluene, 1 mL of THF and 0.5 mL of ethanol were charged in a sealed vial and heated 15 minutes at 120°C on a microwave apparatus (Biotage), then 10 mg of Pd(PPh₃)₄ were added and the mixture was heated 30 minutes at 150°C. Water was added and the mixture was extracted 3 times with ethyl acetate. The combined organic layers were washed with brine, dried with anhydrous MgSO₄, filtered and concentrated in vacuo. The residue was purified by flash chromatography on silica gel (cyclohexane / ethyl acetate) to give 255 mg (100% yield) of 4-[(4-hydroxyphenyl)thio]-N-methyl-N-[2-(N'-boc-1,2,3,6-tetrahydropyridin-4-yl)benzyl]butanamide.
   *Step 3 :* to a solution of the Boc derivative (255 mg, 0.507 mmol) in 3 mL of dichloromethane was added 391 µL of trifluoroacetic acid. The mixture was stirred 3 hours at room temperature, then basified with 1 N NaOH. The mixture was extracted 3 times with dichloromethane, the combined organic layers were washed with brine, dried with anhydrous MgSO₄, filtered and concentrated in vacuo. The residue was purified by flash chromatography on silica gel (dichloromethane / ethanol 99/1 to 90/10) to give 42 mg (21% yield) of 4-[(4-hydroxyphenyl)thio]-N-methyl-N-[2-(1,2,3,6-tetrahydropyridin-4-yl)benzyl]butanamide.
   NMR-¹H (DMSO-D6) : δ (ppm) 7.73-7.18 (m, 6H) ; 6.94 (t, 2H) ; 5.87-5.70 (m, 1H) ; 4.80-4.65 (m, 2H) ; 3.70-3.28 (m, 3H) ; 3.22 (t, 1H) ; 3.14-2.87 (m, 5H) ; 2.64-2.35 (m, 4H) ; 2.05-1.80 (m, 2H)
   MS (ESI+) : *m*/*z* = 397 [M+H]⁺
***Example 128 :***
   4-[(4-Fluorophenyl)thio]-N-methyl-N-1-naphthylbutanamide
   *Step 1 :* 4-[(4-fluorophenyl)thio]-N-1-naphthylbutanamide
   Prepared from 4-[(4-fluorophenyl)thio]butanoic acid [18850-56-1] and commercial 1-naphthylamine in 83% yield.
   *Step 2 :* to a solution of 4-[(4-fluorophenyl)thio]-N-1-naphthylbutanamide (100 mg, 0.295 mmol) in 2 mL of N,N-dimethylformamide were added sodium hydride (60% dispersion in mineral oil, 12 mg, 0.295 mmol). The mixture was stirred 30 minutes at room temperature and iodomethane (20 µL, 0.325 mmol) were added. The mixture was stirred 3 hours at room temperature then water was added. The resulting mixture was extracted 3 times with ethyl acetate, the combined organic layers were dried with anhydrous MgSO₄, filtered and concentrated in vacuo. The residue was purified by flash chromatography on silica gel (cyclohexane / ethyl acetate 9/1) to give 64 mg (62% yield) of 4-[(4-fluorophenyl)thio]-N-methyl-N-1-naphthylbutanamide.
   NMR-¹H (CDCl₃) : δ (ppm) 8.01-7.85 (m, 2H) ; 7.82-7.70 (m, 1H) ; 7.65-7.45 (m, 3H) ; 7.38-7.29 (dd, 1H) ; 7.23-7.12 (m, 2H) ; 6.97-6.84 (m, 2H) ; 3.35 (s, 3H) ; 2.75 (dt, 2H) ; 2.27-1.75 (m, 4H)
   MS (ESI+) : *m*/*z* = 354 [M+H]⁺

### 2) Biological results

Several assays have been described to measure the antiviral activity anti-HIV by compounds. The preferred assays are based on multiple rounds infection experiments by measuring the production of capsid antigen CA p24 by HIV infected cells, or on single round infection experiments by assay of the activity of a reporter luciferase gene inserted in the viral genome deleted of its Env gene.

### 1) Single round infection assay of the expression of the Firefly Luciferase reporter gene harboured by Env-deleted NL4-3 vector virus pseudotyped with the VSVG envelope

To generate the envelope-deleted NL4-3 vector (NL4-3Δenv), a frameshift was introduced in the *env* gene. The Ndel site (nt6399) in pNL4-3 was digested, filled with Klenow, and religated. The Firefly Luciferase gene (De Wet et al Mol Cell Biol. 1987 Feb; 7(2):725-37.) was then inserted in the Nef gene at the XBO1 site resulting in the pNL4-3Δ*env*- Luc plasmid.

Virus stocks were produced by co-transfecting human embryonic kidney 293 T cells using the calcium phosphate method with the pNL4-3Δ*env* -Luc construct and the pSFFV-gE expression vector encoding the varicella-zoster virus [VZV] gE envelope. Supernatants were collected 2 days after transfection, and levels of HIV-1 p24 antigen were monitored by enzyme-linked immunoabsorbent assay (BD Biosciences). 25. 10⁶ Sup T1 or MT4 target cells were infected with viral doses corresponding to MOI 0.01 during 2hours at 37°C in 24 well plates.

After centrifugation at 3000rpm and washings in PBS, pellet of infected cells were diluted in medium and plated in 96 well plates in the presence of test compounds at various concentrations. At day 2 post-infection, cells were lysed and luciferase activity was measured using the PROMEGA* Luciferase Assay System-10 pack (1000 assays)* (10x10ml), ref:*E1501) according manufacturer"s instructions. Cell viability was determined in parallel by MTT cell proliferation assay from LGC Promochem (ATCC), *MTT Cell Proliferation Assay, 2500 tests*, ref: *30-1010K, according manufacturer's instructions. EC50 for antiviral activity corresponded to the concentration of compound effective for a 50% inhibition of luciferase activity.

The CC50 of cytotoxicity of the tested compound corresponded to the concentration that resulted in the decrease of MTT assay of 50%. The therapeutic index was estimated by the ratio of the cytotoxicity CC50 versus the antiviral activity EC50

All compounds have been tested in this assay; in particular, the following compounds present an EC50 below 5 µM : Examples 3, 10, 11, 12, 13, 14, 15, 17, 19, 20, 22, 26, 27, 29, 30, 31, 34, 35, 37, 39, 41, 42, 47, 50, 53, 56, 59, 65, 69, 70, 71, 74, 75, 76, 80, 81, 84, 85, 87, 90, 92, 95, 97, 102, 106, 107, 114, 121, 123, 127, 128.

### 2) Multiple rounds infection assay of CAp24 antigen production (according to Emilianiet al, J Biol Chem. 2005 Jul 8; 280(27):25517-23):

Virus stock of the NL4-3 strain of HIV-1 (Adachi et al, J Virol. 1986 Aug;59(2):284-91) was prepared as described in Lopez-Vergès et al. Proc Natl Acad Sci U S A. 2006 Oct 3; 103(40):14947-52, by transfecting 1.5 x 10⁶ 293T cells with 20µg of NL4-3 proviral DNA by using calcium phosphate procedure. Forty eight hours later, transfected cell supernatants were harvested, filtered through 0.22-µm-pore-size filters, quantified for HIV-1 p24 antigen by using a Coulter HIV-1 p24 antigen assay (Beckman Coulter) according manufacturer's instructions, and used in infection experiments.

8.10⁶ SupT1 or MT4 cells were infected with NL4-3 virus at a multiplicity of infection (MOI) 0.01, or mock infected with medium only (RPMI), during two hours at 37°C in 24 well plates. After centrifugation at 3000 rpm and washings in PBS, pellet of infected cells were diluted in medium and plated in 96 well plates in the presence of test compounds at various concentrations. At day 4 and day -6 post-infection, aliquots of supernatant were taken and assayed for CA p24 production by Elisa assay (BD Biosciences). Cell viability was determined in parallel by MTT cell proliferation assay from LGC Promochem (ATCC), *MTT Cell Proliferation Assay, 2500 tests*, ref: *30-1010K, according manufacturer's instructions. EC50 for antiviral activity corresponded to the concentration of compound effective for a 50% inhibition of CA p24 production.

The CC50 of cytotoxicity of the tested compound corresponded to the concentration that resulted in the decrease of MTT assay of 50%. The therapeutic index was estimated by the ratio of the cytotoxicity CC50 versus the antiviral activity EC50.

The compounds tested in this assay thus confirmed that they are active against wild viruses.

Several assays can measure the inhibition of HIV integration. The preferred assay is based on:

### 3) Quantitation of HIV DNA integration ex vivo in infected cells by Q-PCR analysis (according to Butler et al. Nat Med. 2001 May;7(5):631-4 ; and Brussel et al.,Methods Mol Biol. 2005; 304:139-54):

Target cells (SupT1 or MT4) were infected in single cycle infection experiment with the pNL4-3Δ*env* -Luc pseudotyped with the VZVgE envelope, and treated with tested compounds as described above, except that virus preparation was pre-treated with DNAse prior infection, to avoid contamination of Q-PCR species with proviral plasmid DNA. At different times postinfection (8h, 24h, and 48h), cells were harvested, washed in phosphate-buffered saline, and treated for 1 h at 37 °C with 500 units of DNasel (Roche Diagnostics) prior to DNA extraction using a QlAamp blood DNA mini kit (Qiagen). The amounts of total HIV-1 DNA, two-LTR circles, and integrated HIV-1 DNA were quantified by real-time PCR performed in the Light Cycler instrument (Roche Applied Science) as described in Brussel et al. 2005. Each sample was analyzed in duplicate. Briefly, the total HIV-1 DNA copy number was determined using primer that annealed in the U5 region of the LTR (MH531) and in the 5' region of the gag gene (MH532). Integrated DNA was quantified using an Alu-LTR-based nested PCR procedure. In a first round of PCR, integrated HIV-1 sequences were amplified with two outward facing Alu primers and a HIV-1 LTR-specific primer (L-M667) containing a λphage-specific sequence at the 5'-end of the oligonucleotide. In a second round of PCR, we used specific primers for the λsequence (λ T) and the LTR region (AA5M) (Brussel et al. 2005). To eliminate the signal due to primer extension carried out by the L-M667 primer during the first round PCR, a control PCR assay was performed without Alu primers. The signal of the nested PCR obtained in the absence of Alu primers was subtracted from the integrated HIV-1 DNA signal. Copy numbers of total DNA two-LTR circles and integrated DNA were determined in reference to standard curves prepared by amplification of cloned DNA with matching sequences (Brussel et al. 2005). Results were normalized by the number of cells and the amount of cellular DNA quantified by PCR of the β-globin gene according manufacturer's instructions (Roche Applied Science).

This test was used to demonstrate that the compounds of the invention inhibit the DNA integration.

## Claims

1. Compounds of formula (I): 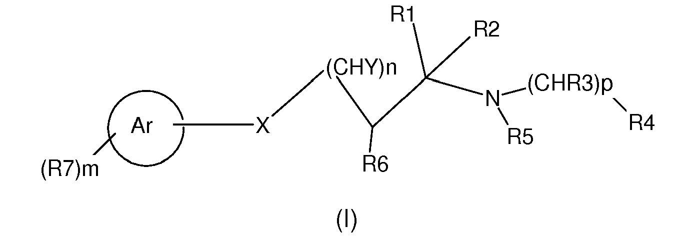 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O) R;
Or a R3 form with a substituent of R4 a cycloalkyl, aryl, heteroaryl or heterocycle fused with said R4;
Or a R3 form with R5 a N-containing heterocyle or heteroaryl;
R4 represents an aryl, heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
- halogen atom; -Alkyl; -Aryl; -OAryl; -Alkyl-OR;
- Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
- C(=O)R; -C(=O)NRR'; -O-Alkyl-C(=O)NRR', -O-Alkyl-C(=O)OR, -OAlkyl-NRR', -NR-C(=O)R'; -C(=O)OR; -S(O)R, -S(O)₂R; -OC(=O)R; -OAlkenyl;
- Alkenyl;
- Heterocycle optionally substituted by an -Alkyl, -AlkylOR, -AlkylNRR',
- Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally comprising one or more further heteroatom and optionally substituted by a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 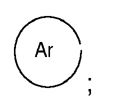
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 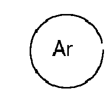
represents an aryl, heteroaryl or unsaturated heterocycle;
m is an integer comprised between 1 and 5, provided that when 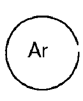 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts,
with the exception of the following compounds :
- the compounds where Ar is a tetrazol, X is S, n is 2, R6 is H, R1 and R2 form together a C=O with the carbon atom to which they are attached; and
- the compounds of formulae :

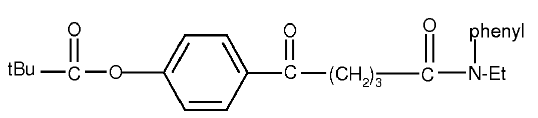

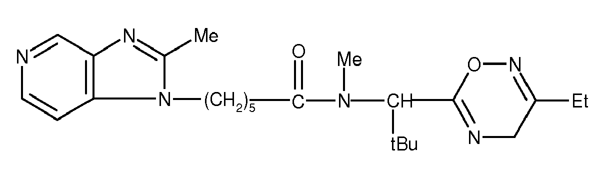

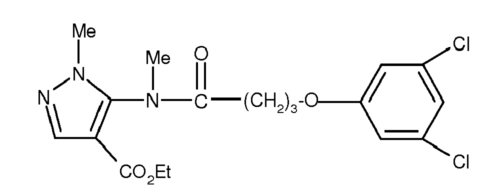

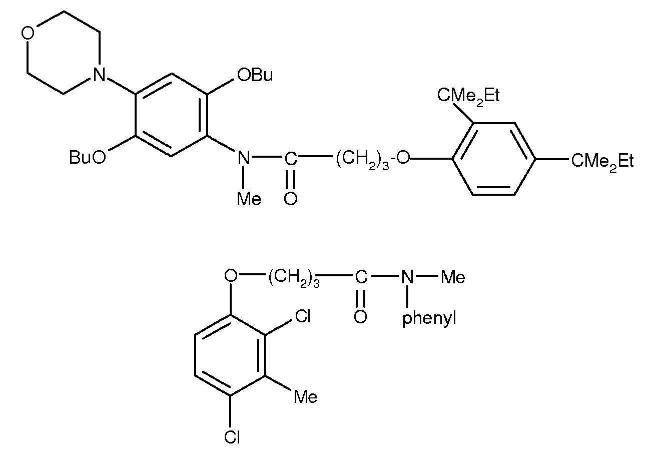

2. Compounds of formula (I) wherein X represents a group chosen from -CRR'-, -NT-, -O-, -S(O)q-, -C(=O)-, where the R of -NR- may be optionally linked with a R7 to form with the N atom to which it is attached a N-containing heterocycle fused with said Ar.

3. Compounds of formula (I), wherein:
R4 represents an aryl or heteroaryl said aryl or heteroaryl group being optionally fused with an aryl, heteroaryl, saturated or unsaturated cycloalkyl or heterocyclic ring and said optionally fused aryl or heteroaryl being optionally substituted by one or more substituent(s) chosen from:
- halogen atom; -Alkyl; -Aryl; -OAryl; -Alkyl-OR;
- Heterocycle optionally substituted by an -Alkyl, -AlkylOR, -AlkylNRR',
- Alkylaryl, -OR, -Alkyl-Heterocycle, -C(=O)Alkyl or -C(=O)OAlkyl group ;
- Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl;
- OR; -NRR'; =O; -C(=O)R; -C(=O)NRR'; -O-Alkyl-C(=O)NRR', -OAlkyl-NRR', -NR-C(=O)R'; -C(=O)OR; -S(O)R -S(O)₂R; -OC(=O)R; 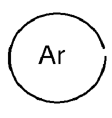
represents an aryl or heteroaryl.

4. Compounds of formula (I) according to claim 1, 2 or 3, wherein R1, R2 form together a C=O group with the carbon atom to which they are attached.

5. Compounds according to anyone of the preceding claims, wherein R3 represents a H atom or a group chosen from -alkyl, -aryl.

6. Compounds according to anyone of the preceding claims, wherein R4 represents an aryl optionally fused with an aryl, heteroaryl, or saturated heterocyclic ring and said optionally fused aryl being optionally substituted by one or more substituent(s) chosen from:
- halogen atom; -Alkyl; -Aryl; -OAryl; -Alkyl-OR;
- Heterocycle optionally substituted by an -Alkyl, -AlkylOR, -AlkylNRR',
- Alkylaryl, -OR, -Alkyl-Heterocycle, -C(=O)Alkyl or -C(=O)OAlkyl group ;
- Alkylaryl; -Alkyl-Heterocycle; perfluoroalkyl-; polyfluoroalkyl-; polyfluoroalkoxy-; -OHeterocycle-Alkyl;
- OR; -O-Alkyl-C(=O)NRR', -OAlkyl-NRR', -NR-C(=O)R'; -C(=O)OR;
- OC(=O)R.

7. Compounds according to anyone of the preceding claims, wherein R5 represents a group -Alkyl, -Alkyl-OR, or -Cycloalkyl, each being optionally substituted by one or more substituents chosen from -polyfluoroalkyl groups.

8. Compounds according to anyone of the preceding claims, wherein R6 represents a H atom or a group chosen from -C(=O)NRR', -C(=O)OR.

9. Compounds according to anyone of the preceding claims, wherein R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -OAlkylAryl; polyfluoroalkyl-; or 2 R7 form together with the atoms to which they are attached an unsubstituted ring chosen from aryl, heteroaryl, or unsaturated heterocyclic ring fused with

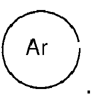

10. Compounds according to anyone of the preceding claims, wherein X represents a group chosen from -CRR'-, -NT-, -S-, -S(O)-, -C(=O)-.

11. Compounds according to anyone of the preceding claims, wherein Y represents a hydrogen atom.

12. Compounds according to anyone of the preceding claims, wherein 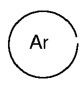 represents an aryl.

13. Compounds according to anyone of the preceding claims, wherein m is 1 or 2.

14. Compounds according to anyone of the preceding claims, wherein n is 2.

15. Compounds according to anyone of the preceding claims, wherein p is 1.

16. A compound of formula (I) according to anyone of the preceding claims chosen from the group consisting in:
N-benzyl-4-[(4-hydroxyphenyl)thio]-N-methylbutanamide
N,N-dibenzyl-4-[(4-hydroxyphenyl)thio]butanamide
4-[(4-hydroxyphenyl)thio]-N-methyl-N-(pyridin-3-ylmethyl)butanamide
N-ethyl-4-[(4-hydroxyphenyl)thio]-N-(pyridin-4-ylmethyl)butanamide
N-benzyl-4-(4-methoxyphenoxy)-N-methylbutanamide
4-(4-methoxyphenoxy)-N-methyl-N-(pyridin-3-ylmethyl)butanamide
N-benzyl-4-[(3-hydroxyphenyl)thio]-N-methylbutanamide
4-[(3-hydroxyphenyl)thio]-N-methyl-N-(pyridin-3-ylmethyl)butanamide
N-benzyl-4-[(4-methoxyphenyl)thio]-N-methylbutanamide
N,N-dibenzyl-4-[(4-methoxyphenyl)thio]butanamide
4-[(4-methoxyphenyl)thio]-N-methyl-N-(pyridin-3-ylmethyl)butanamide
N,N-dibenzyl-4-[(3-hydroxyphenyl)thio]butanamide
N,N-dibenzyl-4-(4-methoxyphenoxy)butanamide
N-benzyl-4-(4-benzyloxyphenoxy)-N-methylbutanamide
4-[(4-hydroxyphenyl)thio]-N-methyl-N-[1-phenylethyl]butanamide
N-(4-chlorobenzyl)-4-[(4-hydroxyphenyl)thio]-N-methylbutanamide
N-(2-chlorobenzyl)-4-[(4-hydroxyphenyl)thio]-N-methylbutanamide
N-(4-fluorobenzyl)-4-[(4-hydroxyphenyl)thio]-N-methylbutanamide
4-[(4-hydroxyphenyl)thio]-N-(4-methoxybenzyl)-N-methylbutanamide
4-[(4-hydroxyphenyl)thio]-N-methyl-N-[4-(trifluoromethyl)benzyl]butanamide
4-[(4-hydroxyphenyl)thio]-N-methyl-N-(4-methylbenzyl)butanamide
N-benzyl-4-(4-hydroxyphenoxy)-N-methylbutanamide
N-(3-chlorobenzyl)-4-[(4-hydroxyphenyl)thio]-N-methylbutanamide
4-[(4-hydroxyphenyl)thio]-N-methyl-N-phenylbutanamide
N-benzhydryl-4-[(4-hydroxyphenyl)thio]-N-methylbutanamide
4-{[4-oxo-4-(2-phenyl-4,5-dihydro-1H-imidazol-1-yl)butyl]thio}phenol
4-[(4-hydroxyphenyl)thio]-N,N-bis(pyridin-2-ylmethyl)butanamide
4-[(4-hydroxyphenyl)thio]-N-methyl-N-(2-methylbenzyl)butanamide
N-(2,4-dichlorobenzyl)-4-[(4-hydroxyphenyl)thio]-N-methylbutanamide
4-[(4-hydroxyphenyl)thio]-N-(2-methoxybenzyl)-N-methylbutanamide
N-(2,3-dichlorobenzyl)-4-[(4-hydroxyphenyl)thio]-N-methylbutanamide
N-(2-fluorobenzyl)-4-[(4-hydroxyphenyl)thio]-N-methylbutanamide
N-(2,6-dichlorobenzyl)-4-[(4-hydroxyphenyl)thio]-N-methylbutanamide
N-(2-chlorobenzyl)-4-[(4-hydroxyphenyl)sulfinyl]-N-methylbutanamide
N-(2-chloro-6-fluorobenzyl)-4-[(4-hydroxyphenyl)thio]-N-methylbutanamide
N-(2-ethoxybenzyl)-4-[(4-hydroxyphenyl)thio]-N-methylbutanamide
N-(2,3-dimethoxybenzyl)-4-[(4-hydroxyphenyl)thio]-N-methylbutanamide
N-(2-furylmethyl)-4-[(4-hydroxyphenyl)thio]-N-methylbutanamide
4-[(4-hydroxyphenyl)thio]-N-methyl-N-[2-(trifluoromethoxy)benzyl]butanamide
4-[(3-hydroxyphenyl)thio]-N-(2-methoxybenzyl)-N-methylbutanamide
4-[(4-fluorophenyl)thio]-N-(2-methoxybenzyl)-N-methylbutanamide
4-[(4-hydroxyphenyl)thio]-N-methyl-N-(pyridin-2-ylmethyl)butanamide
N-(2-hydroxybenzyl)-4-[(4-hydroxyphenyl)thio]-N-methylbutanamide
N-(2,3-dihydro-1-benzofuran-7-ylmethyl)-4-[(4-hydroxyphenyl)thio]-N-methylbutanamide
4-[(2-hydroxyphenyl)thio]-N-(2-methoxybenzyl)-N-methylbutanamide
4-{[4-(acetylamino)phenyl]thio}-N-(2-methoxybenzyl)-N-methylbutanamide
5-(4-hydroxyphenyl)-N-(2-methoxybenzyl)-N-methylpentanamide
N-(2-methoxybenzyl)-N-methyl-4-{[4-(methylsulfonylamino)phenyl]thio}butanamide
N-[2-(acetylamino)benzyl]-4-[(4-hydroxyphenyl)thio]-N-methylbutanamide
4-[(4-hydroxyphenyl)thio]-N-methyl-N-(1-naphthylmethyl)butanamide
N-(2-methoxybenzyl)-N-methyl-4-(2-naphthylthio)butanamide
N-(2-methoxybenzyl)-N-methyl-4-{[4-(trifluoromethyl)phenyl]thio}butanamide
N-(2-methoxybenzyl)-N-methyl-4-(quinolin-2-ylthio)butanamide
N-(1,3-benzodioxol-4-ylmethyl)-4-[(4-hydroxyphenyl)thio]-N-methylbutanamide
N-(2-methoxybenzyl)-N-methyl-4-{[5-(trifluoromethyl)pyridin-2-yl]thio}butanamide
4-[(4-hydroxyphenyl)thio]-N-methyl-N-[2-(trifluoromethyl)benzyl]butanamide
4-[(4-hydroxyphenyl)thio]-N-(2-isopropoxybenzyl)-N-methylbutanamide
N-(2-methoxybenzyl)-4-[(4-methoxyphenyl)thio]-N-methylbutanamide
4-[(5-chloropyridin-2-yl)thio]-N-(2-methoxybenzyl)-N-methylbutanamide
N-(2-chlorobenzyl)-4-[(4-fluorophenyl)thio]-N-methylbutanamide
Methyl 4-({4-[(2-methoxybenzyl)methylamino]-4-oxobutyl}thio)benzoate
N-(2-methoxybenzyl)-N-methyl-4-(quinoxalin-2-ylthio)butanamide
4-[(4-fluorophenyl)thio]-N-(2-hydroxybenzyl)-N-methylbutanamide
4-[(4-hydroxyphenyl)thio]-N-methyl-N-[2-(4-methylpiperazin-1-yl)benzyl]butanamide
4-({4-[(2-methoxybenzyl)(methyl)amino]-4-oxobutyl}thio)benzoic acid
4-[(4-fluorophenyl)thio]-N-(2-methoxyphenyl)-N-methylbutanamide
4-({4-[(2-methoxybenzyl)(methyl)amino]-4-oxobutyl}thio)-N-methylbenzamide
4-[(4-fluorophenyl)(methyl)amino]-N-(2-methoxybenzyl)-N-methylbutanamide
4-[(4-hydroxyphenyl)thio]-N-methyl-N-(2-morpholin-4-ylbenzyl)butanamide
5-[(4-hydroxyphenyl)thio]-N-(2-methoxybenzyl)-N-methylpentanamide
4-[(4-fluorophenyl)thio]-N-[2-(2-methylamino-2-oxoethoxy)benzyl]-N-methylbutanamide
N-[2-(2-aminoethoxy)benzyl]-4-[(4-fluorophenyl)thio]-N-methylbutanamide
4-(1H-indol-5-yloxy)-N-(2-methoxybenzyl)-N-methylbutanamide
N-(2,3-dichlorobenzyl)-4-[(4-fluorophenyl)thio]-N-methylbutanamide
N-{2-[2-(dimethylamino)ethoxy]benzyl}-4-[(4-fluorophenyl)thio]-N-methylbutanamide
Methyl 2-{[{4-[(4-hydroxyphenyl)thio]butanoyl}(methyl)amino]-methyl}benzoate
2-{[{4-[(4-hydroxyphenyl)thio]butanoyl}(methyl)amino]methyl}benzoic acid
Ethyl 4-[2-({4-[(4-hydroxyphenyl)thio]butanoyl}methylamino)methylphenyl]piperazine-1-carboxylate
4-[(4-hydroxyphenyl)thio]-N-[2-(4-hydroxypiperidin-1-yl)benzyl]-N-methylbutanamide
4-[(4-hydroxyphenyl)thio]-N-methyl-N-(2-phenoxybenzyl)butanamide
4-[(4-hydroxyphenyl)thio]-N-methyl-N-(2-piperazin-1-ylbenzyl)butanamide
4-[(4-fluorophenyl)thio]-N-(2-isopropoxybenzyl)-N-methylbutanamide
4-[(4-fluorophenyl)thio]-N-methyl-N-(2-morpholin-4-ylbenzyl)butanamide
Methyl 4-[(4-hydroxyphenyl)thio]-2-{[(2-methoxybenzyl)(methyl)amino]-carbonyl}butanoate
4-[(4-hydroxyphenyl)thio]-N-methyl-N-(2-benzylbenzyl)butanamide
4-[(4-hydroxyphenyl)thio]-N-methyl-N-{2-[4-(2-morpholin-4-ylethyl)piperazin-1-yl]benzyl}butanamide
N-[2-(4-acetylpiperazin-1-yl)benzyl]-4-({4-[benzyl(methyl)amino]-4-oxobutyl}thio)phenyl acetate
4-[(4-hydroxyphenyl)thio]-2-{[(2-methoxybenzyl)(methyl)amino]carbonyl}butanoic acid
N-[2-(4-benzylpiperazin-1-yl)benzyl]-4-[(4-hydroxyphenyl)thio]-N-methylbutanamide
5-(4-hydroxyphenyl)-N-methyl-N-(2-morpholin-4-ylbenzyl)pentanamide
5-(4-fluorophenyl)-N-(2-methoxybenzyl)-N-methyl-5-oxopentanamide
N-(1,1'-biphenyl-2-ylmethyl)-4-[(4-hydroxyphenyl)thio]-N-methylbutanamide
4-[(4-hydroxyphenyl)thio]-N-(2-methoxybenzyl)-N-methylbutanethioamide
5-(4-fluorophenyl)-N-(2-methoxybenzyl)-N-methylpentanamide
N-[2-(4-acetylpiperazin-1-yl)benzyl]-4-[(4-hydroxyphenyl)thio]-N-methylbutanamide
4-[(3,4-difluorophenyl)thio]-N-(2-methoxybenzyl)-N-methylbutanamide
4-[(3-fluorophenyl)thio]-N-(2-methoxybenzyl)-N-methylbutanamide
2-{2-[(4-hydroxyphenyl)thio]ethyl}-N-(2-methoxybenzyl)-N,N'-dimethylmalonamide
5-(4-hydroxyphenyl)-N-(2-isopropoxybenzyl)-N-methylpentanamide
N-(2-hydroxyethyl)-4-[(4-hydroxyphenyl)thio]-N-(2-methoxybenzyl)butanamide
4-[(4-fluorophenyl)methylamino]-N-(2-isopropoxybenzyl)-N-methylbutanamide
4-[(4-fluorophenyl)(methyl)amino]-N-methyl-N-[2-(trifluoromethyl)benzyl]butanamide
4-[(2,4-difluorophenyl)thio]-N-(2-methoxybenzyl)-N-methylbutanamide
N-{2-[4-(2-dimethylaminoethyl)piperazin-1-yl]benzyl}-4-[(4-hydroxyphenyl)thio]-N-methylbutanamide
4-[(4-hydroxyphenyl)thio]-N-(2-methoxybenzyl)-N-(2-morpholin-4-ylethyl)butanamide
4-[(4-hydroxyphenyl)thio]-N-[2-(methoxymethyl)benzyl]-N-methylbutanamide
N-cyclopropylmethyl-4-[(4-hydroxyphenyl)thio]-N-(2-methoxybenzyl)butanamide
N-ethyl-4-[(4-hydroxyphenyl)thio]-N-(2-methoxybenzyl)butanamide
N-(3-fluoro-2-methoxybenzyl)-4-[(4-hydroxyphenyl)thio]-N-methylbutanamide
4-[(4-hydroxyphenyl)thio]-N-(2-methoxy-3-methylbenzyl)-N-methylbutanamide
N-(5-chloro-2-methoxybenzyl)-4-[(4-hydroxyphenyl)thio]-N-methylbutanamide
4-[(3-chloro-4-fluorophenyl)thio]-N-(2-methoxybenzyl)-N-methylbutanamide
4-[(2-chloro-4-fluorophenyl)thio]-N-(2-methoxybenzyl)-N-methylbutanamide
N-(5-fluoro-2-methoxybenzyl)-4-[(4-hydroxyphenyl)thio]-N-methylbutanamide
4-[(4-hydroxyphenyl)thio]-N-methyl-N-[2-(morpholin-4-ylmethyl)benzyl]butanamide
N-{2-[4-(2-hydroxyethyl)piperazin-1-yl]benzyl}-4-[(4-hydroxyphenyl)thio]-N-methylbutanamide
N-(3,5-dichloro-2-methoxybenzyl)-4-[(4-hydroxyphenyl)thio]-N-methylbutanamide
4-[(4-hydroxyphenyl)thio]-N-(2-methoxy-4-methylbenzyl)-N-methylbutanamide
4-[(4-fluorophenyl)thio]-N-(2-methoxybenzyl)-N-(2,2,2-trifluoroethyl)butanamide
(3S)-4-[(4-fluorophenyl)thio]-3-hydroxy-N-(2-methoxybenzyl)-N-methylbutanamide
Methyl 4-({4-[methyl-(2-trifluoromethylbenzyl)amino]-4-oxobutyl}thio)benzoate
N-cyclopropyl-4-[(4-hydroxyphenyl)thio]-N-(2-methoxybenzyl)butanamide
4-[(4-{methyl[2-(trifluoromethyl)benzyl]amino}-4-oxobutyl)thio]benzoic acid
Methyl 5-fluoro-2-({4-[methyl-(2-methoxybenzyl)amino]-4-oxobutyl}thio)benzoate
4-[(4-fluorophenyl)methylamino]-N-[2-(4-methylpiperazin-1-yl)benzyl]-N-methylbutanamide
4-[(4-hydroxyphenyl)thio]-N-methyl-N-{2-[(1-methylpiperidin-4-yl)oxy]benzyl}butanamide
5-fluoro-2-({4-[(2-methoxybenzyl)(methyl)amino]-4-oxobutyl}thio)benzoic acid
4-[(4-hydroxyphenyl)thio]-N-methyl-N-[2-(1,2,3,6-tetrahydropyridin-4-yl)benzyl]butanamide
4-[(4-fluorophenyl)sulfonyl]-N-(2-methoxybenzyl)-N-methylbutanamide
4-[(4-fluorophenyl)(2-hydroxyethyl)amino]-N-(2-methoxybenzyl)-N-methylbutanamide
4-[(4-fluorophenyl)methylamino]-N-methyl-N-(2-{4-[5-(trifluoromethyl)pyridin-2-yl]piperazin-1-yl}benzyl)butanamide
4-(5-fluoro-2,3-dihydro-1H-indolyl-1-yl)-N-(2-isopropoxybenzyl)-N-methylbutanamide
4-(3,4-dihydroquinolin-1 (2H)-yl)-N-(2-isopropoxybenzyl)-N-methylbutanamide
4-(5-fluoro-2,3-dihydro-1H-indolyl-1-yl)-N-(2-methoxybenzyl)-N-methylbutanamide
4-[(4-fluorophenyl)(2-morpholin-4-ylethyl)amino]-N-(2-methoxybenzyl)-N-methylbutanamide
4-[cyclopropyl(4-fluorophenyl)amino]-N-(2-methoxybenzyl)-N-methylbutanamide
4-[ethyl(4-fluorophenyl)amino]-N-(2-methoxybenzyl)-N-methylbutanamide
N-(2,5-difluorobenzyl)-4-[(4-fluorophenyl)thio]-N-methylbutanamide
N-3,4-dihydro-2H-chromen-4-yl-4-[(4-fluorophenyl)thio]-N-methylbutanamide
4-[(4-fluorophenyl)thio]-N-methyl-N-1-naphthylbutanamide
4-[(4-fluorophenyl)thio]-N-methyl-N-1,2,3,4-tetrahydronaphthalen-1-ylbutanamide
N-(5-fluoro-2-methoxybenzyl)-4-[(4-fluorophenyl)thio]-N-methylbutanamide
N-(5-fluoro-2-isopropoxybenzyl)-4-[(4-fluorophenyl)thio]-N-methylbutanamide
4-[(4-fluorophenyl)amino]-N-(2-methoxybenzyl)-N-methylbutanamide
4-[(4-fluorophenyl)thio]-N-(1H-indol-7-ylmethyl)-N-methylbutanamide
N-(6-fluoro-3,4-dihydro-2H-chromen-4-yl)-4-[(4-fluorophenyl)thio]-N-methylbutanamide
N-(5-fluoro-2-methoxybenzyl)-4-[(4-methoxyphenyl)thio]-N-methylbutanamide
N-(2,6-dimethoxybenzyl)-4-[(4-fluorophenyl)thio]-N-methylbutanamide
N-(2-fluoro-5-methoxybenzyl)-4-[(4-fluorophenyl)thio]-N-methylbutanamide
N-[2-(allyloxy)benzyl]-4-[(4-fluorophenyl)thio]-N-methylbutanamide
N-(5-allyl-2-hydroxy-3-methoxybenzyl)-4-[(4-fluorophenyl)thio]-N-methylbutanamide
N-[(4-bromothien-2-yl)methyl]-4-[(4-fluorophenyl)thio]-N-methylbutanamide
N-(5-bromo-2-methoxybenzyl)-4-[(4-fluorophenyl)thio]-N-methylbutanamide
4-[(4-bromophenyl)thio]-N-(5-fluoro-2-methoxybenzyl)-N-methylbutanamide
as well as its racemates, stereoisomers or pharmaceutically acceptable salts.

17. A process of preparation of a compound of formula (I) according to anyone of the preceding claims where R1 and R2 form together with the C atom to which they are attached a C=O group comprising the step of reacting a corresponding compound of formula (II) with a corresponding compound of formula (III), or a precursor thereof : 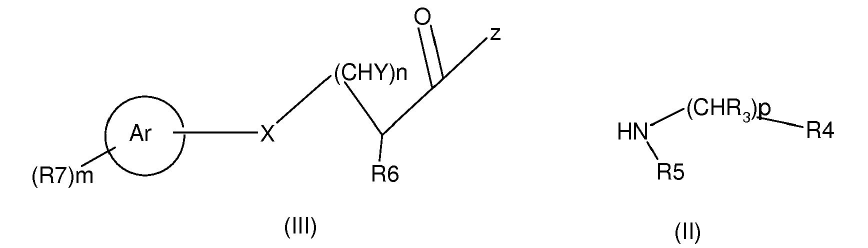 where R7, Ar, X, Y, R6, R5, R3, R4, m, n, p are defined as in formula (I) and Z is either a halogen atom or a OH group, optionally followed by the functionalization of the obtained compound, if appropriate.

18. Process of preparation of a compound according to anyone of claims 1 to 16 where X represents a -NT- by reacting a corresponding compound of formula (IV) with a corresponding compound of formula (V) or a precursor thereof: 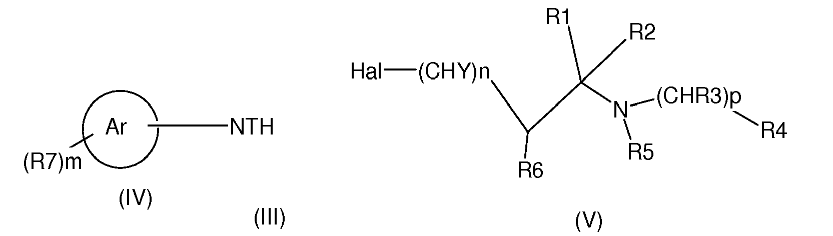 where R7, Ar, X, Y, R6, R5, R1, R2, R3, R4, T, m, n, p are defined as in formula (I) and Hal is a halogen atom, optionally followed by the functionalization of the obtained compound, if appropriate.

19. Process according to claim 17 or 18 further comprising the step of isolating the obtained compound.

20. A pharmaceutical composition comprising a compound of formula (I) 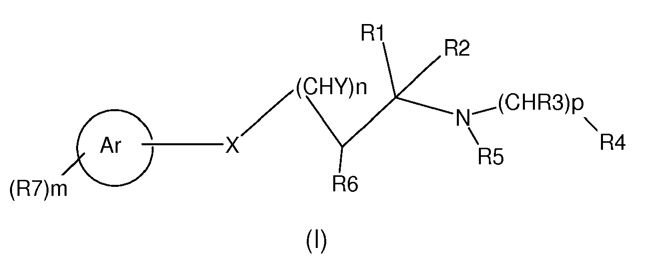 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O) R;
Or a R3 form with a substituent of R4 a cycloalkyl, aryl, heteroaryl or heterocycle fused with said R4;
R4 represents an aryl heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
- halogen atom; -Alkyl; -Aryl; -OAryl; -Alkyl-OR;
- Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
- C(=O)R; -C(=O)NRR'; -O-Alkyl-C(=O)NRR', -OAlkyl-NRR', -NR-C(=O)R';
- C(=O)OR; -S(O)R, -S(O)₂R; -OC(=O)R; -OAlkenyl; -Alkenyl;
- Heterocycle optionally substituted by an -Alkyl, -AlkylOR, -AlkylNRR',
- Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally substituted by a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 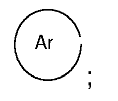
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 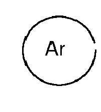 represents an aryl heteroaryl or unsaturated heterocycle;
m is an integer comprised between 1 and 5, provided that when 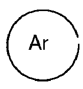 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts,
with the exception of the following compounds :
- the compounds where Ar is a tetrazol, X is S, n is 2, R6 is H, R1 and R2 form together a C=O with the carbon atom to which they are attached; and
- the compounds of formulae : 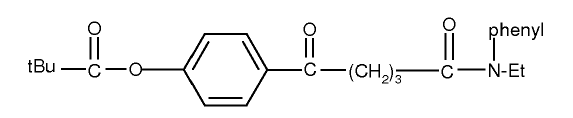 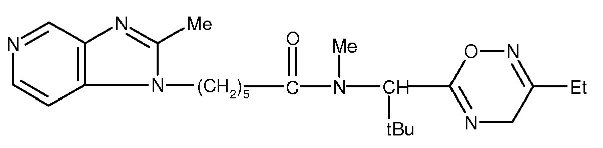 and a pharmaceutically acceptable carrier.

21. The pharmaceutical composition according to claim 18 where the compound of formula (I) is defined as in anyone of claims 2 to 16.

22. A compound of formula (I) for use for inhibiting integration of viruses into the host genom : 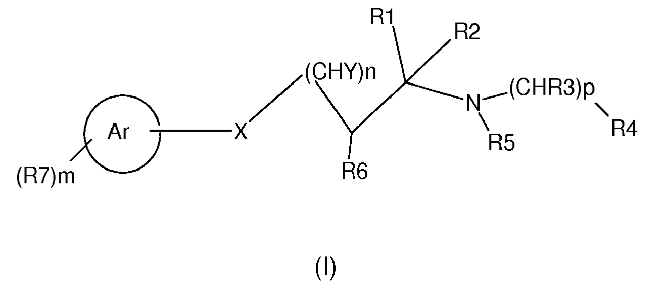 wherein:
R1, R2 form together a C=O or C=S group with the carbon atom to which they are attached;
Each R3, identical or different, represents a H atom or a group chosen from -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
Or a R3 form with a substituent of R4 a cycloalkyl, aryl, heteroaryl or heterocycle fused with said R4;
R4 represents an aryl heteroaryl or an unsaturated heterocycle, said aryl, heteroaryl or unsaturated heterocycle being optionally fused with an aryl, heteroaryl, saturated or unsaturated cycloalkyl or heterocyclic ring and said optionally fused aryl, heteroaryl or unsaturated heterocyle being optionally substituted by one or more identical or different substituent(s) chosen from:
- halogen atom; -Alkyl; -Aryl; -OAryl; -Alkyl-OR;
- Alkylaryl; -Alkyl-Heterocycle; -CN; -NO₂; perfluoroalkyl-; perfluoroalkoxy-; polyfluoroalkyl-; polyfluoroalkoxy-; -OHeterocycle-Alkyl; -OR; -NRR'; =O;
- C(=O)R; -C(=O)NRR'; -O-Alkyl-C(=O)NRR', -OAlkyl-NRR', -NR-C(=O)R';
- C(=O)OR; -S(O)R, -S(O)₂R; -OC(=O)R; -OAlkenyl; -Alkenyl;
- Heterocycle optionally substituted by an -Alkyl, -AlkylOR, -AlkylNRR',
- Alkylaryl, -OR, -Alkyl-Heterocycle, -Heteroaryl-perfluoroalkyl, -C(=O)Alkyl or -C(=O)OAlkyl group ;
R5 represent a group -Alkyl, -AlkylAryl, -Alkyl-Heterocycle, -AlkylHeteroaryl, -AlkylCycloalkyl, -Alkyl-OR, or -Cycloalkyl, each being optionally substituted by one or more substituents chosen from Halogen atoms, -Alkyl, -polyfluoroalkyl groups;
Or R4 and R5 form together with the N atom to which they are attached a N-containing heterocycle or heteroaryl optionally substituted by a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)qR, -OC(=O)R;
R6 represents a H atom or a group chosen from halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)_{q}Alkyl, -OC(=O)R;
Each R7, identical or different is independently chosen from Halogen atoms; -OR; -O-C(=O)R; -NR-C(=O)R'; -NR-S(O)q-R'; perfluoroalkyl; -C(=O)OR; -C(=O)Alkyl; -C(=O)Aryl; -C(=O)-NRR'; -AlkylAryl; -OAlkylAryl; -Alkyl; -Aryl; -CN; perfluoroalkoxy-; polyfluoroalkyl-; polyfluoroalkoxy-; =O; -C(=O)R; -C(=O)NRR'; -S(O)qR; -Alkyl-NRR'; -S(O)qNRR'; -S(O)_{q}Alkyl; -Alkyl-OR or 2 R7 form together with the atoms to which they are attached an unsubstituted ring chosen from aryl, heteroaryl, saturated or unsaturated cycloalkyl or heterocyclic ring fused with 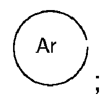
X represents a group chosen from -CRR'-, -NT-, -O-, -S(O)q-, -C(=O)-, where T represent a group chosen from H, aryl, cycloalkyl or alkyl optionally substituted by OH, heterocycle, or T may be optionally linked with a R7 to form with the N atom to which it is attached a N-containing heterocycle fused with said Ar;
Each Y, identical or different, represents a hydrogen atom or a OR group; 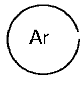 represents an aryl heteroaryl or unsaturated heterocycle;
m is an integer comprised between 1 and 5, provided that when 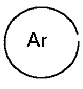 comprises one or more heteroatom, m may be equal to 0 ;
n is 2 or 3;
where R, R' identical or different, independently represent a hydrogen atom or an -alkyl, -aryl;
p is 0 or 1;
q is 0, 1 or 2;
as well as their racemates, stereoisomers or pharmaceutically acceptable salts.

23. The compound according to claim 21, wherein said compound of formula (I) is defined as in anyone of claims 2 to 16.

24. A compound of formula (I) as defined in claim 22 or 23 for use for treating and/or preventing viral infections and/or disorders caused by retroviruses.

25. A compound of formula (I) according to claim 24 for use for treating and/or preventing HIV.

26. A combination of a compound as defined in claim 22 or 23 with one or more agent(s) and/or pharmaceutical composition(s) useful for the treatment and/or prevention of viral infections and/or disorders.

27. The combination according to claim 26, wherein said agent(s) and/pr pharmaceutical composition(s) are useful for the treatment and/or prevention of HIV.
